# EUROPEAN PATENT APPLICATION

(11) **EP 4 635 967 A1**
(43) Date of publication of application: **22.10.2025**
(21) Application number: 23902751.9
(22) Date of filing: 13.12.2023
(51) Int. Cl.: C07H 21/02, C12N 15/113, A61K 31/713, A61P 35/00

(54) **OLIGONUCLEOTIDE COMPRISING LIPOPHILIC MONOMER AND USE THEREOF IN NON-LIVER DELIVERY**

(30) Priority: 13.12.2022 CN 202211602431
(71) Applicant: Tuojie Biotech (Shanghai) Co., Ltd., Shanghai 201203 (CN)
(72) Inventor: HOU, Zhe, Shanghai 201203 (CN); MA, Ningning, Shanghai 201203 (CN); HUANG, Xiaoling, Shanghai 201203 (CN); LI, Yuqi, Shanghai 201203 (CN); LI, Yunfei, Shanghai 201203 (CN)
(74) Representative: Regimbeau
(86) International application number: PCT/CN2023/138483
(87) International publication number: WO 2024/125556

(57) **Abstract**

An oligonucleotide comprising a lipophilic monomer and a use thereof in non-liver delivery. Specifically, the present disclosure relates to an oligonucleotide, comprising an antisense strand complementary to a target gene, a sense strand complementary to the antisense strand, and one or more compounds represented by formula (I). The present disclosure further relates to a pharmaceutical composition comprising the oligonucleotide, and a medicinal use of the oligonucleotide and/or the pharmaceutical composition.

## Description

### TECHNICAL FIELD

The present disclosure pertains to the field of biopharmaceutics and specifically relates to an oligonucleotide comprising a lipophilic monomer. The present disclosure further relates to a preparation method for and use of the oligonucleotide.

### BACKGROUND

RNA interference (RNAi) is an effective way of silencing gene expression and has wide application prospects. While RNA drugs have advantages such as a broad selection of targets, a simple and efficient development process, and high specificity, the number of RNA drugs approved by the FDA for clinical treatment remains very limited. There are several major pharmacological challenges, including poor stability, short half-life, high immunogenicity, small targeting capacity, limited cytoplasmic delivery, etc. Therefore, in addition to improving RNA stability and immunogenicity through chemical modifications, providing an efficient and safe *in vivo* delivery system and improving membrane permeability are also needed.

The delivery technologies for RNA interference drugs mainly include lipid nanoparticle (LNP) delivery, liver-targeted delivery mediated by N-acetylgalactosamine (GalNAC) modification, targeted delivery based on antibody mediation, etc. At present, there are many studies using GalNAC-conjugated drug technology. By the end of 2020, two GalNAC-conjugated siRNA drugs, lumasirna and inclisirna, were approved for clinical use. GalNAC-conjugated RNA drugs enable efficient and specific delivery to the liver. However, for extrahepatic cells that lack ASGPR expression, effective delivery methods are still unavailable. Therefore, there is a need to continue to develop efficient *in vivo* delivery methods to enable RNA drugs to exert their effects in extrahepatic tissues.

The compound represented by formula (I) or the tautomer thereof of the present disclosure, which contains a lipophilic moiety, has improved hydrophobicity and enhanced membrane permeability, which can not only increase the membrane permeability of oligonucleotides but also enable oligonucleotides containing the compound to be effectively delivered into the cytoplasm and exert RNA effects in non-hepatic tissues.

### SUMMARY

The present disclosure provides an oligonucleotide comprising at least one compound represented by formula (I) or a pharmaceutically acceptable salt thereof, wherein a structure of the compound represented by formula (I) is 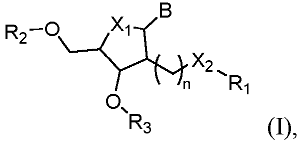 wherein:
X₁ is selected from the group consisting of O, S, N, and C atoms;
X₂ is selected from the group consisting of O and S atoms;
R₁ is selected from the group consisting of C₁₀-C₃₀ linear alkyl (e.g., C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, or C₃₀ linear alkyl), and C₁₀-C₃₀ linear alkyl interrupted by one or more O or S atoms (e.g., C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, or C₃₀ linear alkyl); optionally, the C₁₀-C₃₀ linear alkyl is substituted with one or more Rₐ, or optionally, C₃-₆ cycloalkyl (C₃, C₄, C₅, or C₆ cycloalkyl, including but not limited to cyclopropyl, cyclobutyl, cyclopentyl, and cyclohexyl) forms between two adjacent carbon atoms of the C₁₀-C₃₀ linear alkyl;
R₂ and R₃ are identical or different and are each independently selected from the group consisting of a bond, hydrogen, an activated phosphoester group, an activated phosphite ester group, a phosphoramidite group, a solid support, an internucleotide linking group linked to the oligonucleotide, -P(=O)(OH)-O-, -P(=O)(SH)-O-, -P(=O)(OH)-O-nucleoside, -P(=O)(SH)-O-nucleoside, -P(=O)(OH)-O-oligonucleotide fragment, -P(=O)(SH)-O-oligonucleotide fragment, and a hydroxy protecting group (e.g., an ester group protecting group, an aryl protecting group, an alkyl protecting group, an alkoxymethyl protecting group, or a silyl protecting group, including but not limited to DMTr);
each Rₐ is independently selected from the group consisting of hydrogen, deuterium, halogen (e.g., fluorine, chlorine, or bromine), hydroxy, cyano, alkyl (e.g., C₁, C₂, C₃, C₄, C₅, or C₆ alkyl, including but not limited to methyl, ethyl, and isopropyl), haloalkyl, alkoxy (e.g., C₁ alkoxy, C₂ alkoxy, C₃ alkoxy, C₄ alkoxy, C₅ alkoxy, or C₆ alkoxy, including but not limited to methoxy, ethoxy, propoxy, and isopropoxy), cycloalkyl (including but not limited to cyclopropyl, cyclobutyl, cyclopentyl, and cyclohexyl), and heterocycloalkyl;
n is selected from the group consisting of 0 and 1;
B is a base;
the compound represented by formula (I) is not selected from the group consisting of: 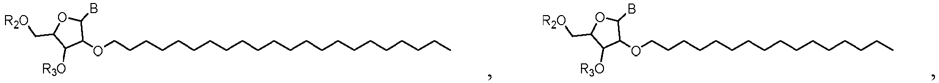 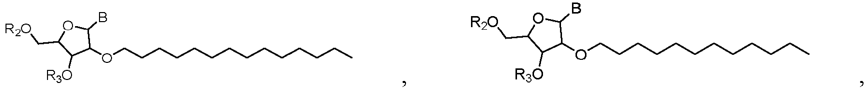 and

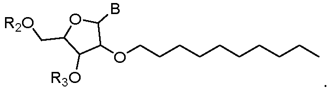

In some embodiments, the structure of the compound represented by formula (I) is selected from the group consisting of: 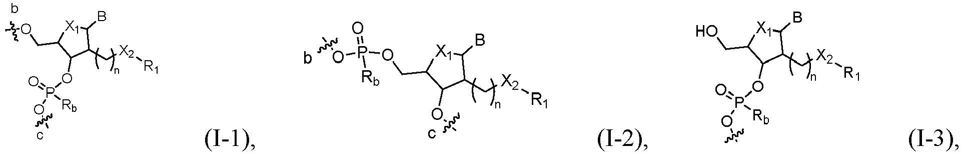 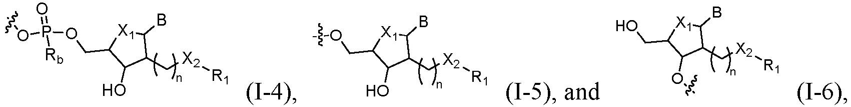 wherein R_{b} is selected from the group consisting of OH and SH.

In some embodiments, X₁ is selected from the group consisting of O and S atoms, preferably from the group consisting of an O atom.

In some embodiments, X₂ is selected from the group consisting of O and S atoms, preferably from the group consisting of an O atom.

In some embodiments, R₁ is selected from the group consisting of C₁₄-C₂₄ linear alkyl (e.g., C₁₄, C₁₅, C₁₆, C₁₇, C₁₈, C₁₉, C₂₀, C₂₁, C₂₂, C₂₃, or C₂₄ linear alkyl), and C₁₄-C₂₄ linear alkyl interrupted by one or more O or S atoms (e.g., C₁₄, C₁₅, C₁₆, C₁₇, C₁₈, C₁₉, C₂₀, C₂₁, C₂₂, C₂₃, or C₂₄ linear alkyl).

In some embodiments, each Rₐ is independently selected from the group consisting of hydrogen, deuterium, halogen (e.g., fluorine, chlorine, or bromine), C₁-₆ alkyl (e.g., C₁, C₂, C₃, C₄, C₅, or C₆ alkyl, including but not limited to methyl, ethyl, and isopropyl), and C₁-₆ alkoxy (e.g., C₁ alkoxy, C₂ alkoxy, C₃ alkoxy, C₄ alkoxy, C₅ alkoxy, or C₆ alkoxy, including but not limited to methoxy, ethoxy, propoxy, and isopropoxy).

In some embodiments, each Rₐ is independently selected from the group consisting of hydrogen, deuterium, fluorine, methyl, and methoxy.

In some embodiments, R₁ is selected from the group consisting of: 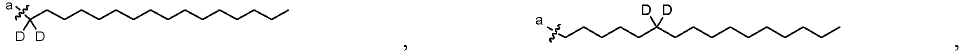 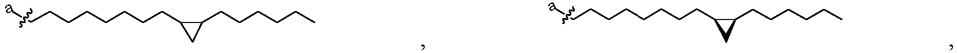 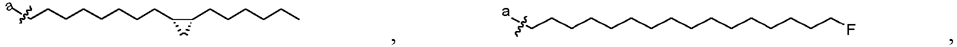 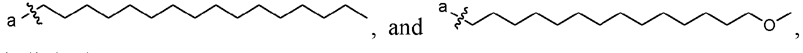 wherein end a is linked to X₂.

In some embodiments, B is selected from the group consisting of adenine, guanine, cytosine, uracil, and thymine.

The present disclosure further provides an oligonucleotide comprising at least one of the following compounds or a pharmaceutically acceptable salt thereof, wherein structures of the compounds are:

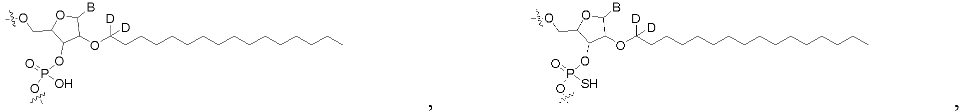

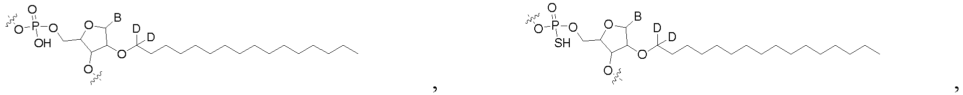

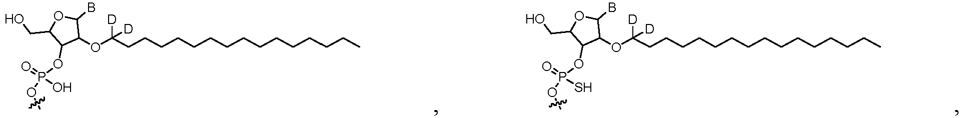

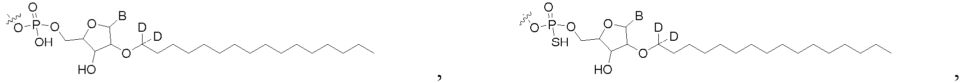

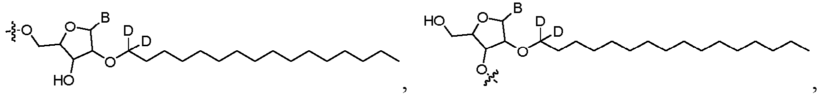

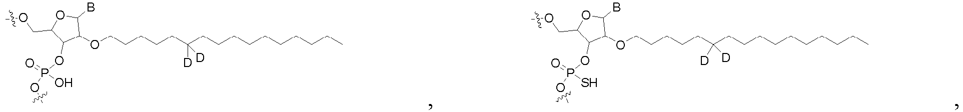

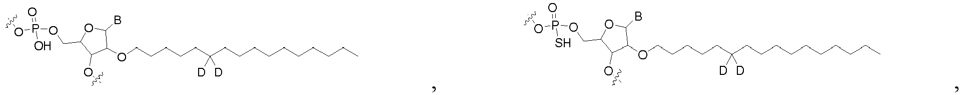

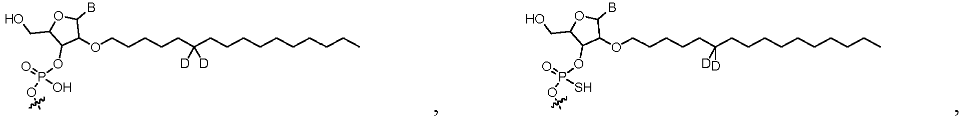

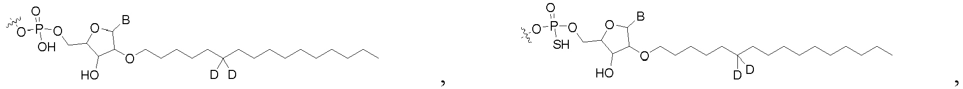

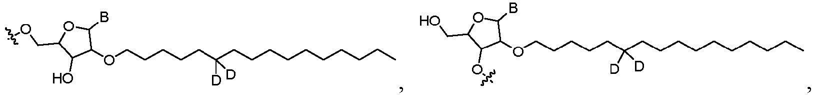

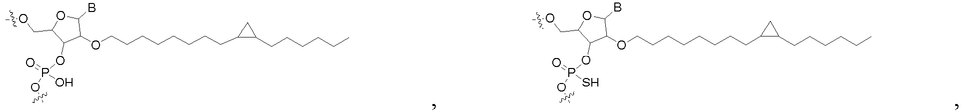

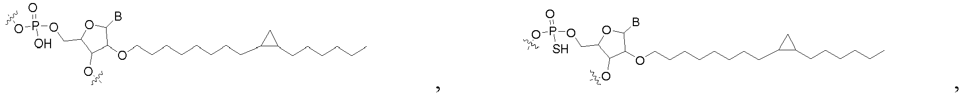

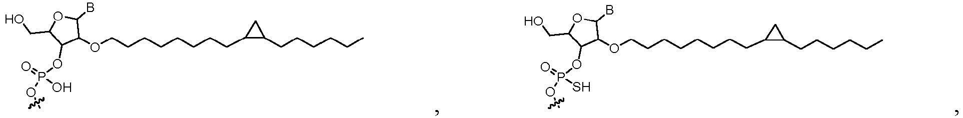

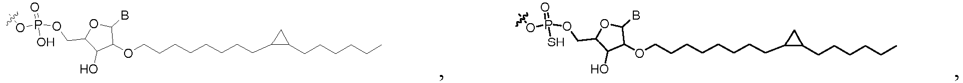

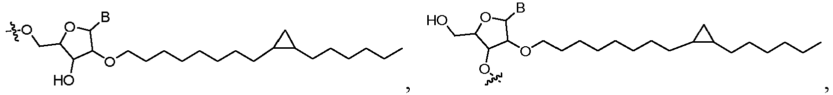

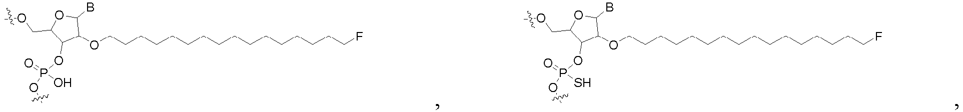

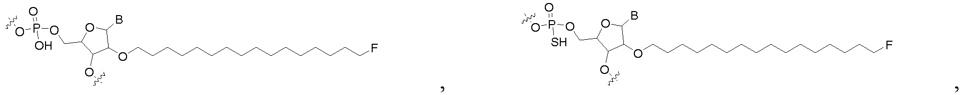

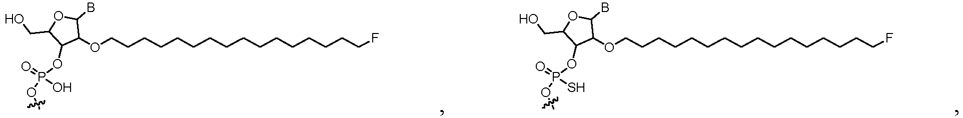

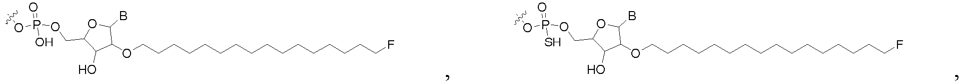

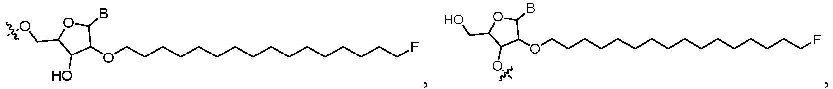

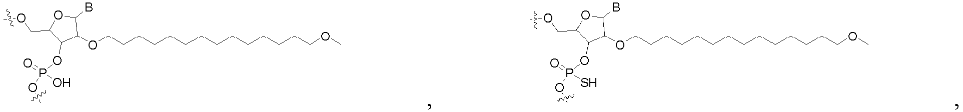

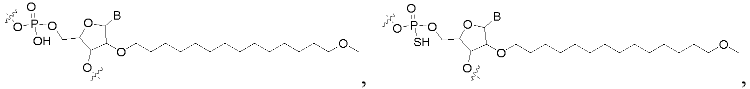

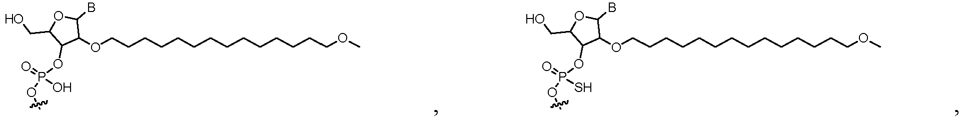

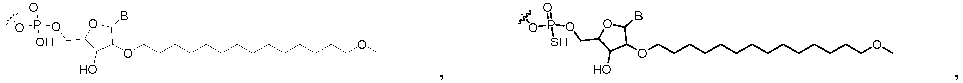

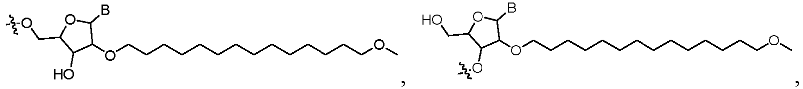

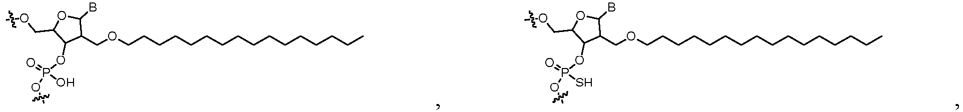

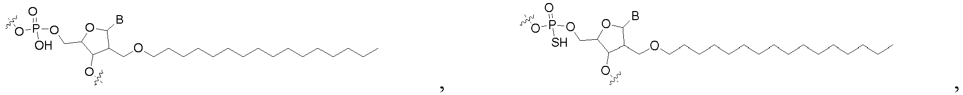

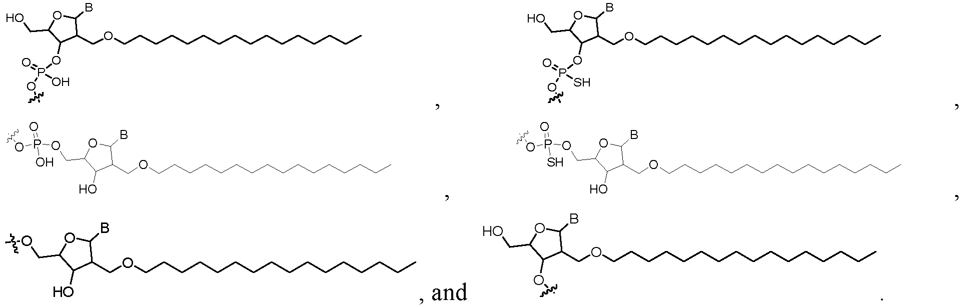

In some embodiments, the structure of the compound represented by formula (I) is selected from the group consisting of:

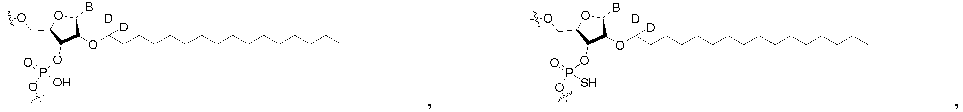

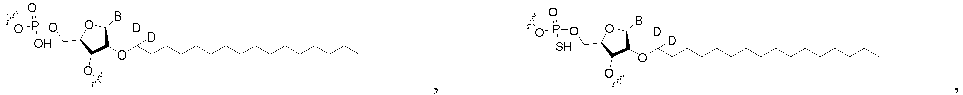

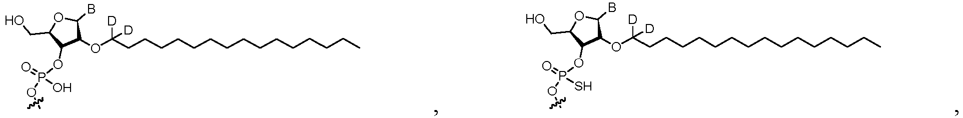

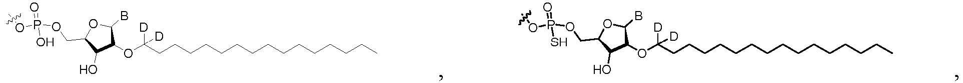

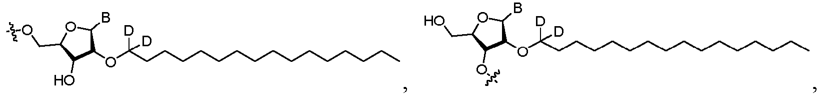

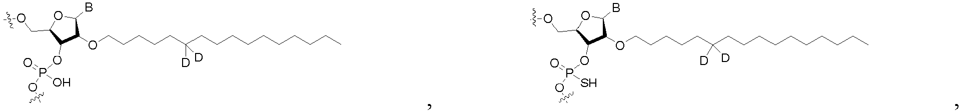

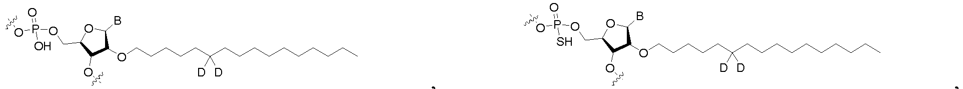

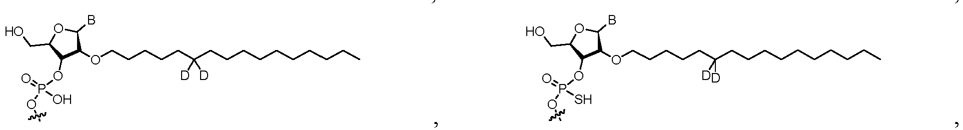

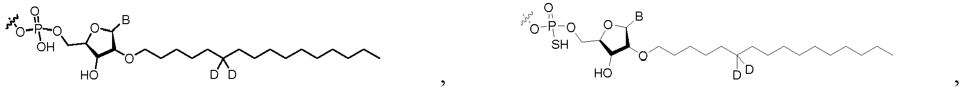

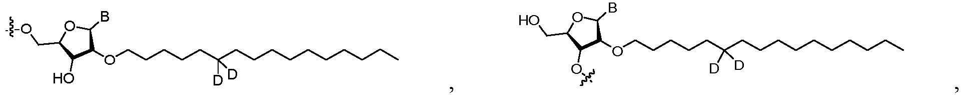

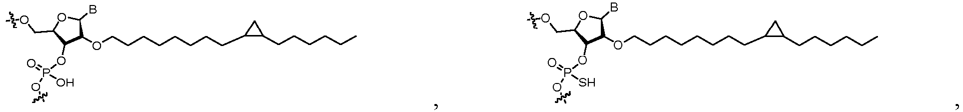

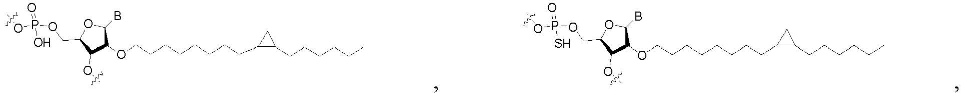

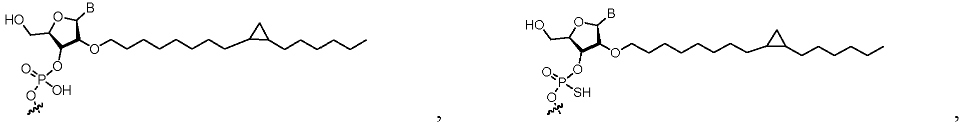

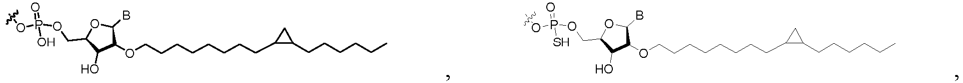

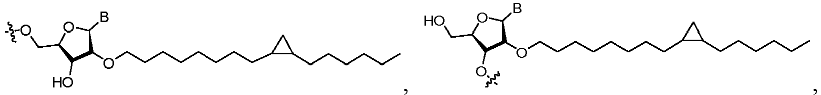

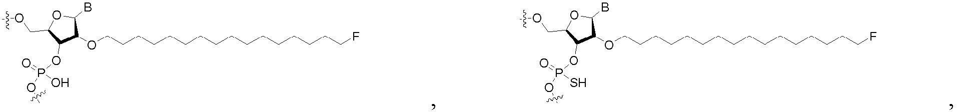

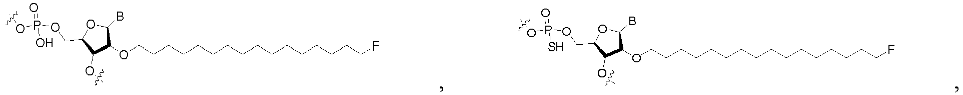

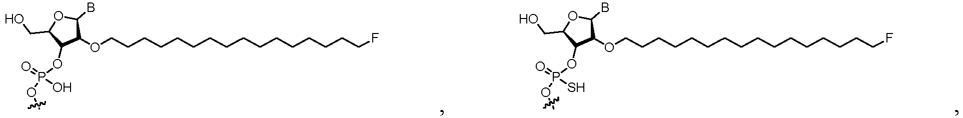

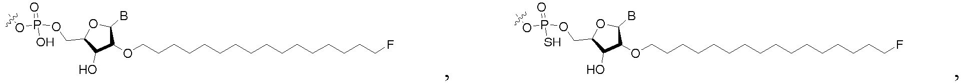

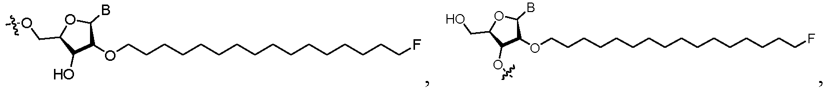

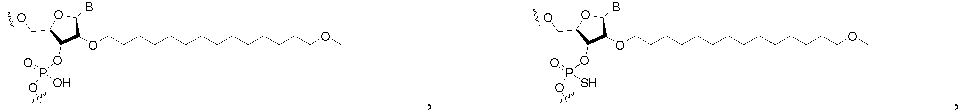

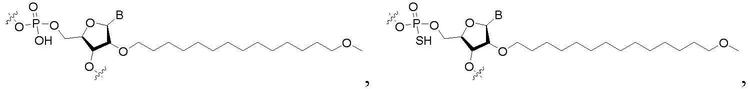

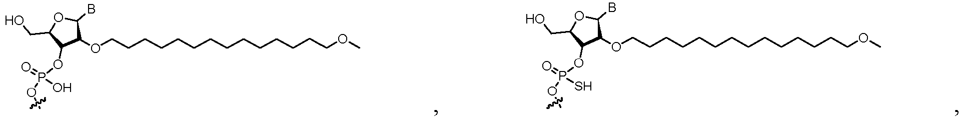

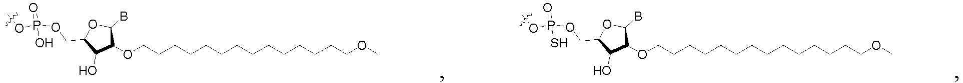

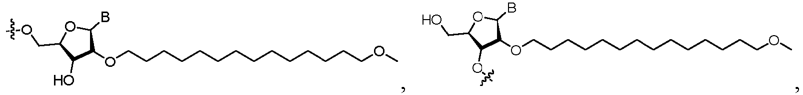

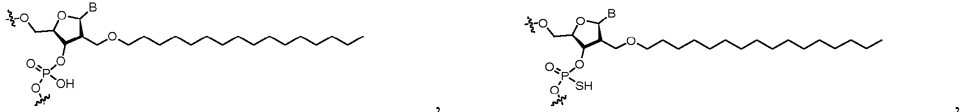

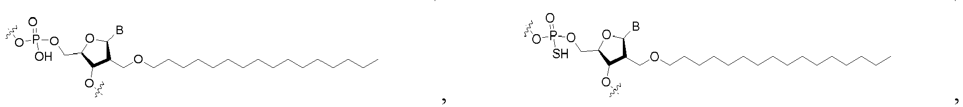

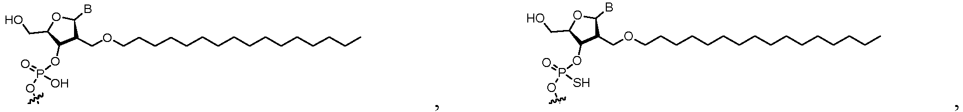

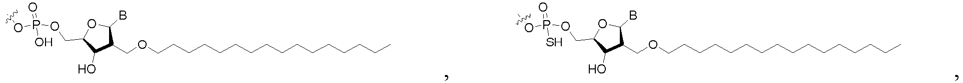

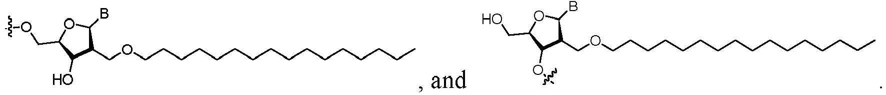

In some embodiments, the oligonucleotide of the present disclosure comprises 1, 2, 3, or 4 compounds represented by formula (I) or pharmaceutically acceptable salts thereof; in some embodiments, the oligonucleotide comprises 1 compound represented by formula (I) or pharmaceutically acceptable salt thereof.

In some embodiments, the compound represented by formula (I) or the pharmaceutically acceptable salt thereof is located at at least one of the following nucleotide positions:
position 1 of the 5' end of the sense strand;
position 1 of the 3' end of the sense strand;
position 1 of the 5' end of the antisense strand;
position 1 of the 3' end of the antisense strand;
positions 2-8 (e.g., position 2, position 3, position 4, position 5, position 6, position 7, or position 8) in the middle of the sense strand; and
positions 2-8 (e.g., position 2, position 3, position 4, position 5, position 6, position 7, or position 8) in the middle of the antisense strand.

The position in the middle refers to the position in the middle when counting from the first nucleotide at the 5' or 3' end of the antisense strand and/or the sense strand; for example, "position 6 in the middle of the sense strand" refers to the sixth nucleotide when counting from the first nucleotide at the 5' or 3' end of the sense strand. Preferably, the the position in the middle refers to the the position in the middle when counting from the first nucleotide at the 5' end; for example, "position 6 in the middle of the sense strand" refers to the position of the sixth nucleotide when counting from the first nucleotide at the 5' end of the sense strand.

In some embodiments, the compound represented by formula (I) or the pharmaceutically acceptable salt thereof is located at at least one of the following nucleotides:
position 1 of the 5' end of the sense strand;
position 1 of the 3' end of the sense strand; and
position 6 in the middle of the sense strand.

In some embodiments, the structure of the compound represented by formula (I) or the pharmaceutically acceptable salt thereof is selected from the group consisting of: 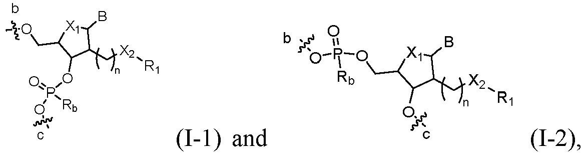 wherein end b is linked to the fifth nucleotide of the 5' end of the sense strand, and end c is linked to the seventh nucleotide of the 5' end of the sense strand; the B in formulas (I-1) and (I-2) represents the base of the nucleotide at position 6 in the direction from the 5' end to the 3' end of the sense strand.

In some embodiments, the structure of the compound represented by formula (I) or the pharmaceutically acceptable salt thereof is selected from the group consisting of: 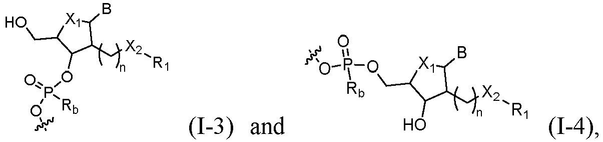 wherein the linking end 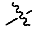 is linked to the nucleotide at position 2 of the 5' end of the sense strand; that is, formula (I-3) or (I-4) is located at the nucleotide at position 1 of the 5' end of the sense strand; the B in formulas (I-3) and (I-4) represents the base of the nucleotide at position 1 of the 5' end of the sense strand.

In some embodiments, the structure of the compound represented by formula (I) or the pharmaceutically acceptable salt thereof is selected from the group consisting of: 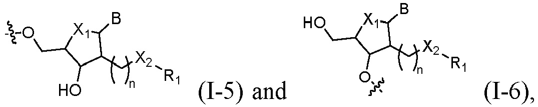 wherein the linking end 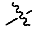 is linked to the nucleotide at position 2 of the 3' end of the sense strand; that is, formula (I-5) or (I-6) is located at the nucleotide at position 1 of the 3' end of the sense strand; the B in formulas (I-5) and (I-6) represents the base of the nucleotide at position 1 of the 3' end of the sense strand.

In some embodiments, the oligonucleotide of the present disclosure comprises a compound represented by formula (I) or a pharmaceutically acceptable salt thereof, wherein the compound represented by formula (I) or the pharmaceutically acceptable salt thereof is located at the nucleotide at position 6 of the 5' end of the sense strand and is selected from the group consisting of:

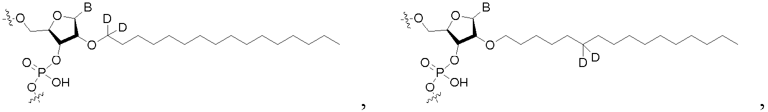

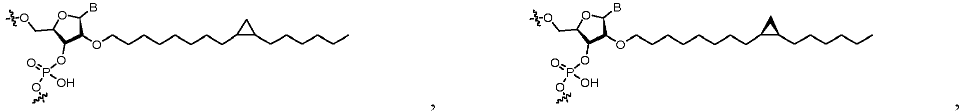

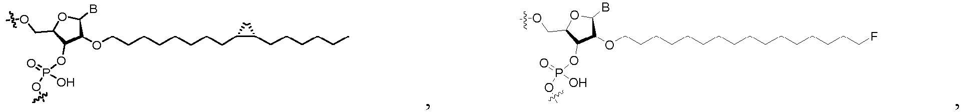

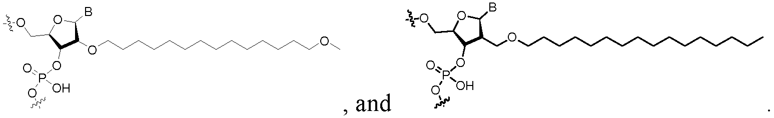

In some embodiments, the oligonucleotide of the present disclosure comprises a compound represented by formula (I) or a pharmaceutically acceptable salt thereof, wherein the compound represented by formula (I) or the pharmaceutically acceptable salt thereof is located at the nucleotide at position 6 of the 5' end of the sense strand and is selected from the group consisting of: 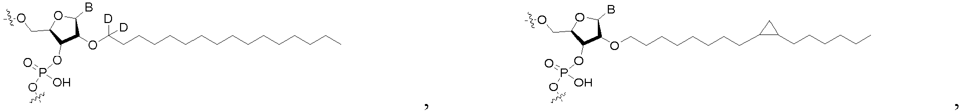 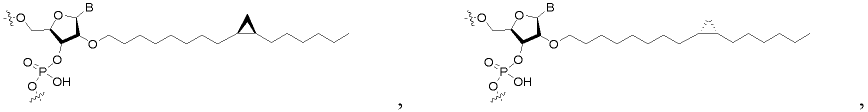 and

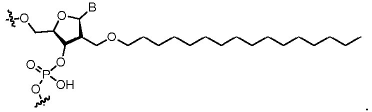

**In** some embodiments, the oligonucleotide of the present disclosure is selected from the group consisting of a double-stranded RNAi inhibitor molecule; in some embodiments, the oligonucleotide is selected from the group consisting of an siRNA comprising a sense strand and an antisense strand that form a double-stranded region; in some embodiments, the sense strand of the siRNA is 15-35 nucleotides in length, and the antisense strand is 15-30 nucleotides in length.

In some embodiments, the sense strand and the antisense strand are identical or different in length; the sense strand is 19-23 nucleotides in length, and the antisense strand is 19-26 nucleotides in length. Therefore, the length ratio of the sense strand to the antisense strand of the siRNA provided in the present disclosure may be 19/20, 19/21, 19/22, 19/23, 19/24, 19/25, 19/26, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25, or 23/26. In some embodiments, the length ratio of the sense strand to the antisense strand of the siRNA is 19/21, 21/23, or 23/25. In some embodiments, the length ratio of the sense strand to the antisense strand of the siRNA is 21/23.

In some embodiments, the antisense strand is at least partially reverse complementary to a target sequence to mediate RNA interference; in some embodiments, there are no more than 5, no more than 4, no more than 3, no more than 2, or no more than 1 mismatch between the antisense strand and the target sequence; in some embodiments, the antisense strand is fully reverse complementary to the target sequence.

In some embodiments, the sense strand is at least partially reverse complementary to the antisense strand to form a double-stranded region; in some embodiments, there are no more than 5, no more than 4, no more than 3, no more than 2, or no more than 1 mismatch between the sense strand and the antisense strand; in some embodiments, the sense strand is fully reverse complementary to the antisense strand.

In some embodiments, the siRNA of the present disclosure comprises one or two blunt ends.

In some specific embodiments, the siRNA comprises an overhang having 1 to 4 unpaired nucleotides, e.g., 1, 2, 3, or 4 unpaired nucleotides.

In some embodiments, the siRNA of the present disclosure comprises an overhang at the 3' end of the antisense strand of the siRNA.

In some embodiments, in the oligonucleotide of the present disclosure, at least one nucleotide other than the structure of the compound represented by formula (I) is a modified nucleotide. In some embodiments, each of the sense strand and the antisense strand of the present disclosure comprises at least one nucleotide that is a modified nucleotide.

In some embodiments, the modified nucleotide is selected from the group consisting of: a 2'-methoxy-modified nucleotide, a 2'-substituted alkoxy-modified nucleotide, a 2'-alkyl-modified nucleotide, a 2'-substituted alkyl-modified nucleotide, a 2'-amino-modified nucleotide, a 2'-substituted amino-modified nucleotide, a 2'-fluoro-modified nucleotide, a 2'-deoxynucleotide, a 2'-deoxy-2'-fluoro-modified nucleotide, a 3'-deoxy-thymine (dT) nucleotide, an isonucleotide, LNA, ENA, cET, UNA, and GNA.

In some embodiments, the modified nucleotides are each independently selected from the group consisting of: a 2'-methoxy-modified nucleotide and a 2'-fluoro-modified nucleotide.

In some embodiments, the oligonucleotide is selected from the group consisting of a double-stranded RNAi inhibitor molecule comprising a sense strand and an antisense strand that form a double-stranded region, wherein the sense strand comprises three consecutive nucleotides that are 2'-fluoro-modified nucleotides.

In some embodiments, in the direction from the 5' end to the 3' end, the nucleotides at positions 9, 10, and 11 of the sense strand are each independently a 2'-fluoro-modified nucleotide.

In some embodiments, in the direction from the 5' end to the 3' end, the nucleotides at positions 9, 10, and 11 of the sense strand are each independently a 2'-fluoro-modified nucleotide, the nucleotide at position 7 of the sense strand is selected from the group consisting of a 2'-fluoro-modified nucleotide, and the nucleotides at other positions of the sense strand are selected from the group consisting of a 2'-methoxy-modified nucleotide.

In some embodiments, the oligonucleotide is selected from the group consisting of a double-stranded RNAi inhibitor molecule comprising a sense strand and an antisense strand that form a double-stranded region, wherein in the direction from the 5' end to the 3' end, the nucleotides at positions 2, 6, 8, 9, 14, and 16 of the antisense strand are each independently a 2'-fluoro-modified nucleotide.

In some embodiments, in the direction from the 5' end to the 3' end, the nucleotides at positions 2, 6, 8, 9, 14, and 16 of the antisense strand are each independently a 2'-fluoro-modified nucleotide, and the nucleotides at other positions of the antisense strand are selected from the group consisting of a 2'-methoxy-modified nucleotide.

In some embodiments, the sense strand comprises or is a sequence represented by the following formula:
5' N_{L}NₐNₐNₐNₐNₐNₐNₐN_{b}N_{b}N_{b}NₐNₐNₐNₐNₐNₐNₐNₐNₐ Nₐ3', or
5' NₐNₐNₐNₐNₐNₐNₐNₐN_{b}N_{b}N_{b}NₐNₐNₐNₐNₐNₐNₐNₐ NₐN_{L}3', or
5' NₐNₐNₐNₐNₐN_{L}NₐNₐN_{b}N_{b}N_{b}NₐNₐNₐNₐNₐNₐNₐNₐNₐ Nₐ3', or
5' N_{L}NₐNₐNₐNₐNₐN_{b}NₐN_{b}N_{b}N_{b}NₐNₐNₐNₐNₐNₐNₐNₐNₐ Nₐ3', or
5' NₐNₐNₐNₐNₐNₐN_{b}NₐN_{b}N_{b}N_{b}NₐNₐNₐNₐNₐNₐNₐNₐ NₐN_{L}3', or
5' NₐNₐNₐNₐNₐN_{L}N_{b}NₐN_{b}N_{b}N_{b}NₐNₐNₐNₐNₐNₐNₐNₐNₐNₐ3', wherein each NL is independently selected from the group consisting of the compound represented by formula (I) and the pharmaceutically acceptable salt thereof of the present disclosure; Nₐ is a 2'-methoxy-modified nucleotide, and N_{b} is a 2'-fluoro-modified nucleotide.

In some embodiments, the antisense strand comprises or is a sequence represented by the following formula:
5'N_{L}'N_{b}'Nₐ'Nₐ'Nₐ'N_{b}'Nₐ'N_{b}'N_{b}'Nₐ'Nₐ'Nₐ'Nₐ'N_{b}'Nₐ'N_{b}'Nₐ'Nₐ'Nₐ'Nₐ'Nₐ'Nₐ'Nₐ'3, or
5'Nₐ'N_{b}'Nₐ'Nₐ'Nₐ'N_{b}'Nₐ'N_{b}'N_{b}'Nₐ'Nₐ'Nₐ'Nₐ'N_{b}'Nₐ'N_{b}'Nₐ'Nₐ'Nₐ'Nₐ'Nₐ'Nₐ'N_{L}'3, or
5'Nₐ'N_{b}'Nₐ'Nₐ'Nₐ'N_{b}'Nₐ'N_{b}'N_{b}'Nₐ'Nₐ'Nₐ'Nₐ'N_{b}'Nₐ'N_{b}'Nₐ'Nₐ'Nₐ'Nₐ'Nₐ'Nₐ'Nₐ'3, wherein each N_{L}' is independently selected from the group consisting of the compound represented by formula (I) and the pharmaceutically acceptable salt thereof of the present disclosure; Nₐ' is a 2'-methoxy-modified nucleotide, and N_{b}' is a 2'-fluoro-modified nucleotide.

In some embodiments, the sense strand comprises or is a sequence represented by the following formula:
5' N_{L}NNNNNNNNNNNNNNNNNN3', or
5' N_{L}NₐNₐNₐNₐNₐNₐNₐN_{b}N_{b}N_{b}NₐNₐNₐNₐNₐNₐNₐNₐNₐ Nₐ3', or
5' N_{L}NₐNₐNₐNₐNₐN_{b}NₐN_{b}N_{b}N_{b}NₐNₐNₐNₐNₐNₐNₐNₐNₐ Nₐ3', wherein N is a modified or unmodified nucleotide; Nₐ is a 2'-methoxy-modified nucleotide, and N_{b} is a 2'-fluoro-modified nucleotide;
N_{L} is selected from the group consisting of:

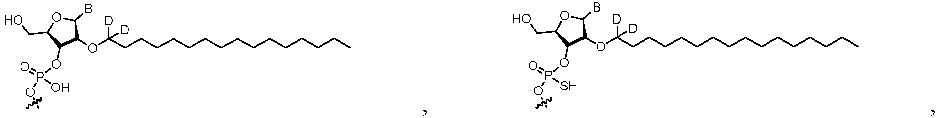

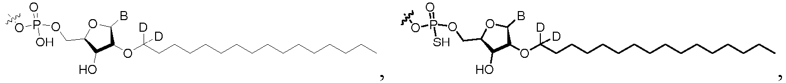

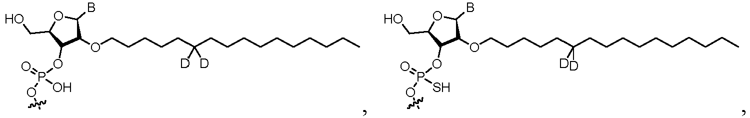

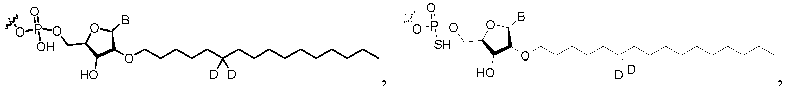

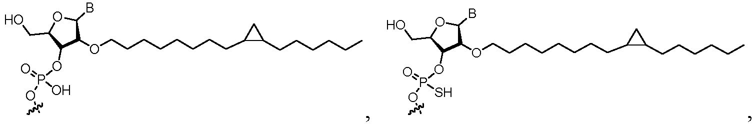

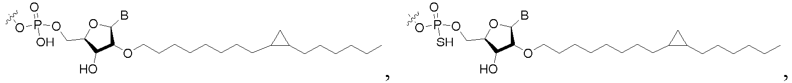

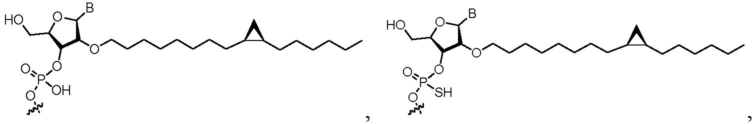

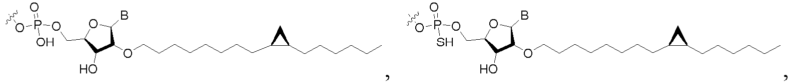

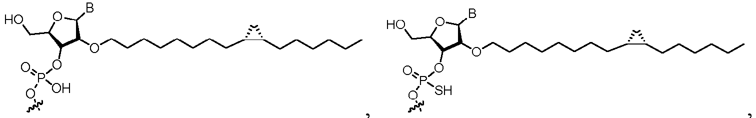

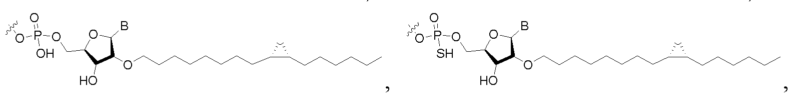

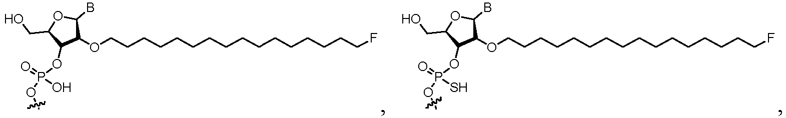

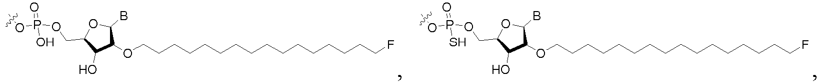

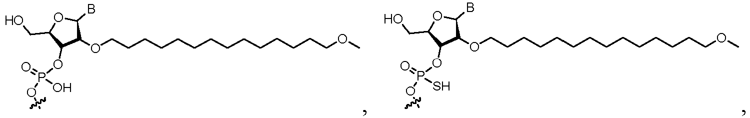

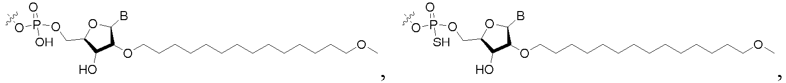

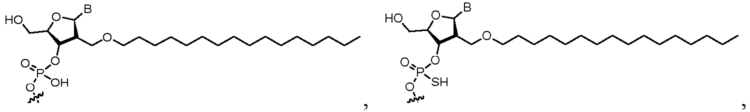

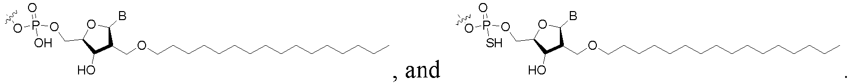

In some embodiments, the sense strand comprises or is a sequence represented by the following formula:
5' NNNNNNNNNNNNNNNNNNN_{L}3', or
5' NₐNₐNₐNₐNₐNₐNₐNₐN_{b}N_{b}N_{b}NₐNₐNₐNₐNₐNₐNₐNₐ NₐN_{L}3', or
5' NₐNₐNₐNₐNₐNₐN_{b}NₐN_{b}N_{b}N_{b}NₐNₐNₐNₐNₐNₐNₐNₐ NₐN_{L}3', wherein N is a modified or unmodified nucleotide; Nₐ is a 2'-methoxy-modified nucleotide, and N_{b} is a 2'-fluoro-modified nucleotide;
N_{L} is selected from the group consisting of:

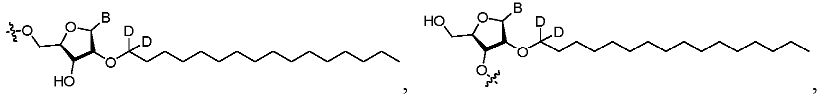

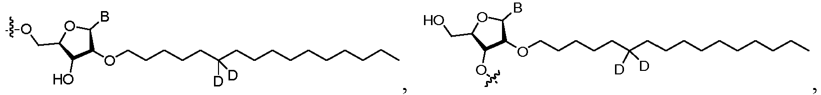

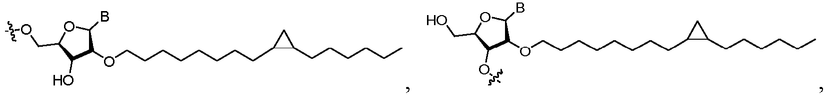

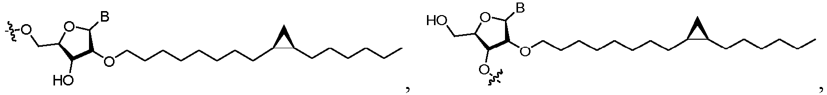

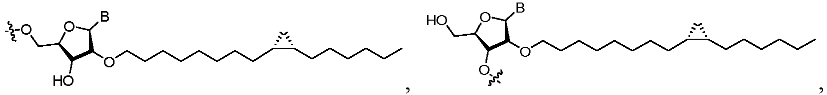

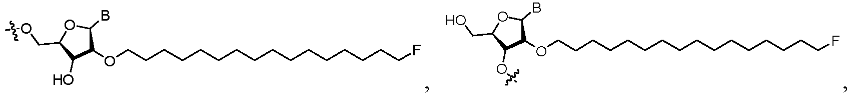

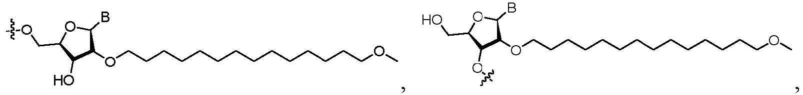

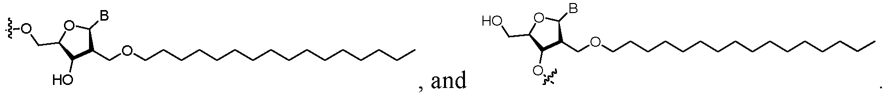

In some embodiments, the sense strand comprises or is a sequence represented by the following formula:
5' NNNNNN_{L}NNNNNNNNNNNNN3', or
5' NₐNₐNₐNₐNₐN_{L}NₐNₐN_{b}N_{b}N_{b}NₐNₐNₐNₐNₐNₐNₐNₐNₐ Nₐ3', or
5' NₐNₐNₐNₐNₐN_{L}N_{b}NₐN_{b}N_{b}N_{b}NₐNₐNₐNₐNₐNₐNₐNₐNₐ Nₐ3', wherein N is a modified or unmodified nucleotide; Nₐ is a 2'-methoxy-modified nucleotide, and N_{b} is a 2'-fluoro-modified nucleotide;
N_{L} is selected from the group consisting of:

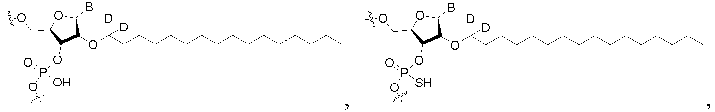

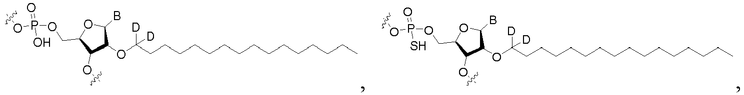

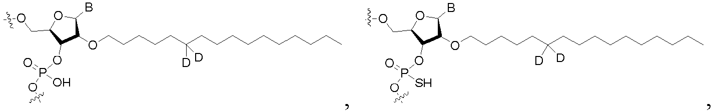

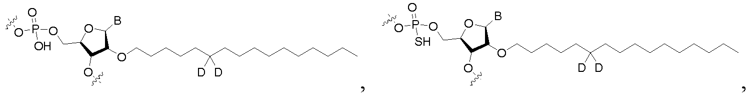

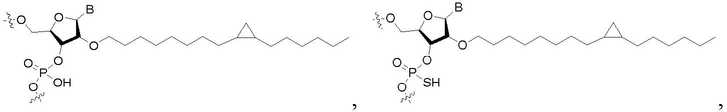

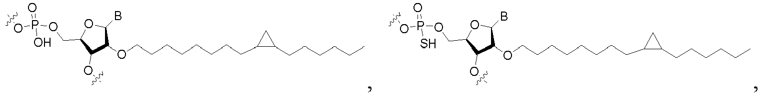

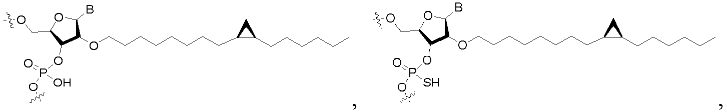

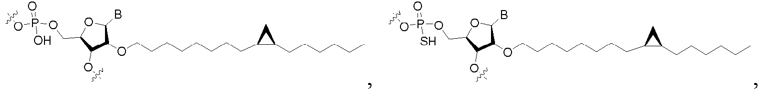

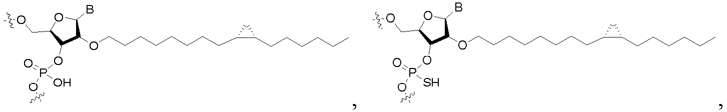

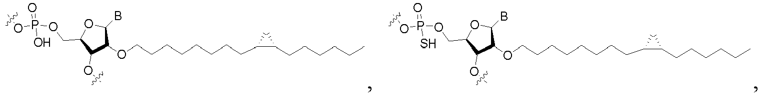

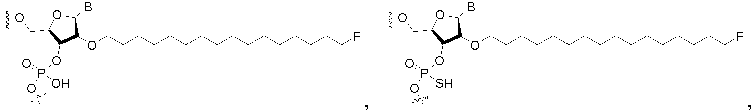

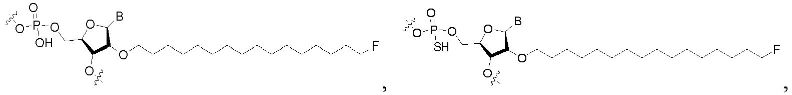

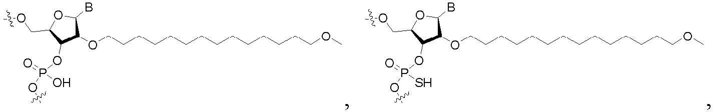

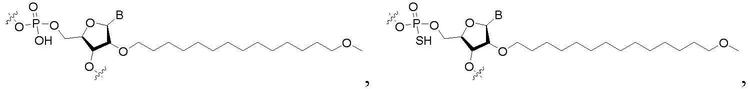

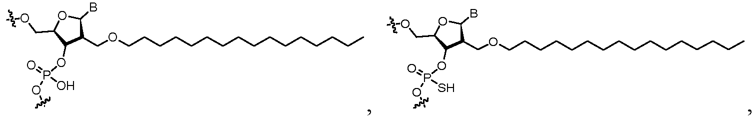

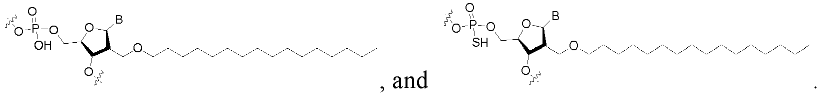

In some embodiments, the sense strand is a sequence represented by the following formula:
5' NNNNNN_{L}NNNNNNNNNNNNNNN3', or
5' NₐNₐNₐNₐNₐN_{L}NₐNₐN_{b}N_{b}N_{b}NₐNₐNₐNₐNₐNₐNₐNₐNₐNₐ3', or
5' NₐNₐNₐNₐNₐN_{L}N_{b}NₐN_{b}N_{b}N_{b}NₐNₐNₐNₐNₐNₐNₐNₐNₐ Nₐ3', wherein N is a modified or unmodified nucleotide; Nₐ is a 2'-methoxy-modified nucleotide, and N_{b} is a 2'-fluoro-modified nucleotide;
N_{L} is selected from the group consisting of: 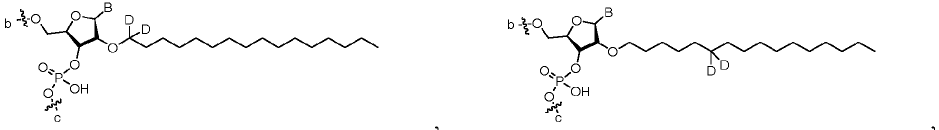 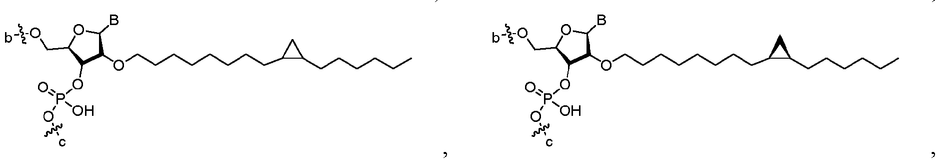 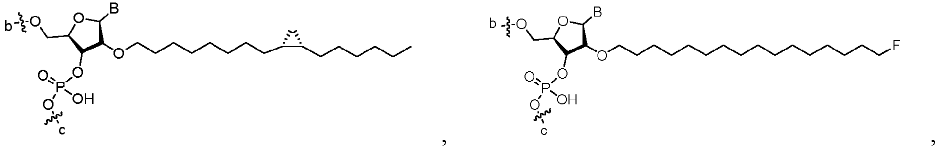 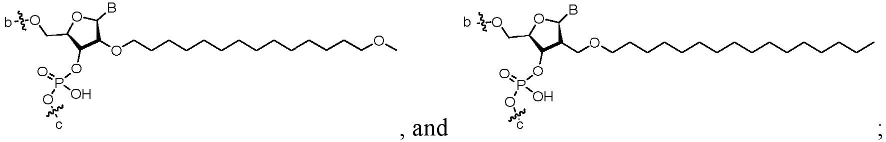
the end b of the above structure is linked to the nucleotide at position 5 of the 5' end of the sense strand, and the end c is linked to the nucleotide at position 7 of the 5' end of the sense strand; the B in the above structure represents the base of the nucleotide at position 6 of the 5' end of the sense strand.

In some embodiments, at least one phosphoester group in the sense strand and/or the antisense strand is a phosphoester group with a modifying group; in some embodiments, at least one phosphoester group in the sense strand and/or the antisense strand is a phosphorothioate diester group.

In some embodiments, the phosphorothioate diester group is present at at least one of the following positions:
between the first and second nucleotides of the 5' end of the sense strand;
between the second and third nucleotides of the 5' end of the sense strand;
between the first and second nucleotides of the 3' end of the sense strand;
between the second and third nucleotides of the 3' end of the sense strand;
between the first and second nucleotides of the 5' end of the antisense strand;
between the second and third nucleotides of the 5' end of the antisense strand;
between the first and second nucleotides of the 3' end of the antisense strand; and
between the second and third nucleotides of the 3' end of the antisense strand.

In some embodiments, at least one phosphoester group in the sense strand and/or the antisense strand is a 5'-vinyl phosphodiester group; in some embodiments, the 5'-vinyl phosphodiester group is present on the first nucleotide of the 5' end of the antisense strand.

In some embodiments, the oligonucleotide of the present disclosure targets MUC5B.

The present disclosure further provides a compound represented by formula (II) or a pharmaceutically acceptable salt thereof: 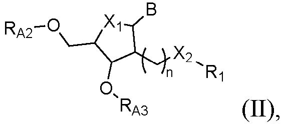 wherein:
in the formula, X₁, X₂, B, and R₁ have the same definitions and selection ranges as in the structure of the compound represented by formula (I) described above;
n is selected from the group consisting of 1 and 0; R_{A2} is selected from the group consisting of a leaving group, and R_{A3} is selected from the group consisting of a phosphorus-containing reactive group; or R_{A3} is selected from the group consisting of a leaving group, and R_{A2} is selected from the group consisting of a phosphorus-containing reactive group; the compound represented by formula (II) is not selected from the group consisting of:

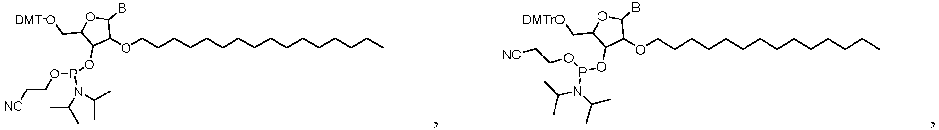

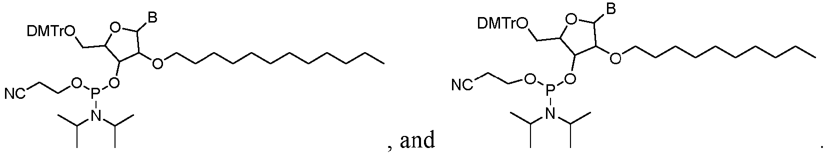

In some embodiments, the compound represented by formula (II) or the pharmaceutically acceptable salt thereof is selected from the group consisting of: 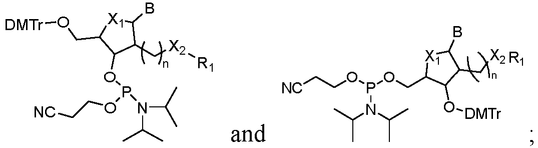 in the formulas, X₁, X₂, B, R₁, and n have the same definitions and selection ranges as in the structure of the compound represented by formula (I) described above.

In some embodiments, X₁ is selected from the group consisting of O and S atoms, preferably from the group consisting of an O atom.

In some embodiments, X₂ is selected from the group consisting of O and S atoms, preferably from the group consisting of an O atom.

In some embodiments, R₁ is selected from the group consisting of C₁₀-C₃₀ linear alkyl (e.g., C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, or C₃₀ linear alkyl), and C₁₀-C₃₀ linear alkyl interrupted by one or more O or S atoms (e.g., C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, or C₃₀ linear alkyl); optionally, the C₁₀-C₃₀ linear alkyl may be substituted with one or more Rₐ, or optionally, C₃-₆ cycloalkyl (C₃, C₄, C₅, or C₆ cycloalkyl, including but not limited to cyclopropyl, cyclobutyl, cyclopentyl, and cyclohexyl) forms between two adjacent carbon atoms of the C₁₀-C₃₀ linear alkyl.

In some embodiments, R₁ is selected from the group consisting of C₁₄-C₂₄ linear alkyl (e.g., C₁₄, C₁₅, C₁₆, C₁₇, C₁₈, C₁₉, C₂₀, C₂₁, C₂₂, C₂₃, or C₂₄ linear alkyl), and C₁₄-C₂₄ linear alkyl interrupted by one or more O or S atoms (e.g., C₁₄, C₁₅, C₁₆, C₁₇, C₁₈, C₁₉, C₂₀, C₂₁, C₂₂, C₂₃, or C₂₄ linear alkyl).

In some specific embodiments, R₁ is selected from the group consisting of: 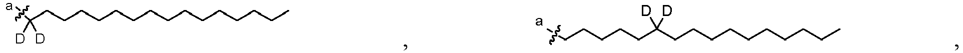 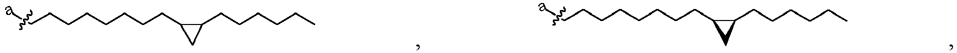 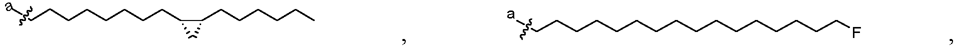 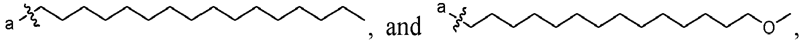 wherein end a is linked to X₂.

In some embodiments, each Rₐ is independently selected from the group consisting of hydrogen, deuterium, halogen (e.g., fluorine, chlorine, or bromine), C₁-₆ alkyl (e.g., C₁, C₂, C₃, C₄, C₅, or C₆ alkyl, including but not limited to methyl, ethyl, and isopropyl), and C₁-₆ alkoxy (e.g., C₁ alkoxy, C₂ alkoxy, C₃ alkoxy, C₄ alkoxy, C₅ alkoxy, or C₆ alkoxy, including but not limited to methoxy, ethoxy, propoxy, and isopropoxy).

In some embodiments, each Rₐ is independently selected from the group consisting of hydrogen, deuterium, fluorine, methyl, and methoxy.

In some embodiments, B is selected from the group consisting of adenine, guanine, cytosine, uracil, and thymine.

In some embodiments, the compound represented by formula (II) or the pharmaceutically acceptable salt thereof is selected from the group consisting of:

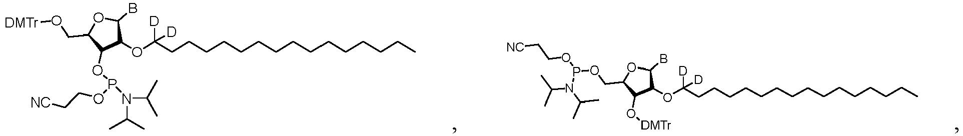

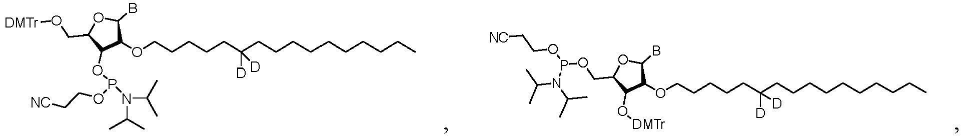

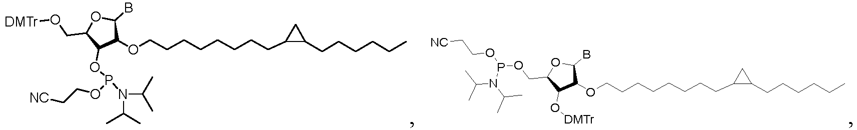

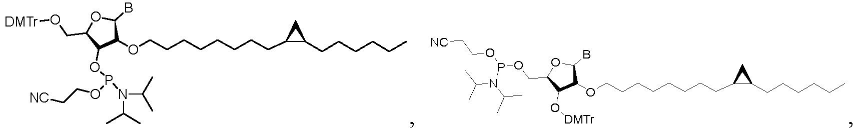

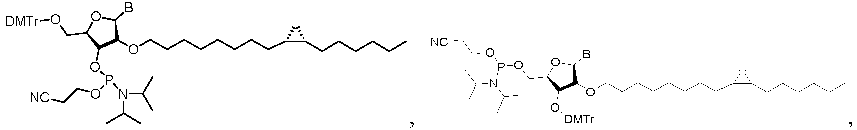

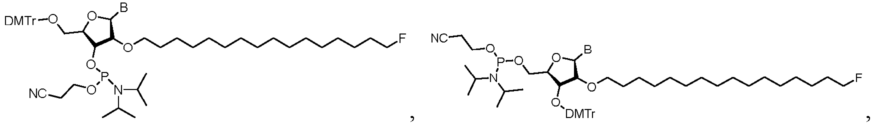

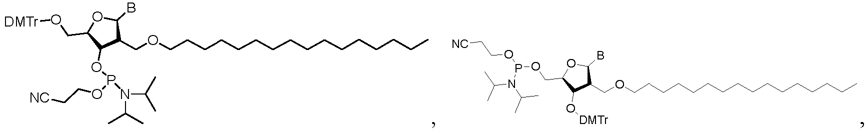

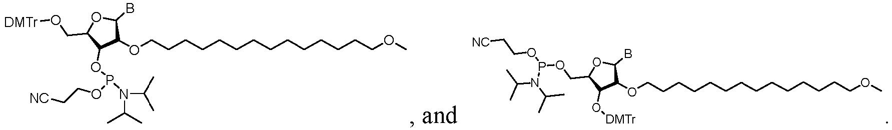

The present disclosure further provides a method for preparing the oligonucleotide of the present disclosure, the method comprising the following steps:
(1) synthesizing the compound represented by formula (II) or the pharmaceutically acceptable salt thereof of the present disclosure; and
(2) using the compound represented by formula (II) or the pharmaceutically acceptable salt thereof synthesized in step (1) to synthesize the oligonucleotide of the present disclosure.

In another aspect, the present disclosure provides a pharmaceutical composition comprising the oligonucleotide described above in the present disclosure.

In some embodiments, the pharmaceutical composition further comprises one or more pharmaceutically acceptable excipients.

In some embodiments, the pharmaceutically acceptable excipients may be, for example, a carrier, a vehicle, a diluent, and/or a delivery polymer.

In some embodiments, the oligonucleotide may be present in an amount of 0.01-99.99%, or 0.1-99.9%, or 0.5%-99.5%, further 1%-99%, and still further 2%-98%, based on the total weight of the pharmaceutical composition.

In some embodiments, the pharmaceutically acceptable excipients may be present in an amount of 0.01-99.99%, or 0.1-99.9%, or 0.5%-99.5%, further 1%-99%, and still further 2%-98%, based on the total weight of the pharmaceutical composition.

In another aspect, the present disclosure provides a method for inhibiting expression of a target gene, the method comprising administering to a subject an effective amount or effective dose of the oligonucleotide of the present disclosure or a pharmaceutical composition comprising the oligonucleotide. In some embodiments, the target gene is one or more target genes selected from the group consisting of MUC5B, APP, PMP22, and MAPT.

In some embodiments, the oligonucleotide or pharmaceutical composition may be in a therapeutically effective amount.

In some embodiments, a unit dose of the oligonucleotide or pharmaceutical composition may be 0.001 mg-1000 mg.

In some embodiments, the effective amount or effective dose of the oligonucleotide or pharmaceutical composition is about 0.001 mg/kg body weight to about 200 mg/kg body weight, about 0.01 mg/kg body weight to about 100 mg/kg body weight, or about 0.5 mg/kg body weight to about 50 mg/kg body weight.

In another aspect, the present disclosure provides use of the oligonucleotide described above or the pharmaceutical composition described above in the manufacture of a medicament.

In some embodiments, the medicament is used for preventing and/or treating diseases related to lung, eye, and central nervous system (CNS) disorders; in some embodiments, the medicament is used for preventing and/or treating lung-related diseases.

In some embodiments, the medicament is used for preventing and/or treating tumor-related diseases.

In another aspect, the present disclosure provides a method for delivering an oligonucleotide extrahepatically, the method comprising administering to a subject an effective amount or effective dose of the oligonucleotide or pharmaceutical composition of the present disclosure.

In some embodiments, the oligonucleotide of the present disclosure is delivered into the lungs, eyes, or CNS.

In some embodiments, the oligonucleotide of the present disclosure is delivered to the lungs.

In some embodiments, the oligonucleotide of the present disclosure is delivered to a tumor.

In some embodiments, the oligonucleotide of the present disclosure is delivered to pancreatic cancer, prostate cancer, breast cancer, or lung cancer.

In the present disclosure, when the oligonucleotide or pharmaceutical composition described above is in contact with a cell expressing the target gene, the oligonucleotide or pharmaceutical composition described above inhibits expression of the target gene by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In the present disclosure, when the oligonucleotide or pharmaceutical composition described above is in contact with a cell expressing the target gene, the oligonucleotide or pharmaceutical composition described above results in a target gene mRNA remaining expression percentage of no more than 99%, no more than 95%, no more than 90%, no more than 85%, no more than 80%, no more than 75%, no more than 70%, no more than 65%, no more than 60%, no more than 55%, no more than 50%, no more than 45%, no more than 40%, no more than 35%, no more than 30%, no more than 25%, no more than 20%, no more than 15%, or no more than 10%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In the present disclosure, when the oligonucleotide or pharmaceutical composition described above is in contact with a cell expressing the target gene, the oligonucleotide reduces off-target activity by at least 20%, at least 25%, at least 30%, at least 35%, at least 40%, at least 45%, at least 50%, at least 55%, at least 60%, at least 65%, at least 70%, or at least 75%, while maintaining on-target activity, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In the present disclosure, when the oligonucleotide or pharmaceutical composition described above is in contact with a cell expressing the target gene, the oligonucleotide reduces off-target activity by at least 20%, at least 25%, at least 30%, at least 35%, at least 40%, at least 45%, at least 50%, at least 55%, at least 60%, at least 65%, at least 70%, or at least 75%, while reducing on-target activity by at most 20%, at most 19%, at most 15%, at most 10%, at most 5%, or more than 1%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In the present disclosure, when the dsRNA or pharmaceutical composition described above is in contact with a cell expressing the target gene, the oligonucleotide reduces off-target activity by at least 20%, at least 25%, at least 30%, at least 35%, at least 40%, at least 45%, at least 50%, at least 55%, at least 60%, at least 65%, at least 70%, or at least 75%, while increasing on-target activity by at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, or at least 80%, as measured by, for example, psiCHECK activity screening and luciferase reporter gene assay, other mthods such as PCR or branched DNA (bDNA)-based methods, or protein-based methods such as immunofluorescence assay, e.g., western blot or flow cytometry.

Where the configuration is not specified, the compounds of the present disclosure can be present in specific geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures, and other mixtures thereof, such as enantiomerically or diastereomerically enriched mixtures, all of which are within the scope of the present disclosure. Additional asymmetric carbon atoms may be present in substituents such as an alkyl group. All such isomers and mixtures thereof are included within the scope of the present disclosure.

The compounds of the present disclosure can be asymmetric; for example, the compounds have one or more stereoisomers. Where the configuration is not specified, all stereoisomers include, for example, enantiomers and diastereomers. The compounds of the present disclosure containing asymmetric carbon atoms may be separated in optically active pure form or racemic form. The optically active pure form may be isolated from a racemic mixture or synthesized using chiral starting materials or chiral reagents.

Optically active (R)- and (S)-enantiomers, and D- and L-isomers may be prepared by chiral synthesis methods, using chiral reagents, or other conventional techniques in the art. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. In addition, the separation of enantiomers and diastereomers is generally accomplished by chromatography using a chiral stationary phase, optionally in combination with chemical derivatization (e.g., carbamate formation from amines).

The present disclosure also includes isotopically labeled compounds that are identical to those recited herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H , ³H , ¹¹C, ¹³C , ¹⁴C , ¹³N, ¹⁵N, ¹⁵O, ¹⁷O, ¹⁸O, ³¹P, ³²P, ³⁵S , ¹⁸F , ¹²³I, ¹²⁵I, and ³⁶Cl.

Unless otherwise specified, when a position is specifically assigned deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure further comprises various deuterated forms of the compounds. Each available hydrogen atom linked to a carbon atom may be independently replaced by a deuterium atom. Those skilled in the art are able to synthesize the deuterated forms of the compound of general formula I with reference to the relevant literature. Commercially available deuterated starting materials can be used in preparing the deuterated forms of the compound of formula I, or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, etc.

In the chemical structures of the compounds of the present disclosure, a bond " 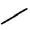 " represents an unspecified configuration; that is, if chiral isomers exist in the chemical structures, the bond " 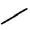 " may be " 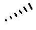 " or " 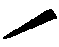 ", or both the configurations of " 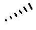 " and " 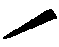 " are included simultaneously. Although all of the above structural formulas are drawn as certain isomeric forms for the sake of simplicity, the present disclosure may include all isomers, such as tautomers, rotamers, geometric isomers, diastereomers, racemates, and enantiomers. In the chemical structure of the compound of the present disclosure, a bond " 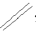 " does not specify a configuration; that is, the configuration for the bond " 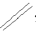 " can be an E configuration or a Z configuration, or includes both the E configuration and the Z configuration. 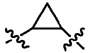 may be 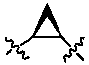 or

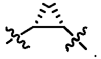

### Terms and Definitions

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains.

As used herein, "RNAi agent" refers to an agent that contains an RNA or RNA-like (e.g., chemically modified RNA) oligonucleotide molecule that is capable of degrading or inhibiting transcription and translation of a target messenger RNA (mRNA) in a sequence-specific manner. In the present disclosure, RNAi agents may operate through the RNA interference mechanism (i.e., inducing RNA interference through interaction with the RNA interference pathway machinery (RNA-induced silencing complex or RISC) of mammalian cells), or act by any other mechanisms or pathways. RNAi agents include, but are not limited to: single-stranded oligonucleotides, single-stranded antisense oligonucleotides, short interfering RNAs (siRNAs), double-stranded RNAs (dsRNAs), microRNAs (miRNAs), short hairpin RNAs (shRNAs), and Dicer substrates.

The RNAi agents of the present disclosure comprise an oligonucleotide having a strand that is at least partially complementary to the targeted mRNA.

In the present disclosure, the "5' region" of the sense or antisense strand, i.e., the "5' end" or "5' terminus", is used interchangeably. For example, the nucleotides at positions 2 to 8 of the 5' region of the antisense strand may be replaced with the nucleotides at positions 2 to 8 of the 5' end of the antisense strand. Likewise, the "3' region", "3' terminus", and "3' end" of the sense or antisense strand are also used interchangeably.

"Position 6 in the direction from the 5' end to the 3' end of the sense strand" and "position 6 of the 5' end of the sense strand" are used interchangeably. For example, in 5' N_{A}NNNNN_{C}N NNNNNNNNNNNNNN_{B} 3', when the compound represented by formula (I) or the pharmaceutically acceptable salt thereof is located at position 1 of the 5' end of the sense strand, N_{A} represents the compound represented by formula (I) or the pharmaceutically acceptable salt thereof; when the compound represented by formula (I) or the pharmaceutically acceptable salt thereof is located at position 1 of the 3' end of the sense strand, N_{B} represents the compound represented by formula (I) or the pharmaceutically acceptable salt thereof, when the compound represented by formula (I) or the pharmaceutically acceptable salt thereof is located at position 6 in the direction from the 5' end to the 3' end of the sense strand, N_{C} represents the compound represented by formula (I) or the pharmaceutically acceptable salt thereof.

In the present disclosure, "one end is linked to the nucleotide at position 5 of the 5' end of the sense strand, and the other end is linked to the nucleotide at position 7 of the 5' end of the sense strand" is intended to indicate the position of the compound represented by formula (I) or the pharmaceutically acceptable salt thereof of the present disclosure. For example, in 5' NNNNN₅N₆N₇NNNNNNNNNNNNNN3', the end b in formulas (I-1) and (I-2) is linked to N₅, and the end c is linked to N₇; the B in formulas (I-1) and (I-2) represents the base of N₆. For example, in 5' N₁N₂NNNNNNNNNNNNNNNNNNN 3', the linking end 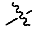 in formulas (I-3) and (I-4) is linked to N₂, and the B in formulas (I-3) and (I-4) represents the base of N₁. For example, in 5' NNNNNNNNNNNNNNNNNNNN_{2'}N_{1'} 3', the linking end 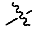 in formulas (I-5) and (I-6) is linked to N_{2'}, and the B in formulas (I-5) and (I-6) represents the base of N_{1'}.

"Optionally" or "optional" means that the event or circumstance subsequently described may, but does not necessarily, occur, and this description includes instances where the event or circumstance occurs or does not occur. For example, "C₁₋₆ alkyl optionally substituted with halogen or cyano" means that the halogen or cyano may, but does not necessarily, exist, and this description includes an instance where the alkyl is substituted with halogen or cyano and an instance where the alkyl is not substituted with halogen or cyano.

The term "lipophilic monomer" or "lipophilic moiety" broadly refers to any compound or chemical moiety that has an affinity for lipids. One way to characterize the lipophilicity of a lipophilic moiety is by the octanol-water partition coefficient, logK_{ow}, where K_{ow} is the ratio of the concentration of a chemical substance in the octanol phase to its concentration in the aqueous phase in a biphasic system in equilibrium. In principle, a chemical substance is lipophilic when its logK_{ow} exceeds 0. Typically, a lipophilic moiety has a logK_{ow} exceeding 1, exceeding 1.5, exceeding 2, exceeding 3, exceeding 4, exceeding 5, or exceeding 10; for example, 6-aminohexanol has a logK_{ow} of about 0.7, and cholesteryl N-(hexan-6-ol)carbamate has a logK_{ow} of 10.7.

The lipophilicity of a molecule may vary relative to the functional groups it carries. For example, adding hydroxy or an amine group to an end of a lipophilic moiety may increase or decrease the partition coefficient (e.g., logK_{ow}) value of the lipophilic moiety. For example, a lipophilic moiety may be an aliphatic, cyclic such as alicyclic, or polycyclic such as polyalicyclic compounds, such as steroids (e.g., sterols) or linear or branched aliphatic hydrocarbons. A lipophilic moiety may generally contain a hydrocarbon chain, and the hydrocarbon chain may be cyclic or acyclic. The hydrocarbon chain may contain various substituents and/or one or more heteroatoms, such as oxygen or sulfur atoms. Such lipophilic aliphatic moieties include, but are not limited to, saturated or unsaturated C₄-C₃₀ hydrocarbons (e.g., C₁₀-C₃₀ hydrocarbons), saturated or unsaturated fatty acids, waxes (e.g., fatty acids and monohydric alcohol esters of fatty diamides), terpenes (e.g., C₁₀ terpenes, C₁₅ sesquiterpenes, C₂₀ diterpenes, C₃₀ triterpenes, and C₄₀ tetraterpenes), and other polyalicyclic hydrocarbons; for example, a lipophilic moiety may be an optionally substituted C₁₀₋₃₀ linear alkyl group; for example, a lipophilic moiety may be an optionally substituted C₁₄₋₂₄ linear alkyl group.

The term "pharmaceutically acceptable salt" includes pharmaceutically acceptable acid addition salts and pharmaceutically acceptable base addition salts.

"Pharmaceutically acceptable acid addition salt" refers to salts that are capable of retaining the biological effectiveness of free bases without having any undesirable effects and that are formed with inorganic or organic acids. Inorganic acid salts include, but are not limited to, hydrochlorides, hydrobromides, sulfates, nitrates, phosphates, etc.; organic acid salts include, but are not limited to, formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, mesylates, benzenesulfonates, p-toluenesulfonates, alginates, ascorbates, salicylates, 4-aminosalicylates, napadisylates, etc. These salts can be prepared using methods known in the art.

"Pharmaceutically acceptable base addition salt" refers to salts that are capable of retaining the biological effectiveness of free acids without having any undesirable effects and that are formed with inorganic bases or organic bases. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. Preferred inorganic salts are ammonium salts, sodium salts, potassium salts, calcium salts, and magnesium salts; sodium salts are preferred. Salts derived from organic bases include, but are not limited to, salts of the following: primary, secondary, and tertiary amines, substituted amines including naturally occurring substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, etc. Preferred organic bases include isopropylamine, diethylamine, ethanolamine, trimethylamine, dicyclohexylamine, choline, and caffeine. These salts can be prepared using methods known in the art.

The term "alkyl" refers to a saturated aliphatic hydrocarbon group which is a linear or branched group containing 1 to 30 carbon atoms; in some embodiments, alkyl is selected from the group consisting of alkyl groups containing 10 to 30 carbon atoms. In some embodiments, alkyl is selected from the group consisting of alkyl groups containing 10 to 20 carbon atoms, e.g., from the group consisting of alkyl groups of 14, 15, 16, 17, 18, 19, or 20 carbon atoms. Non-limiting examples include tetradecane, hexadecane, octadecane, eicosane, etc. In some embodiments, alkyl is selected from the group consisting of alkyl groups containing 1 to 6 carbon atoms; non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl may be substituted or unsubstituted, and when it is substituted, the substituent can be substituted at any available linking point; the substituent is selected, in some embodiments, from one or more of the following groups, which are independently selected from the group consisting of alkyl, alkoxy, halogen, hydroxy, cyano, cycloalkyl, and heterocycloalkyl; the alkyl is optionally substituted with halogen. For example, alkyl may be a linear group containing 1 to 30 carbon atoms, including but not limited to C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, and C₃₀ linear alkyl groups.

The term "alkoxy" refers to -O-(alkyl), wherein the alkyl is as defined above. Non-limiting examples of alkoxy include: methoxy, ethoxy, propoxy, and butoxy. Alkoxy may be optionally substituted or unsubstituted, and when it is substituted, the substituent is preferably one or more of the following groups, which are independently selected from the group consisting of halogen, hydroxy, oxo, cyano, amino, C₁-₆ alkyl, C₁-₆ alkoxy, 3- to 7-membered cycloalkyl, and 3- to 7-membered heterocycloalkyl; the alkyl, alkoxy, cycloalkyl, or heterocycloalkyl is optionally substituted with halogen, hydroxy, nitro, cyano, or amino.

Likewise, the definitions of "cycloalkoxy" and "heterocycloalkoxy" are similar to the above definition of "alkoxy".

The term "cycloalkyl" or "carbocycle" refers to a saturated or partially unsaturated, monocyclic or polycyclic hydrocarbon substituent; the cycloalkyl ring contains 3 to 20 carbon atoms and is selected, in some embodiments, from the group consisting of cycloalkyl rings containing 3 to 7 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, etc. Polycyclic cycloalkyl groups include spiro-ring, fused-ring, and bridged-ring cycloalkyl groups. Cycloalkyl may be substituted or unsubstituted, and when it is substituted, the substituent may be substituted at any available linking point and is selected, in some embodiments, from the group consisting of one or more of the following groups, which are independently selected from the group consisting of halogen, deuterium, hydroxy, oxo, nitro, cyano, C₁₋₆ alkyl, C₁₋₆ alkoxy, C₃-₆ cycloalkoxy, and 3- to 6-membered heterocycloalkoxy; the C₁₋₆ alkyl, C₁₋₆ alkoxy, C₃₋₆ cycloalkoxy, or 3- to 6-membered heterocycloalkoxy is optionally substituted with one or more groups selected from the group consisting of halogen, deuterium, hydroxy, oxo, nitro, and cyano.

The cycloalkyl ring may be fused to an aryl or heteroaryl ring, wherein the ring linked to the parent structure is a cycloalkyl group; non-limiting examples include indanyl, tetrahydronaphthyl, benzocycloheptyl, etc. Cycloalkyl may be optionally substituted or unsubstituted, and when it is substituted, the substituent is selected, in some embodiments, from the group consisting of one or more of the following groups, which are independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl; the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, or 5- to 6-membered aryl or heteroaryl is optionally substituted with one or more groups selected from the group consisting of halogen, deuterium, hydroxy, oxo, nitro, and cyano.

The term "heterocycloalkyl" or "heterocycle" or "heterocyclyl" refers to a saturated or partially unsaturated, monocyclic or polycyclic hydrocarbon substituent containing 3 to 20 ring atoms, one or more of which are heteroatoms selected from the group consisting of nitrogen, oxygen, and S(O)ₘ (where m is an integer of 0 to 2), but a ring moiety of -O-O-, -O-S-, or -S-S- is excluded, and the other ring atoms are carbon atoms. In some embodiments, heterocycloalkyl is selected from the group consisting of heterocycloalkyl groups containing 3 to 12 ring atoms, 1-4 of which are heteroatoms; in some embodiments, heterocycloalkyl is selected from the group consisting of heterocycloalkyl groups containing 3 to 7 ring atoms. Non-limiting examples of monocyclic heterocycloalkyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, etc. Polycyclic heterocycloalkyl groups include spiro-ring, fused-ring, and bridged-ring heterocycloalkyl groups. Non-limiting examples of "heterocycloalkyl" include: 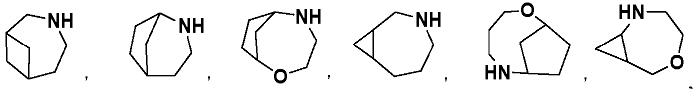 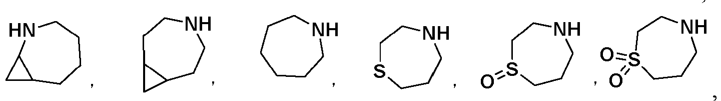 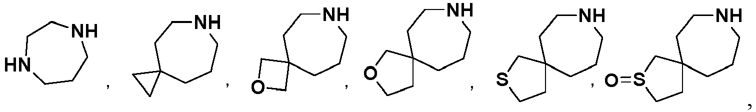 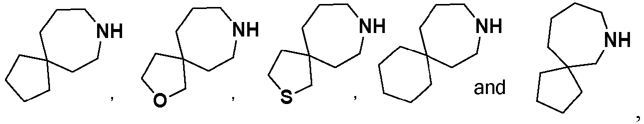 etc.

The heterocyclyl ring may be fused to an aryl, heteroaryl, or cycloalkyl ring, wherein the ring linked to the parent structure is a heterocyclyl group; its non-limiting examples include: 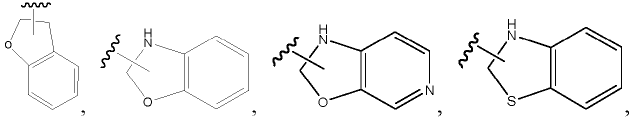 etc.

Heterocyclyl may be optionally substituted or unsubstituted, and when it is substituted, the substituent is preferably one or more of the following groups, which are independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, and a carboxylate group.

The term "aryl" refers to a 6- to 14-membered, preferably 6- to 10-membered, all-carbon monocyclic or fused polycyclic (i.e., rings that share a pair of adjacent carbon atoms) group having a conjugated π-electron system, such as phenyl and naphthyl. The aryl ring may be fused to a heteroaryl, heterocyclyl, or cycloalkyl ring, wherein the ring linked to the parent structure is the aryl ring. "Aromatic ring" refers to the ring system in aryl. Non-limiting examples of aryl include:

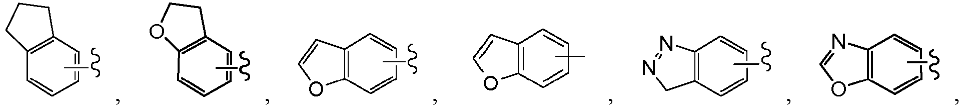

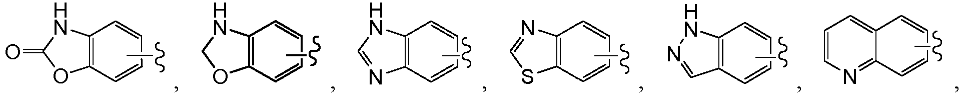

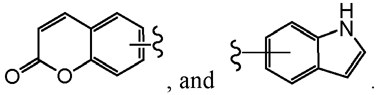

Aryl may be substituted or unsubstituted, and when it is substituted, the substituent is preferably one or more of the following groups, which are independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl, and a carboxylate group, preferably from the group consisting of phenyl.

The term "fused-ring aryl" may be an unsaturated aromatic fused-ring structure containing 8-14 ring atoms, preferably 8-12 ring atoms, formed by connecting two or more ring structures that share two adjacent atoms with each other, including, for example, all unsaturated fused-ring aryl groups such as naphthalene and phenanthrene, as well as partially saturated fused-ring aryl groups such as benzo 3- to 8-membered saturated monocyclic cycloalkyl groups and benzo 3- to 8-membered partially saturated monocyclic cycloalkyl groups. "Fused aromatic ring" refers to the ring system in fused-ring aryl. Specific examples of fused-ring aryl include 2,3-dihydro-1H-indenyl, IH-indenyl, 1,2,3,4-tetrahydronaphthyl, 1,4-dihydronaphthyl, etc.

The term "heteroaryl" refers to a heteroaromatic system containing 1 to 4 heteroatoms and 5 to 14 ring atoms, wherein the heteroatoms are selected from the group consisting of oxygen, sulfur, and nitrogen. Heteroaryl is preferably 5- to 12-membered, e.g., imidazolyl, furyl, thienyl, thiazolyl, pyrazolyl, oxazolyl, pyrrolyl, tetrazolyl, pyridyl, pyrimidinyl, thiadiazole, or pyrazinyl, preferably imidazolyl, pyrazolyl, pyrimidinyl, or thiazolyl, and more preferably pyrazolyl or thiazolyl. The heteroaryl ring may be fused to an aryl, heterocyclyl, or cycloalkyl ring, wherein the ring linked to the parent structure is the heteroaryl ring. "Heteroaromatic ring" refers to the ring system in heteroaryl. Non-limiting examples of heteroaryl include:

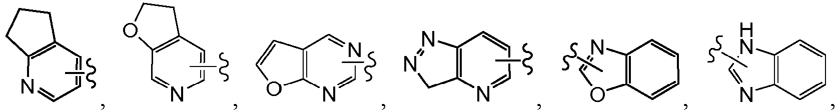

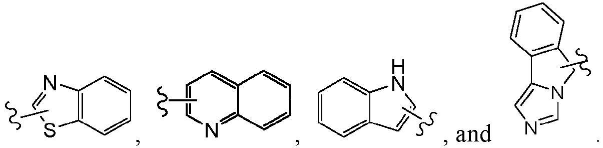

Heteroaryl may be optionally substituted or unsubstituted, and when it is substituted, the substituent is selected, in some embodiments, from the group consisting of one or more of the following groups, which are independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl; the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, or 5- to 6-membered aryl or heteroaryl is optionally substituted with one or more groups selected from the group consisting of halogen, deuterium, hydroxy, oxo, nitro, and cyano.

The term "hydroxyalkyl" refers to an alkyl group substituted with one or more hydroxy groups, wherein the alkyl group is as defined above.

The term "hydroxy" refers to the -OH group.

The term "halogen" refers to fluorine, chlorine, bromine, or iodine.

The term "haloalkyl" refers to an alkyl group substituted with halogen, wherein the alkyl group is as defined above.

The term "haloalkoxy" refers to an alkoxy group substituted with halogen, wherein the alkoxy group is as defined above.

The term "cyano" refers to -CN.

The term "nitro" refers to -NO₂.

The term "oxo" refers to the =O group; for example, a carbon atom is linked to the oxygen atom by a double bond, resulting in a keto or aldehyde group.

The term "amino" refers to -NH₂.

The term "carboxyl" refers to -C(O)OH.

The term "substituted" means that any one or more hydrogen atoms on the specified atom (usually a carbon, oxygen, or nitrogen atom) are replaced with any group as defined herein, provided that the normal valency of the specified atom is not exceeded and that the substitution results in a stable compound. Non-limiting examples of substituents include C₁-C₆ alkyl, C₂-C₆ alkenyl, C₂-C₆ alkynyl, cyano, hydroxy, oxo, carboxyl, cycloalkyl, cycloalkenyl, heterocyclyl, heteroaryl, aryl, ketones, alkoxycarbonyl, aryloxycarbonyl, heteroaryloxycarbonyl, or halogen (e.g., F, Cl, Br, or I). When the substituent is a ketone or oxo (i.e., =O), two (2) hydrogen atoms on the atom are replaced.

"Substituted with one or more" means that it may be substituted with a single substituent or multiple substituents. In the case of substitution with multiple substituents, there may be a plurality of identical substituents, or one or a combination of a plurality of different substituents.

In the chemical structural formulas of the present disclosure, the wavy line " 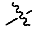 " represents a linking site.

The term "link", when referring to a relationship between two molecules, means that the two molecules are linked by a covalent bond or that the two molecules are associated via a non-covalent bond (e.g., a hydrogen bond or an ionic bond), and includes direct linkage and indirect linkage.

The term "direct linkage" means that a first compound or group is linked to a second compound or group without any atom or group of atoms interposed between.

The term "indirect linkage" means that a first compound or group is linked to a second compound or group by an intermediate group, a compound, or a molecule (e.g., a linking group).

As used herein, in the context of RNA-mediated gene silencing, the sense strand (also referred to as SS or SS strand) of an siRNA refers to a strand that comprises a sequence that is identical or substantially identical to a target mRNA sequence; the antisense strand (also referred to as AS or AS strand) of an siRNA refers to a strand having a sequence complementary to a target mRNA sequence.

The term "base" encompasses any known DNA and RNA bases and base analogs such as purines or pyrimidines, and also includes the natural compounds adenine, thymine, guanine, cytosine, uracil, inosine, and natural analogs.

As used herein, "chemical modification" or "modification" includes all changes made to a nucleotide by chemical means, such as the addition or removal of a chemical moiety, or the substitution of one chemical moiety for another.

As used herein, the term "fluoro-modified nucleotide" refers to a nucleotide in which the hydroxy group at the 2' position of the ribosyl group of the nucleotide is substituted with fluorine, and "non-fluoro-modified nucleotide" refers to a nucleotide or a nucleotide analog in which the hydroxy group at the 2' position of the ribosyl group of the nucleotide is substituted with a non-fluorine group; "nucleotide analog" refers to a group that can substitute for a nucleotide in a nucleic acid but differs in structure from adenine ribonucleotide, guanine ribonucleotide, cytosine ribonucleotide, uracil ribonucleotide, or thymine deoxyribonucleotide, e.g., an isonucleotide, a bridged nucleic acid (BNA for short), or an acyclic nucleotide. The methoxy-modified nucleotide refers to a nucleotide in which the 2'-hydroxy group of the ribosyl group is substituted with a methoxy group. An isonucleotide refers to a compound formed by changing the position of a base on the ribose ring in a nucleotide. In some embodiments, the isonucleotide can be a compound formed by moving a base from the 1'-position to the 2'-position or 3'-position of the ribose ring. BNA refers to a constrained or inaccessible nucleotide. BNA may contain five-membered, six-membered, or seven-membered ring bridged structure with a "fixed" C3'-endo sugar puckering. The bridge is typically incorporated at the 2'-, 4'-position of the ribose to afford a 2',4'-BNA nucleotide. In some embodiments, BNA may be LNA, ENA, cET BNA, etc. Acyclic nucleotides are a class of nucleotides in which the sugar ring of the nucleotide is opened. In some embodiments, the acyclic nucleotide can be an unlocked nucleic acid (UNA) or a glycerol nucleic acid (GNA).

As used herein, the terms "complementary" and "reverse complementary" are used interchangeably and have the meaning well known to those skilled in the art; that is, in a double-stranded nucleic acid molecule, the bases of one strand are paired with the bases of the other strand in a complementary manner. In DNA, the purine base adenine (A) is always paired with the pyrimidine base thymine (T) (or uracil (U) in RNA), and the purine base guanine (C) is always paired with the pyrimidine base cytosine (G). Each base pair comprises a purine and a pyrimidine. When adenines of one strand are always paired with thymines (or uracils) of another strand and guanines are always paired with cytosines, the two strands are considered complementary to each other, and the sequences of the strands can be deduced from the sequences of their complementary strands. Accordingly, "mismatch" in the art means that in a double-stranded nucleic acid, the bases at the corresponding positions are not paired in a complementary manner.

Unless otherwise stated, in the context of the present disclosure, the uppercase letters C, G, U, A, and T represent base components of a nucleotide; the lowercase letter d indicates that the adjacent nucleotide to the right of the letter d is a deoxyribonucleotide; the lowercase letter m indicates that the adjacent nucleotide to the left of the letter m is a methoxy-modified nucleotide; the lowercase letter f indicates that the adjacent nucleotide to the left of the letter f is a fluoro-modified nucleotide; the lowercase letter s indicates that the two adjacent nucleotides flanking the letter s are linked by a phosphorothioate group; the uppercase letters VP indicate that the two adjacent nucleotides flanking the letters VP are linked by a vinyl phosphate linkage, and if VP is located at the 5' end of a sequence, it indicates that the adjacent nucleotide to the right of the letters VP is linked by a vinyl phosphate linkage.

"Pharmaceutical composition" comprises the oligonucleotide or double-stranded RNAi inhibitor molecule of the present disclosure and a pharmaceutically acceptable auxiliary material and/or adjuvant; the auxiliary material may be one or more of various formulations or compounds conventionally used in the art. For example, the pharmaceutically acceptable auxiliary material can include at least one of a pH buffer, a protective agent, and an osmotic pressure regulator.

As used herein, the term "inhibit" is used interchangeably with "decrease", "silence", "down-regulate", "repress", or other similar terms, and includes any level of inhibition.

The terms "blunt end" and "blunt ended" are used interchangeably and mean that there are no unpaired nucleotides or nucleotide analogs at a given terminus of an siRNA, i.e., no nucleotide overhang. In most cases, an siRNA both ends of which are blunt ended will be double-stranded over its entire length.

The terms "about" and "approximately" mean that a numerical value is within an acceptable error range for the specific value determined by those of ordinary skill in the art, and the numerical value depends in part on how the value is measured or determined (i.e., the limitations of the measurement system). For example, "about" may mean a standard deviation within 1 or more than 1. Alternatively, "about" or "comprising essentially" may mean a range of at most 20%, e.g., a change of between 1% and 15%, between 1% and 10%, between 1% and 5%, between 0.5% and 5%, or between 0.5% and 1%. In the present disclosure, every instance where a number or numerical range is preceded by the term "about" also includes an embodiment of the given number. Unless otherwise stated, when a specific value is provided in the present application and claims, the meaning of "about" or "substantially comprise" should be assumed to be within an acceptable error range for that specific value.

"Effective amount" or "effective dose" refers to the amount of a drug, a compound, or a pharmaceutical composition necessary to obtain any one or more beneficial or desired therapeutic results. For preventive use, the beneficial or desired results include elimination or reduction of risk, reduction of severity or delay of the onset of a disorder, including the biochemistry, histology, and/or behavioral symptoms of the disorder, complications thereof, and intermediate pathological phenotypes that appear during the progression of the disorder.

For therapeutic applications, the beneficial or desired results include clinical results, such as reducing the incidence of various disorders related to the target gene, target mRNA, or target protein of the present disclosure or ameliorating one or more symptoms of the disorders, reducing the dose of other agents required to treat the disorders, enhancing the therapeutic effect of another agent, and/or delaying the progression of disorders related to the target gene, target mRNA, or target protein of the present disclosure in patients.

In some embodiments, an effective amount or effective dose of siRNA is about 0.001 mg/kg body weight to about 200 mg/kg body weight, about 0.01 mg/kg body weight to about 100 mg/kg body weight, or about 0.5 mg/kg body weight to about 50 mg/kg body weight.

As used herein, "object", "patient", "subject", and "individual" are used interchangeably and include human or non-human animals, e.g., mammals, e.g., humans or monkeys.

The siRNA provided by the present disclosure can be obtained using a preparation method conventional in the art (e.g., solid-phase synthesis and liquid-phase synthesis). Solid-phase synthesis has been commercially available as a customization service. A modified nucleotide group can be introduced into the siRNA of the present disclosure using a nucleoside monomer with a corresponding modification. Methods for preparing the nucleoside monomer with the corresponding modification and introducing the modified nucleotide group into the siRNA are also well known to those skilled in the art.

Some abbreviations in the present disclosure are defined as follows:
DCE: dichloroethane;
MeOH: methanol;
PE: petroleum ether;
EtOAc: ethyl acetate;
THF: tetrahydrofuran;
DMF: dimethylformamide;
TFA: trifluoroacetic acid;
LiAlH₄: lithium aluminum hydride;
LiAlD₄: deuterated lithium aluminum hydride;
TsCl: p-toluenesulfonyl chloride;
DMAP: 4-dimethylaminopyridine;
MeCN: acetonitrile;
NMI: N-methylimidazole;
TBDPSCl: tert-butyl(chloro)diphenylsilane;
HoBt: 1-hydroxybenzotriazole;
EDCI: 1-ethyl-(3-dimethylaminopropyl)carbodiimide hydrochloride;
DIEA: N,N-diisopropylethylamine;
DMTrCl: 4,4'-dimethoxytrityl chloride;
DMTr: a dimethoxytrityl protecting group.

### BRIEF DESCRIPTION OF THE DRAWINGS

FIG. 1 shows mRNA expression levels in cells in the treatment groups of TJR100072, TJR100073, TJR100344, and TJR100345 at different concentrations, where *** indicates *p* < 0.001, and ** indicates *p* < 0.01.
FIG. 2 shows expression levels of mouse MUC5B mRNA for TJR100072 and TJR100073, where the data results are expressed as mean ± SEM.
FIG. 3 shows expression levels of mouse MUC5B mRNA for TJR100073, TJR100344, and TJR100345, where the data results are expressed as mean ± SEM, and *** indicates *p* < 0.001.
FIG. 4 shows expression levels of mouse MAPT mRNA for TJR101646 and TJR101647, where the data results are expressed as mean ± SEM; **** indicates *p* < 0.0001 relative to blank vehicle, and *** indicates *p* < 0.001 relative to blank vehicle.
FIG. 5 shows expression levels of mouse MAPT mRNA for TJR101740, where the data results are expressed as mean ± SEM; **** indicates *p* < 0.0001 relative to blank vehicle, and *** indicates *p* < 0.001 relative to blank vehicle.
FIG. 6 shows expression levels of mouse APP mRNA for TJR102160, TJR102161, and TJR102162, where the data results are expressed as mean ± SEM; **** indicates *p* < 0.0001 relative to blank vehicle, ** indicates *p* < 0.01 relative to blank vehicle, and #### indicates *p <* 0.0001 relative to TJR102160.

### DETAILED DESCRIPTION

The present disclosure is further described below with reference to examples, which are not intended to limit the scope of the present disclosure. Experimental procedures without specific conditions indicated in the examples of the present disclosure were generally conducted under conventional conditions or conditions recommended by the manufacturers of the starting materials or commercial products. Where the specific source of a reagent is not indicated, the reagent may be obtained from any supplier of reagents for molecular biology at a quality/purity level for application in molecular biology.

### Example

Unless otherwise specified, all reagents used in the following examples were commercially available.

### Example 1: Synthesis of Compounds with Lipid Delivery

### Example 1.1: Synthesis of Compound NA0108 (also referred to as "Uhd")

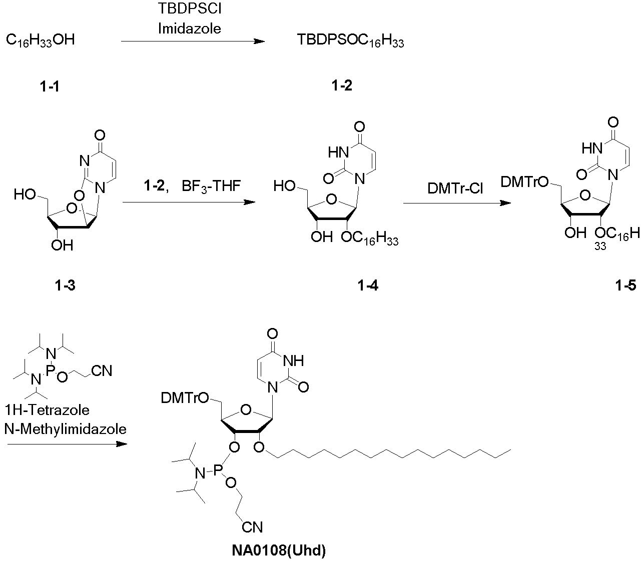

### Compound 1-2:

After compound 1-1 (7.00 g, 32.19 mmol) was dissolved in DMF (60 mL) under a nitrogen atmosphere, TDBPSCl (9.70 g, 35.41 mmol) and imidazole (2.40 g, 35.41 mmol) were added separately. The mixture was stirred at room temperature for 2 h. The reaction mixture was extracted twice with petroleum ether (60 mL), and the combined organic phases were washed 3-4 times with saturated brine, dried over Na₂SO₄, and concentrated under reduced pressure to give compound 1-2 (13.42 g, yield: 96.7%). The product was used in the next step without purification.

### Compound 1-4:

Compound 1-3 (2.50 g, 11.0 mmol) was added to a mixed solution of compound 1-2 (13.42 g, 27.9 mmol) in DMF (11.8 mL) and diglyme (7.9 mL). Boron trifluoride tetrahydrofuran complex (3.0 g) was slowly added dropwise to the mixed solution under a nitrogen atmosphere in an ice bath. Subsequently, the reaction mixture was heated to 140 °C and left to react for 16 h. The reaction mixture was cooled to room temperature, quenched by being poured into water (100 mL), and extracted with ethyl acetate (60 mL × 3). The combined organic phases were washed with saturated brine (100 mL), dried over Na₂SO₄, filtered, and concentrated, and the resulting residue was purified by alkalinized normal phase silica gel column chromatography (0.1% Et₃N in DCM:MeOH = 10:1, product peak at 5%) to give compound **1-4** (1.32 g, yield: 25.4%).

LCMS: t_{R} = 2.00 min, MS (ESI) m/z = 467.4 [M-H]⁻.

### Compound 1-5:

After compound **1-4** (1.02 g, 2.2 mmol) was dissolved in pyridine (8 mL) under a nitrogen atmosphere, 4A molecular sieve and DMTrCl (1.10 g, 3.2 mmol) were added at 0 °C, and the mixture was then warmed to room temperature and left to react for 16 h. The reaction mixture was quenched by being added to water (60 mL) and was extracted three times with ethyl acetate (40 mL). The combined organic phases were washed three times with saturated brine, dried over anhydrous sodium sulfate, and concentrated, and the resulting residue was purified by alkalinized normal phase silica gel column chromatography (0.1% Et₃N in PE:EtOAc = 1:1, product peak at 37%) to give compound **1-5** (1.02 g, yield: 62.6%).

LCMS: t_{R} = 2.37 min, MS (ESI) m/z = 769.7 [M-H]⁻.

¹H NMR (400 MHz, DMSO) δ ppm 8.00 (d, *J* = 8.0 Hz, 1H), 7.38 (d, *J* = 7.2 Hz, 2H), 7.37-7.18 (m, 7H), 6.84 (d, *J* = 8.8 Hz, 4H), 5.86 (s, 1H), 5.17 (s, 1H), 4.46-4.41 (m, 1H), 3.99-3.93 (m, 1H), 3.80-3.77 (m, 6H), 3.76-3.63 (m, 2H), 3.45 (d, J = 2.4 Hz, 2H), 1.62-1.54 (m, 2H), 1.35-1.26 (m, 26H), 0.91-0.87 (m, 3H).

### Compound NA0108:

3A molecular sieve was added to a solution of compound **1-5** (630 mg, 0.8 mmol) in dry acetonitrile (6 mL) at 0 °C under a nitrogen atmosphere. After 5 min of stirring, bis(diisopropylamino)(2-cyanoethoxy)phosphine (540 mg, 1.79 mmol) and a pre-prepared solution of 1H-tetrazole (0.45 M) and N-methylimidazole (0.18 M) in dry acetonitrile (1.8 mL) were added, and the mixture was then warmed to room temperature and left to react for 3 h. After the reaction mixture was filtered, a 5% aqueous sodium bicarbonate solution (20 mL) was added to the reaction mixture. The mixture was extracted twice with dichloromethane (40 mL). The combined organic phases were dried over sodium sulfate, filtered, and concentrated, and the resulting residue was purified by C18 reversed-phase column chromatography (H₂O/MeCN, from 0% to 100% elution) followed by lyophilization to give compound **NA0108** (also referred to as "Uhd") (475 mg, yield: 59.9%).

LCMS: t_{R} = 2.67 min, MS (ESI) m/z = 969.4 [M-H]⁻.

¹H NMR (400 MHz, DMSO-d*₆*) δ ppm 11.39 (s, 1H), 7.83-7.77 (m, 1H), 7.42-7.24 (m, 9H), 6.94-6.89 (m, 4H), 5.83-5.81 (m, 1H), 5.32-5.25 (m, 1H), 4.44-4.32 (m, 1H), 4.15-4.06 (m, 2H), 3.76 (s, 7.3H), 3.65-3.51 (m, 4.7H), 2.79 (t, *J* = 6.4 Hz, 0.7H), 2.65-2.58 (m, 1.3H), 1.51-1.49 (m, 2H), 1.24 (s, 28H), 1.16-1.11 (m, 10H), 1.01 (d, *J =* 6.8 Hz, 2H), 0.88 (d, *J =* 6.4 Hz, 3H).

³¹P NMR (100 MHz, DMSO-d₆) *δ* ppm 149.14, 148.63.

### Example 1.2: Synthesis of Compound NA0124

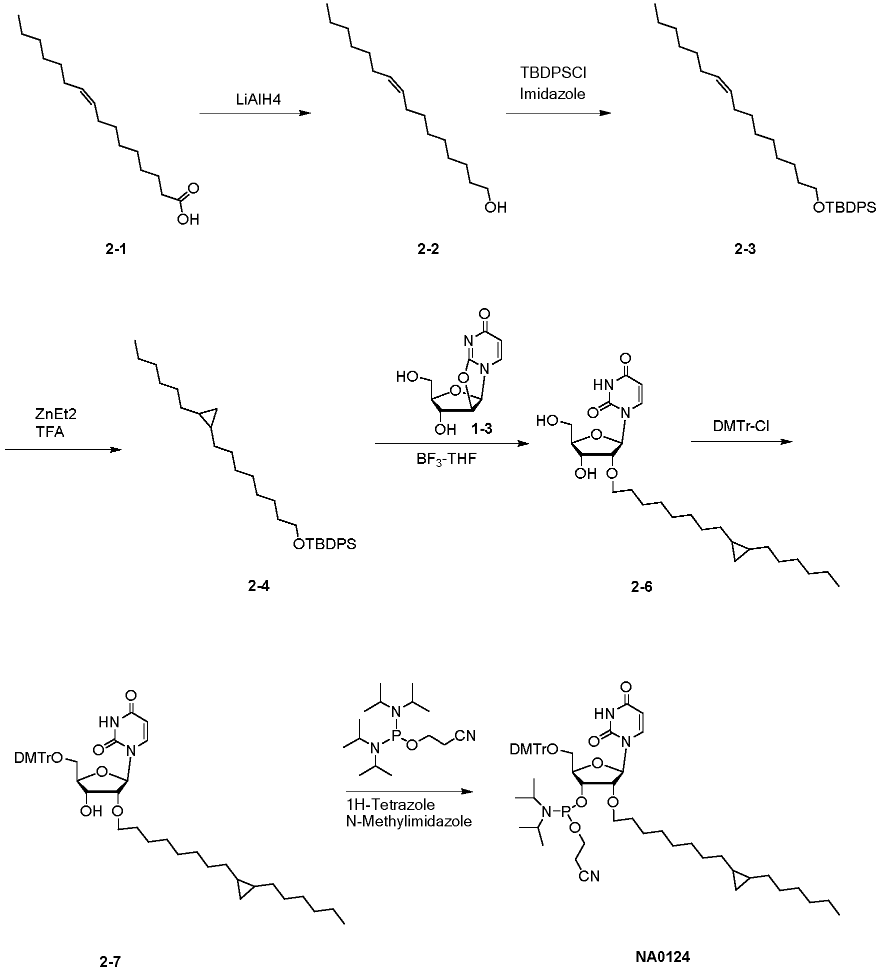

### Compound 2-2:

Under a nitrogen atmosphere in an ice bath, 15.7 mL of a 2.5 M solution of LiAlH₄ in THF was slowly added dropwise to a solution of compound 2-1 (10.0 g, 39.3 mmol) in dry THF (60 mL). Subsequently, the reaction mixture was warmed to room temperature and stirred for 2 h. The reaction mixture was quenched by addition of a saturated aqueous ammonium chloride solution in an ice bath and repeatedly extracted 3 times with ethyl acetate (100 mL). The combined organic phases were washed with saturated brine (80 mL), dried over Na₂SO₄, filtered, and concentrated to give compound 2-2 (8.77 g, yield: 92.8%), and the product was directly used in the next step.

LCMS: t_{R} = 1.24 min, MS (ESI) m/z = 239.3 [M+H]⁺.

### Compound 2-3:

Compound **2-2** (8.77 g, 36.5 mmol) was dissolved in DMF (50 mL) under a nitrogen atmosphere, and TBDPSCl (11.03 g, 40.1 mmol) and imidazole (2.73 g, 40.1 mmol) were added in an ice bath. The mixture was warmed to room temperature and left to react for 2 h.

The reaction mixture was extracted once with petroleum ether (300 mL). The organic phase was washed three times with saturated brine, dried over anhydrous sodium sulfate, filtered, and concentrated, and the resulting residue was purified by column chromatography (elution with pure petroleum ether) to give compound **2-3** (14.33 g, yield: 82.0%).

### Compound 2-4:

TFA (5.95 g, 52.1 mmol) was added slowly to a 1 N solution of ZnEt₃ (52.1 mmol) in n-hexane under a nitrogen atmosphere and a dry ice-ethanol bath. After 20 min of stirring, a solution of CH₂I₂ (22.37 g, 83.5 mmol) in 20 mL of DCM was added to the reaction mixture over 10 min. After another 20 min of stirring, a solution of compound **2-3** (10.00 g, 20.8 mmol) in DCM was slowly added. Then the mixture was warmed to room temperature and stirred for 4 h. The reaction mixture was quenched by addition of a small amount of ice water, and the aqueous phase was extracted three times with dichloromethane (80 mL). The combined organic phases were washed three times with saturated brine, dried over anhydrous sodium sulfate, filtered, and concentrated, and the resulting residue was purified by column chromatography (elution with n-hexane) to give compound **2-4** (7.21 g, yield: 70.0%).

¹H NMR (400 MHz, DMSO) δ ppm 7.63-7.60 (m, 4H), 7.47-7.41 (m, 6H), 3.66-3.63 (m, 2H), 1.54-1.50 (m, 2H), 1.36-1.34 (m, 20H), 1.24 (s, 2H), 1.15 (s, 8H), 1.11 (m, 4H), 0.98-0.54 (m, 4H).

### Compound 2-6:

Compound **1-3** (1.80 g, 7.9 mmol) was added to a mixed solution of compound **2-4** (9.81 g, 19.9 mmol) in DMF (8.5 mL) and diglyme (5.7 mL). Boron trifluoride tetrahydrofuran complex (2.3 g) was slowly added dropwise to the mixed solution under a nitrogen atmosphere in an ice bath. Subsequently, the reaction mixture was heated to 140 °C and left to react for 16 h. The reaction mixture was cooled to room temperature, quenched by being poured into water (100 mL), and then extracted with EtOAc (60 mL × 3). The organic phases were combined, washed with saturated brine (100 mL), dried over Na₂SO₄, filtered, and concentrated, and the resulting residue was purified by alkalinized normal phase silica gel column chromatography (0.1% Et₃N in DCM:MeOH = 10:1, product peak at 5%) to give compound **2-6** (1.08 g, yield: 28.2%).

LCMS: t_{R} = 2.02 min, MS (ESI) m/z = 479.3 [M-H]⁻.

### Compound 2-7:

After compound **2-6** (1.00 g, 2.1 mmol) was dissolved in pyridine (8 mL) under a nitrogen atmosphere, 4A molecular sieve and DMTrCl (1.06 g, 3.1 mmol) were added at 0 °C, and the mixture was then warmed to room temperature and left to react for 4 h. The reaction mixture was quenched by being added to water (60 mL) and was extracted three times with ethyl acetate (40 mL). The combined organic phases were washed three times with saturated brine, then dried over anhydrous sodium sulfate, filtered, and concentrated, and the resulting residue was purified by alkalinized normal phase silica gel column chromatography (0.1% Et₃N in PE:EtOAc = 1:1, product peak at 37%) to give compound 2-7 (0.86 g, yield: 52.5%).

LCMS: t_{R} = 2.36 min, MS (ESI) m/z = 781.5 [M-H]⁻.

¹H NMR (400 MHz, DMSO-d*₆*) δ ppm 11.36 (s, 1H), 7.72 (d, *J =* 8.4 Hz, 1H), 7.38-7.32 (m, 4H), 7.24 (d, *J =* 8.8 Hz, 5H), 6.90 (d, *J =* 8.8 Hz, 4H), 5.80 (d, *J =* 4.0 Hz, 1H), 5.28 (d, *J =* 8.4 Hz, 1H), 5.10 (d, *J =* 6.4 Hz, 1H), 4.16 (q, *J =* 5.6 Hz, 1H), 3.96-3.89 (m, 2H), 3.74 (s, 6H), 3.60-3.55 (m, 2H), 3.29-3.23 (m, 2H), 1.50-1.32 (m, 2H), 1.32-1.23 (m, 20H), 1.13 (s, 2H), 0.86-0.63 (m, 3H), 0.86 (s, 1.8H), 0.62 (s, 1.2H), -0.33 (q, *J =* 4.4 Hz, 1H).

### Compound NA0124:

3A molecular sieve was added to a solution of compound 2-7 (850 mg, 1.0 mmol) in dry acetonitrile (6 mL) at 0 °C under a nitrogen atmosphere. After 5 min of stirring, bis(diisopropylamino)(2-cyanoethoxy)phosphine (721 mg, 2.4 mmol) and a pre-prepared solution of 1H-tetrazole (0.45 M) and N-methylimidazole (0.18 M) in dry acetonitrile (2.4 mL) were added, and the mixture was then warmed to room temperature and left to react for 3 h. After the reaction mixture was filtered, a 5% aqueous sodium bicarbonate solution (20 mL) was added to the reaction mixture. The mixture was extracted twice with dichloromethane (40 mL). The organic phases were combined, dried over sodium sulfate, filtered, and concentrated, and the resulting residue was purified by C18 reversed-phase column chromatography (H₂O/MeCN, from 0% to 100% elution) followed by lyophilization to give compound **NA0124** (841 mg, yield: 79.4%).

LCMS: t_{R} = 2.67 min, MS (ESI) m/z = 981.6 [M-H]⁻.

¹H NMR (400 MHz, DMSO-d₆) δ ppm 11.38 (s, 1H), 7.82-7.77 (m, 1H), 7.41 (t, *J =* 7.6 Hz, 2H), 7.36-7.24 (m, 7H), 6.94-6.89 (m, 4H), 5.82 (t, *J* = 3.2 Hz, 1H), 5.31-5.25 (m, 1H), 4.44-4.34 (m, 1H), 4.15-4.06 (m, 2H), 3.83-3.70 (m, 8H), 3.65-3.51 (m, 5H), 3.39-3.30 (m, 2H), 2.79 (t, *J =* 6.4 Hz, 0.8H), 2.64-2.61 (m, 1.2H), 1.54-1.49 (m, 2H), 1.34-1.26 (m, 20H), 1.16-1.11 (m, 11H), 1.00 (d, *J* = 6.4 Hz, 2H), 0.87 (t, *J =* 7.2 Hz, 3H), 0.66 (br, 2H), 0.57-0.52 (m, 1H), -0.32 (q, *J =* 4.8 Hz, 1H).

³¹P NMR (100 MHz, DMSO-d*₆*) δ ppm 149.14, 148.64.

### Example 1.3: Synthesis of Compound NA130

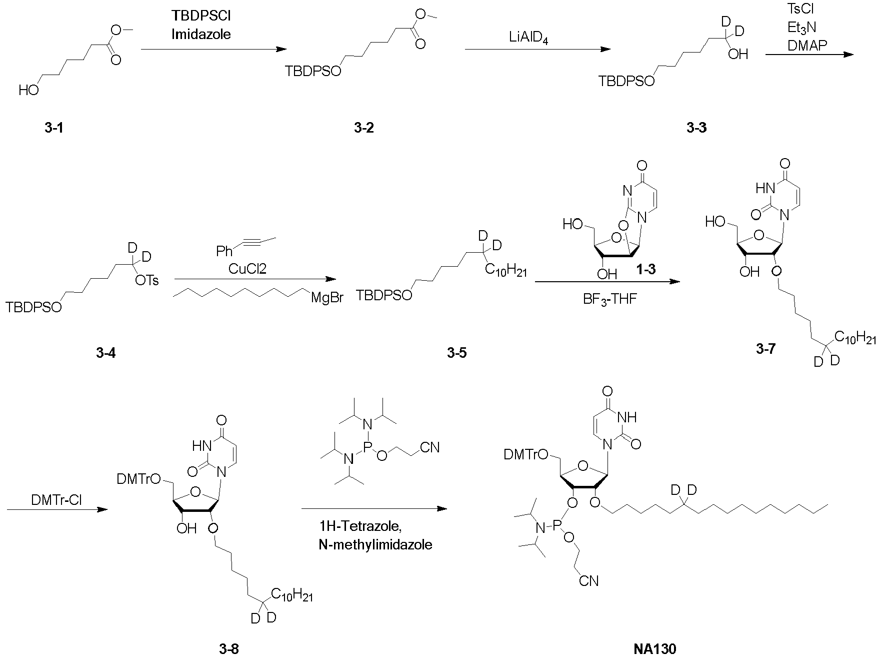

### Compound 3-2:

Compound **3-1** (9.00 g, 61.6 mmol) was dissolved in DMF (60 mL) under a nitrogen atmosphere, and TBDPSCl (18.61 g, 67.7 mmol) and imidazole (4.61 g, 67.7 mmol) were added in an ice bath. Then the mixture was warmed to room temperature and left to react for 16 h. The reaction mixture was extracted once with petroleum ether (300 mL). The organic phase was washed three times with saturated brine, dried over anhydrous sodium sulfate, and concentrated, and the resulting residue was purified by column chromatography (PE:EtOAc = 4:1, product peak at 17%) to give compound **3-2** (19.31 g, yield: 81.5%).

LCMS: t_{R} = 2.04 min, MS (ESI) m/z = 385.4 [M+H]⁺.

### Compound 3-3:

LiAlD₄ (0.59 g, 14.0 mmol) was added to 10 mL of dry THF under a nitrogen atmosphere in an ice bath. After the solution was stirred for 10 min, a solution of compound **3-2** (2.61 g, 7.0 mmol) in THF (10 mL) was slowly injected into the solution of LiAlD₄. Subsequently, the reaction mixture was warmed to room temperature and stirred for 3 h. The reaction mixture was quenched by addition of a saturated aqueous ammonium chloride solution in an ice bath and repeatedly extracted 3 times with ethyl acetate (40 mL). The combined organic solutions were washed with saturated brine (40 mL), dried over Na₂SO₄, filtered, and concentrated to give compound **3-3** (2.31 g, yield: 91.8%), and the product was directly used in the next step.

LCMS: t_{R} = 2.10 min, MS (ESI) m/z = 381.3 [M+H]⁺.

### Compound 3-4:

After compound **3-3** (13.00 g, 36.2 mmol) was dissolved in dichloromethane (80 mL) under a nitrogen atmosphere, triethylamine (11.8 mL, 79.7 mmol), p-toluenesulfonyl chloride (10.37 g, 54.4), and DMAP (2.21 g, 18.1 mmol) were sequentially added at 0 °C. Subsequently, the reaction mixture was warmed to room temperature and stirred overnight. The reaction mixture was washed twice with water (60 mL), washed with saturated brine (100 mL), dried over Na₂SO₄, filtered, and concentrated, and the resulting residue was separated and purified by column chromatography (PE:EtOAc = 20:1, product peak at 5%) to give compound **3-4** (14.5 g, yield: 78.00%).

### Compound 3-5:

After compound **3-4** (14.49 g, 28.2 mmol) was dissolved in tetrahydrofuran (80 mL) under a nitrogen atmosphere, cupric chloride (0.19 g, 1.41 mmol), phenyl-1-propyne (0.68 mL, 5.6 mmol), and a 1 N solution of decylmagnesium bromide in tetrahydrofuran (84.7 mL, 84.7 mmol) were sequentially added at 0 °C. Subsequently, the reaction mixture was warmed to room temperature and stirred for 3 h. The reaction mixture was quenched by addition of ice water (60 mL) in an ice bath and repeatedly extracted 3 times with ethyl acetate (60 mL). The combined organic solutions were washed with saturated brine (40 mL), dried over Na₂SO₄, filtered, and concentrated, and the resulting residue was purified by column chromatography (PE:EtOAc = 20:1, product peak at 0%) to give compound **3-5** (10.1 g, yield: 74.39%).

¹H NMR (400 MHz, DMSO-d₆) δ ppm 7.60-7.58 (4H, m), 7.33-7.27 (m, 6H), 3.57 (t, *J =* 6.4 Hz, 2H), 1.49-1.46 (m, 2H), 1.26-1.16 (m, 26H), 0.97 (s, 10H), 0.82-0.78 (m, 3H).

### Compound 3-7:

Compound **1-3** (2.20 g, 9.7 mmol) was added to a mixed solution of compound **3-5** (10.33 g, 21.4 mmol) in DMF (8.5 mL) and diglyme (5.7 mL). Boron trifluoride tetrahydrofuran complex (2.30 g) was slowly added dropwise to the mixed solution under a nitrogen atmosphere in an ice bath. Subsequently, the reaction mixture was heated to 140 °C and left to react for 16 h. The reaction mixture was cooled to room temperature, quenched by being poured into water (60 mL), and then extracted with EtOAc (50 mL × 3). The combined organic phases were washed with saturated brine (80 mL), dried over Na₂SO₄, filtered, and concentrated, and the resulting residue was purified by alkalinized normal phase silica gel column chromatography (0.1% Et₃N in DCM:MeOH = 10:1, product peak at 6%) to give compound **3-7** (0.97 g, yield: 21.19%).

LCMS: t_{R} = 2.05&2.20 min, MS (ESI) m/z = 469.5 [M-H]⁻.

### Compound 3-8:

After compound **3-7** (0.76 g, 1.6 mmol) was dissolved in pyridine (7 mL) under a nitrogen atmosphere, 4A molecular sieve and DMTrCl (0.82 g, 2.4 mmol) were added at 0 °C, and the mixture was then warmed to room temperature and left to react for 4 h. The reaction mixture was quenched by being added to water (50 mL) and was extracted three times with ethyl acetate (50 mL). The combined organic phases were washed three times with saturated brine, dried over anhydrous sodium sulfate, filtered, and concentrated, and the resulting residue was purified by alkalinized normal phase silica gel column chromatography (0.1% Et₃N in PE:EtOAc = 1:1, product peak at 37%) to give compound **3-8** (0.58 g, yield: 46.54%).

LCMS: t_{R} = 2.26&2.50 min, MS (ESI) m/z = 771.7 [M-H]⁻.

¹H NMR (400 MHz, DMSO-d₆) δ ppm 11.36 (s, 1H) 7.72 (d, *J* = 8.4 Hz, 1.3H), 7.38 (d, *J* = 7.6H, 2H), 7.33 (t, *J =* 7.6 Hz, 2H), 7.28-7.24 (m, 4H), 6.90 (d, *J =* 8.8 Hz, 4H), 5.80 (d, *J =* 4.0 Hz, 1H), 5.29 (d, *J* = 8.0 Hz, 1H), 5.11 (d, *J* = 6.8 Hz, 1H), 4.18 (q, *J* = 5.6 Hz, 1H), 3.98-3.95 (m, 1H), 3.90 (t, *J =* 4.8 Hz, 1H), 3.79 (s, 6.7 Hz), 3.61-3.52 (m, 2.3H), 3.30-3.22 (m, 2H), 1.52-1.48 (m, 2H), 1.23 (s, 27H), 0.85 (t, *J =* 6.4 Hz, 3H).

### Compound NA0130:

3A molecular sieve was added to a solution of compound **3-8** (580 mg, 0.8 mmol) in dry acetonitrile (5 mL) at 0 °C under a nitrogen atmosphere. After 5 min of stirring, bis(diisopropylamino)(2-cyanoethoxy)phosphine (500 mg, 1.7 mmol) and a pre-prepared solution of 1H-tetrazole (0.45 M) and N-methylimidazole (0.18 M) in dry acetonitrile (1.7 mL) were added, and the mixture was then warmed to room temperature and left to react for 3 h. After the reaction mixture was filtered, a 5% aqueous sodium bicarbonate solution (20 mL) was added to the reaction mixture, and the mixture was extracted twice with dichloromethane (40 mL). The organic phases were combined, dried over sodium sulfate, filtered, and concentrated, and the resulting residue was purified by C18 reversed-phase column chromatography (H₂O/MeCN, from 0% to 100% elution) followed by lyophilization to give compound **NA0130** (610 mg, yield: 83.41%).

LCMS: t_{R} = 2.46&2.70 min, MS (ESI) m/z = 971.8 [M-H]⁻.

¹H NMR (400 MHz, DMSO-d₆) *δ* ppm 11.35 (s, 1H), 7.80-7.75 (m, 1H), 7.39 (t, *J =* 7.6 Hz, 2H), 7.36-7.23 (m, 7H), 6.93-6.87 (m, 4H), 5.80 (t, *J* = 3.6 Hz, 1H), 5.30-5.24 (m, 1H), 4.43-4.32 (m, 1H), 4.13-4.04 (m, 2H), 3.81-3.63 (m, 8H), 3.62-3.49 (m, 5H), 3.38-3.34 (m, 2H), 2.77 (t, *J* = 6.4 Hz, 0.8H), 2.63-2.61 (m, 1.3H), 1.53-1.46 (m, 2H), 1.23 (s, 23H), 1.14-1.10 (m, 10H), 0.98 (d, *J =* 6.4 Hz, 2H), 0.85 (t, *J =* 6.4 Hz, 3H).

³¹P NMR (100 MHz, DMSO-d₆) *δ* ppm 149.15, 148.64.

### Example 1.4: Synthesis of Compound NA0131

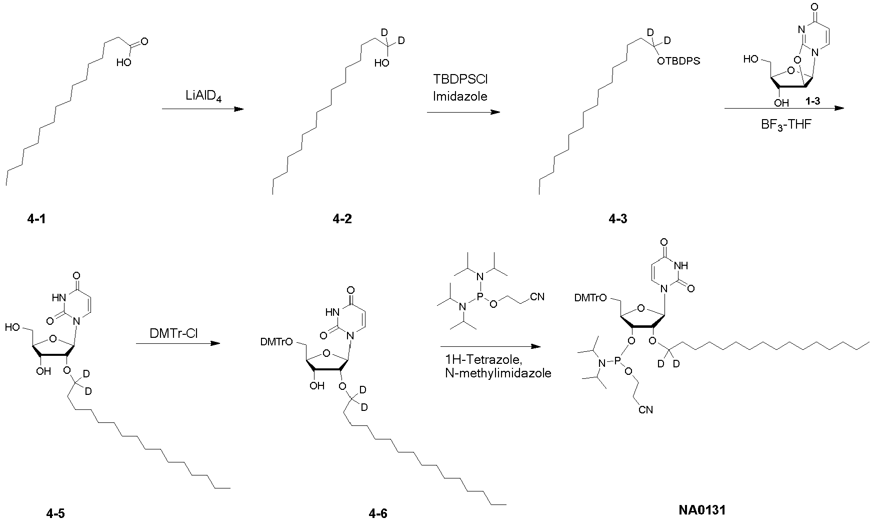

### Compound 4-2:

LiAlD₄ (1.64 g, 38.99 mmol) was added to 20 mL of dry THF under a nitrogen atmosphere in an ice bath. After the solution was stirred for 10 min, a solution of compound **4-1** (10.0 g, 38.9 mmol) in tetrahydrofuran (60 mL) was slowly injected into the solution of LiAlD₄. Subsequently, the reaction mixture was warmed to room temperature and stirred for 2 h. The reaction mixture was quenched by addition of a saturated aqueous ammonium chloride solution in an ice bath and repeatedly extracted 3 times with ethyl acetate (100 mL). The combined organic solutions were washed with saturated brine (80 mL), dried over Na₂SO₄, filtered, and concentrated to give compound **4-2** (7.32 g, yield: 76.8%), and the product was directly used in the next step.

### Compound 4-3:

After compound **4-2** (7.00 g, 28.6 mmol) was dissolved in DMF (50 mL) under a nitrogen atmosphere, TBDPSCl (8.66 g, 31.5 mmol) and imidazole (2.14 g, 31.5 mmol) were added in an ice bath. Then the mixture was warmed to room temperature and left to react for 2 h. The reaction mixture was extracted once with petroleum ether (300 mL). The organic phase was washed three times with saturated brine, dried over anhydrous sodium sulfate, filtered, and concentrated, and the resulting residue was purified by column chromatography (elution with pure petroleum ether) to give compound **4-3** (10.10 g, yield: 73.0%).

LCMS: t_{R} = 2.41 min, MS (ESI) m/z = 483.8 [M+H]⁺.

### Compound 4-5:

Compound **1-3** (1.35 g, 5.9 mmol) was added to a mixed solution of compound **4-3** (8.65 g, 17.9 mmol) in DMF (5 mL) and diglyme (3.3 mL). Boron trifluoride tetrahydrofuran complex (1.60 g) was slowly added dropwise to the mixed solution under a nitrogen atmosphere in an ice bath. Subsequently, the reaction mixture was heated to 140 °C and left to react for 16 h. The reaction mixture was cooled to room temperature, quenched by being poured into water (60 mL), and then extracted with EtOAc (50 mL × 3). The combined organic phases were washed with saturated brine (80 mL), dried over Na₂SO₄, filtered, and concentrated, and the resulting residue was purified by alkalinized normal phase silica gel column chromatography (0.1% Et₃N in DCM:MeOH = 10:1, product peak at 5%) to give compound **4-5** (0.61 g, yield: 21.72%).

LCMS: t_{R} = 2.04 min, MS (ESI) m/z = 469.6 [M-H]⁻.

### Compound 4-6:

After compound **4-5** (0.61 g, 1.3 mmol) was dissolved in pyridine (5 mL) under a nitrogen atmosphere, 4A molecular sieve and DMTrCl (0.75 g, 2.2 mmol) were added at 0 °C, and the mixture was then warmed to room temperature and left to react for 4 h. The reaction mixture was quenched by being added to water (50 mL) and was extracted three times with ethyl acetate (40 mL). The combined organic phases were washed three times with saturated brine, then dried over anhydrous sodium sulfate, filtered, and concentrated, and the resulting residue was purified by alkalinized normal phase silica gel column chromatography (0.1% Et₃N in PE:EtOAc = 1:1, product peak at 37%) to give compound **4-6** (0.58 g, yield: 58.1%).

LCMS: t_{R} = 2.26&2.37 min, MS (ESI) m/z = 771.7 [M-H]⁻.

¹H NMR (400 MHz, DMSO-d₆) *δ* ppm 11.36 (s, 1H) 7.72 (d, *J =* 8.4 Hz, 1.3H), 7.38-7.31 (m, 3.7H), 7.25 (d, *J =* 8.4 Hz, 4H), 6.90 (d, *J =* 8.8 Hz, 3.5H), 5.79 (d, *J =* 3.6 Hz, 1H), 5.28 (d, *J* = 8.4 Hz, 1H), 5.10 (d, *J =* 6.8 Hz, 1H), 4.16 (q, *J =* 5.6 Hz, 1.5H), 3.96-3.88 (m, 2.7H), 3.74 (s, 5.7H), 3.28-3.21 (m, 2H), 1.48 (s, 2H), 1.22 (s, 24H), 0.84 (t, *J =* 6.0 Hz, 3H).

### Compound NA0131:

3A molecular sieve was added to a solution of compound **4-6** (580 mg, 0.8 mmol) in dry acetonitrile (5 mL) at 0 °C under a nitrogen atmosphere. After 5 min of stirring, bis(diisopropylamino)(2-cyanoethoxy)phosphine (497 mg, 1.7 mmol) and a pre-prepared solution of 1H-tetrazole (0.45 M) and N-methylimidazole (0.18 M) in dry acetonitrile (1.8 mL) were added, and the mixture was then warmed to room temperature and left to react for 3 h. After the reaction mixture was filtered, a 5% aqueous sodium bicarbonate solution (20 mL) was added to the reaction mixture. The mixture was extracted twice with dichloromethane (40 mL). The organic phases were combined, dried over sodium sulfate, filtered, and concentrated, and the resulting residue was purified by C18 reversed-phase column chromatography (H₂O/MeCN, from 0% to 100% elution) followed by lyophilization to give compound **NA0131** (630 mg, yield: 86.3%).

LCMS: t_{R} = 2.54&2.68 min, MS (ESI) m/z = 971.8 [M-H]⁻.

¹H NMR (400 MHz, DMSO-d₆) δ ppm 11.31 (s, 1H), 7.75-769 (m, 1H), 7.32 (t, *J =* 7.6 Hz, 2H), 7.27-7.16 (m, 7H), 6.85-6.80 (m, 4H), 5.73 (t, *J* = 3.6 Hz, 1H), 5.22-5.16 (m, 1H), 4.36-4.21 (m, 1H), 4.06-3.97 (m, 2H), 3.84-3.63 (m, 7H), 3.60-3.42 (m, 3H), 3.30-3.28 (m, 2H), 2.70 (t, *J* = 6.4 Hz, 0.8H), 2.60-2.48 (m, 1.3H), 1.41-1.36 (m, 2H), 1.15 (s, 26H), 1.08-1.02 (m, 10H), 0.91 (d, *J =* 6.4 Hz, 2H), 0.77 (t, *J =* 6.4 Hz, 3H).

³¹P NMR (100 MHz, DMSO-d₆) δ ppm 149.13, 148.60.

### Example 1.5: Synthesis of Compound NA0133

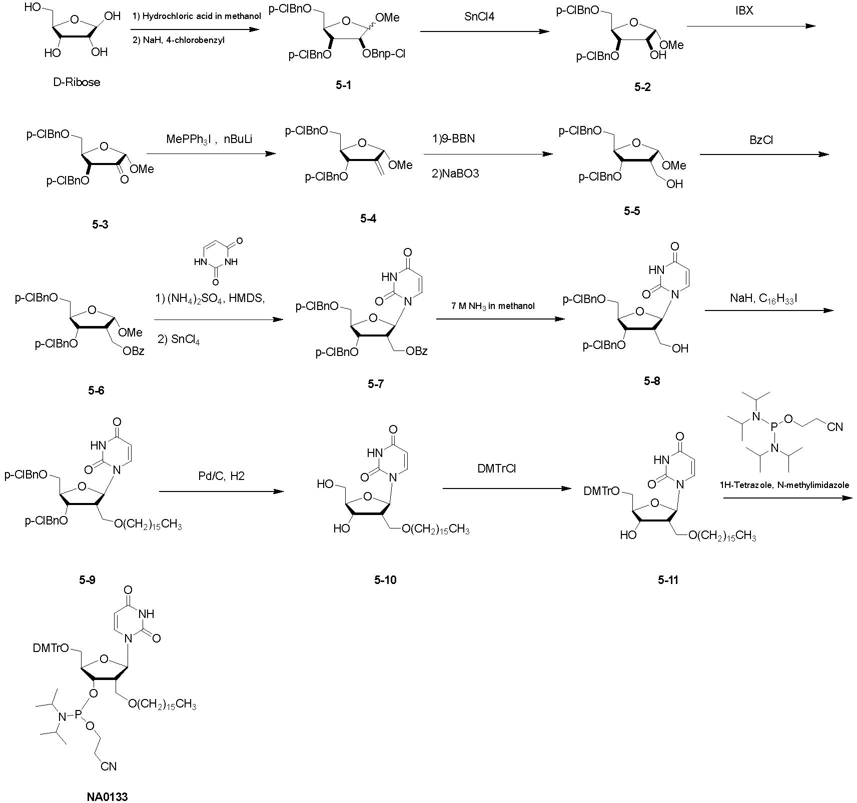

### Compound 5-1:

A 1% solution of HCl in methanol (60 mL) was added to a solution of D-ribose (30.0 g, 67 mmol) in methanol (200 mL) at 0 °C. Subsequently, the reaction mixture was warmed to room temperature and stirred for 3 days. The reaction mixture was neutralized and quenched with solid sodium bicarbonate, and a solid formed. After filtration, the filtrate was dried and concentrated to give a crude product. The crude product was redissolved in 500 mL of DMF solution, and NaH (36.0 g, 1500.0 mmol) was slowly added to the above solution under a nitrogen atmosphere in an ice bath. After the reaction mixture was stirred for 30 min, 4-chlorobenzyl (108.7 g, 675.0 mmol) was added. Subsequently, the reaction mixture was warmed to room temperature and stirred overnight. Ice water was added in an ice bath to quench the reaction. The reaction mixture was extracted three times with DCM (150 mL). The combined organic phases were washed with saturated brine, dried over Na₂SO₄, filtered, and concentrated, and the resulting residue was purified by silica gel column chromatography (PE:EA = 4/1, peak at 10%) to give compound **5-1** (54.5 g, yield: 67.55%).

LCMS: t_{R} = 2.09&2.32 min, MS (ESI) m/z = 561.3 [M+Na]⁺.

### Compound 5-2:

Under a nitrogen atmosphere, 60 mL of a 10% solution of SnCl₄ in DCM was slowly added to a solution of compound **5-1** (30.00 g, 55.7 mmol) in DCM (100 mL), and the reaction was stirred overnight at room temperature. The reaction was neutralized and quenched with a saturated aqueous sodium bicarbonate solution in an ice bath. The reaction mixture was extracted three times with DCM (150 mL). The combined organic phases were washed with saturated brine, dried over Na₂SO₄, filtered, and concentrated, and the resulting residue was purified by silica gel column chromatography (PE:EA = 5/1, peak at 20%) to give compound 5-2 (16.00 g, yield: 69.41%).

¹H NMR (400 MHz, DMSO-d6) δ ppm 7.39 (d, *J =* 8.4 Hz, 2H), 7.35 (s, 4H), 7.30 (d, *J =* 8.4 Hz, 2H), 4.77 (d, *J =* 4.4 Hz, 1H), 4.63 (d, *J =* 12.8 Hz, 1H), 4.50-4.42 (m, 3H), 4.06-4.05 (m, 2H), 3.67 (dd, *J =* 6.4, .3.2 Hz, 1H), 3.45 (d, *J =* 4.4 Hz, 2H), 3.31 (s, 3H).

LCMS: t_{R} = 2.13 min, MS (ESI) m/z = 435.1 [M+Na]⁺.

### Compound 5-3:

IBX (40.65 g, 145.2 mmol) was added to a solution of compound 5-2 (20.0 g, 48.4 mmol) in acetonitrile (150 mL), and the reaction mixture was heated to 83 °C and stirred for 5 h. The reaction mixture was filtered and extracted 3 times with ethyl acetate (100 mL). The combined organic phases were washed with an aqueous Na₂SO₃ solution, a saturated aqueous NaHCO₃ solution, and saturated brine in sequence. The organic phase was dried over Na₂SO₄, filtered, and concentrated to give crude compound 5-3 (15.11 g).

### Compound 5-4:

Under an argon atmosphere, 20 mL of nBuLi (2.5 N in THF) was slowly injected into a solution of methyltriphenylphosphonium iodide (19.10 g, 53.2 mmol) in THF (60 mL) at -78 °C. After the reaction mixture was stirred for 1 h, a solution of compound **5-3** (15.1 g) in THF was added to the above reaction mixture, and the reaction was stirred overnight. The reaction mixture was quenched with a saturated aqueous ammonium chloride solution at low temperature and then extracted 3 times with EA (100 mL). The combined organic phases were washed with saturated brine, dried over Na₂SO₄, filtered, and concentrated, and the resulting residue was purified by silica gel column chromatography (PE:EA = 5/1, peak at 20%) to give compound **5-4** (10.0 g, yield: 50.49%).

LCMS: t_{R} = 1.81 min, MS (ESI) m/z = 431.3 [M+Na]⁺.

### Compound 5-5:

Compound **5-4** (26.2 g, 64.2 mmol) was dissolved in a 0.5 M solution of 9-borabicyclo[3,3,1]-nonane in tetrahydrofuran (384 mL, 192.2 mmol) at room temperature under a nitrogen atmosphere, and the reaction mixture was heated to 40 °C and stirred for 16 h. A mixture of sodium perborate (tetrahydrate) (102.4 g, 1152.2 mmol) and water (100 mL) was added to the reaction mixture in an ice bath, and the resulting mixture was stirred for 3-4 h. The reaction mixture was filtered through diatomaceous earth. The filter cake was washed 2-3 times with ethyl acetate, and the filtrate was extracted 3 times with ethyl acetate (200 mL). The combined organic phases were washed with saturated brine, dried over anhydrous sodium sulfate, filtered, and concentrated, and the resulting residue was purified by silica gel column chromatography (PE:EA= 35%:65%) to give compound **5-5** (25 g, yield: 91%).

LCMS: t_{R} = 1.50 min, MS (ESI) m/z = 449.4 [M+Na]⁺.

### Compound 5-6:

Compound **5-5** (2.0 g, 4.6 mmol) was dissolved in anhydrous pyridine (20 mL), and benzoyl chloride (1.09 mL, 9.36 mmol) was added. The mixture was stirred overnight at room temperature. After the reaction mixture was concentrated, the residue was redissolved in dichloromethane (20 mL). The solution was washed with a saturated aqueous sodium bicarbonate solution and saturated brine in sequence, dried over anhydrous sodium sulfate, filtered, and concentrated, and the resulting residue was purified by reversed-phase column chromatography to give compound **5-6** (2.3 g, yield: 92%).

LCMS: t_{R} = 2.07 min, MS (ESI) m/z = 553.3 [M+Na]⁺.

¹H NMR (400 MHz, DMSO) δ ppm 7.95 (d, *J =* 1.2 Hz, 2H), 7.59 (t, *J =* 0.8 Hz, 1H), 7.46 (t, *J* = 0.8 Hz, 2H), 7.31 (d, *J =* 0.8 Hz, 2H), 7.22-7.15 (m, 6H), 5.07 (d, *J =* 0.8 Hz, 1H), 4.63-4.57 (m, 3H), 4.52-4.41 (m, 3H), 3.95 (d, *J =* 0.8 Hz, 1H), 3.47-3.33 (m, 5H), 2.65-2.55 (m, 1H).

### Compound 5-7:

Uracil (2.7 g, 24.1 mmol) and ammonium sulfate (0.16 g, 0.2 mmol) were added to hexamethyldisilazane (16 mL), and the mixture was stirred at 140 °C until it was clear, then left to react for another half an hour, and concentrated to give a tetramethylsilane-protected uracil intermediate. Compound **5-6** (1.6 g, 3.0 mmol) in anhydrous acetonitrile (30 mL) was added to the tetramethylsilane-protected uracil intermediate, and tin tetrachloride (1.4 mL, 12.0 mmol) was subsequently added. The mixture was stirred at room temperature for 16 h. The reaction mixture was quenched by addition of saturated sodium bicarbonate and extracted 3 times with dichloromethane (20 mL). The combined organic phases were washed with saturated brine, dried over anhydrous sodium sulfate, filtered, and concentrated, and the resulting residue was purified by reversed-phase column chromatography to give compound 5-7 (1.0 g, yield: 54%).

LCMS: t_{R} = 1.79 min, MS (ESI) m/z = 633.4 [M+Na]⁺.

¹H NMR (400 MHz, DMSO) δ ppm 7.89 (s, 1H), 7.87 (s, 1H), 7.65-7.52 (m, 2H), 7.47-7.41 (m, 2H), 7.37-7.28 (m, 4H), 7.26-7.18 (m, 4H), 6.34 (d, *J =* 0.8 Hz, 1H), 5.50 (d, *J =* 0.8 Hz, 1H), 4.74-4.69 (m, 1H), 4.65-4.42 (m, 6H), 4.29 (d, *J =* 0.4 Hz, 1H), 4.19 (d, *J =* 0.4 Hz, 1H), 3.73-3.68 (m, 1H), 3.56-3.50 (m, 1H), 2.85-2.79 (m, 1H).

### Compound 5-8:

Compound **5-7** (1.7 g, 2.8 mmol) was dissolved in a 7 M solution of ammonia water in methanol (20 mL), and the solution was stirred overnight at 60 °C. The reaction mixture was concentrated, and the resulting residue was purified by reversed-phase column chromatography to give compound **5-8** (1.05 g, yield: 74%).

LCMS: t_{R} = 1.35 min, MS (ESI) m/z = 529.4 [M+H]⁺.

### Compound 5-9:

Sodium hydride (0.89 g) was slowly added to a solution of compound **5-8** (4.50 g, 8.9 mmol) in N,N-dimethylformamide (15 mL) in an ice bath. After 30 min of stirring at that temperature, iodohexadecane (6.30 g, 17.8 mmol) was added, and the reaction was warmed to room temperature and stirred for two hours. The reaction mixture was quenched with a saturated aqueous ammonium chloride solution and extracted three times with ethyl acetate (30 mL). The combined organic phases were washed with saturated brine, dried over anhydrous sodium sulfate, filtered, and concentrated, and the resulting residue was purified by reversed-phase column chromatography to give compound **5-9** (3.5 g, yield: 54%).

LCMS: t_{R} = 3.83 min, MS (ESI) m/z = 731.7 [M+H]⁺.

### Compound 5-10:

10% palladium on carbon (0.5 g) was added to a solution of compound **5-9** (3.5 g, 4.8 mmol)) in methanol (100 mL), and the mixture was stirred overnight at room temperature under a hydrogen atmosphere. The reaction mixture was concentrated, and the resulting residue was purified by reversed-phase column chromatography to give compound **5-10** (2.2 g, yield: 96%).

LCMS: t_{R} = 1.45 min, MS (ESI) m/z = 481.6 [M-H]⁺.

### Compound 5-11:

4,4'-Dimethoxytriphenylmethyl chloride (4.7 g, 13.8 mmol) was added to a solution of compound **5-10** (2.2 g, 4.6 mol) in pyridine (20 mL), and the mixture was stirred overnight at room temperature. Methanol (5 mL) was added at 0 °C to quench the reaction. After the reaction mixture was concentrated, water (30 mL) was added to redissolve the residue, and the solution was extracted twice with dichloromethane (50 mL). The combined organic phases were washed with saturated brine, dried over anhydrous sodium sulfate, filtered, and concentrated, and the resulting residue was purified by silica gel column chromatography to give compound **5-11** (2.6 g, yield: 72%).

LCMS: t_{R} = 3.22 min, MS (ESI) m/z = 783.9 [M-H]⁺.

¹H NMR (400 MHz, DMSO) δ ppm 12.11 (s, 1H), 8.38 (d, J = 8.0 Hz, 1H), 8.24-8.17 (m, 3H), 8.18-8.09 (m, 3H), 8.11-8.03 (m, 7H), 8.07-7.98 (m, 5H), 7.93-7.86 (m, 2H), 7.75-7.68 (m, 4H), 7.70-7.63 (m, 2H), 7.04 (s, 1H), 6.83 (d, J = 8.4 Hz, 1H), 6.23 (dd, J = 8.0, 2.0 Hz, 1H), 6.18 (d, J = 5.2 Hz, 1H), 5.07-4.99 (m, 1H), 4.75 (d, J = 6.0 Hz, 1H), 4.56 (s, 9H), 4.55 (s, 9H), 4.48 (dd, J = 10.0, 5.2 Hz, 1H), 4.29-4.20 (m, 1H), 4.11-4.04 (m, 4H), 3.97 (dd, J = 10.4, 4.0 Hz, 1H), 2.21 (s, 2H), 2.11-2.01 (m, 25H), 1.71-1.63 (m, 4H).

### Compound NA0133:

3A molecular sieve was added to a solution of compound **5-11** (1.6 g, 2.0 mmol) in dry acetonitrile (15 mL) at room temperature under a nitrogen atmosphere. After 5 min of stirring at room temperature, bis(diisopropylamino)(2-cyanoethoxy)phosphine (2.4 g, 8.9 mmol) was added, and a pre-prepared solution of 1H-tetrazole (0.45 M, 0.045 mmol) and N-methylimidazole (0.18 M, 0.018 mmol) in dry acetonitrile (7 mL) was then added. The mixture was left to react at room temperature for 3 h. After filtration, a 5% aqueous sodium bicarbonate solution (30 mL) was added to the reaction mixture, and the mixture was extracted with dichloromethane (30 mL). The extract was dried over sodium sulfate, filtered, and concentrated, and the residue was purified by C18 reversed-phase column chromatography followed by lyophilization to give compound **NA0133** (1.6 g, yield: 80%).

LCMS: t_{R} = 3.97 min, MS (ESI) m/z = 984.1 [M-H]⁺.

¹H NMR (400 MHz, DMSO) *δ* ppm 11.33 (s, 1H), 7.60 (t, *J =* 8.0 Hz, 1H), 7.42-7.35 (m, 2H), 7.34-7.19 (m, 7H), 6.92-6.84 (m, 4H), 5.98 (dd, *J =* 16.0, 7.6 Hz, 1H), 5.43 (dd, *J =* 11.2, 8.0 Hz, 1H), 4.55-4.36 (m, 1H), 4.16-4.03 (m, 1H), 3.74 (d, *J =* 2.4 Hz, 6H), 3.70-3.48 (m, 5H), 3.42 (dd, *J =* 9.6, 6.8 Hz, 1H), 3.29-3.25 (m, 4H), 2.76 (t, *J =* 6.0 Hz, 1H), 2.71-2.64 (m, 1H), 2.60-2.55 (m, 1H), 1.45-1.35 (m, 2H), 1.31-1.16 (m, 28H), 1.15-1.09 (m, 8H), 1.00 (d, *J=* 6.8 Hz, 2H), 0.87-0.82 (m, 3H).

### Example 2: Synthesis of siRNAs with Lipid Delivery

The siRNA synthesis followed the conventional phosphoramidite solid-phase synthesis method. In synthesizing the modified nucleotide at position 6 of the 5' end of the SS strand, the original nucleoside phosphoramidite monomer of the parent sequence was replaced with the phosphoramidite monomer synthesized above. The synthesis process is briefly described below: Nucleoside phosphoramidite monomers were linked one by one according to the synthesis program on a Dr. Oligo48 synthesizer (Biolytic), beginning with a Universal CPG support. Other than the phosphoramidite monomer at position 6 of the 5' end of the SS strand described above, nucleoside phosphoramidite monomers such as 2'-F RNA, 2'-O-methyl RNA, and Chd and VPUm monomers were purchased from Shanghai Hongene, Suzhou Genepharma, or Jiangsu Synthgene Biotechnology Co., Ltd. 5-Ethylthio-1H-tetrazole (ETT) was used as an activator (a 0.6 M solution in acetonitrile), a 0.22 M solution of PADS in acetonitrile and collidine (1:1 by volume) (Suzhou Kroma) as a sulfurizing agent, and iodopyridine/water solution (Kroma) as an oxidant.

After the solid-phase synthesis was completed, oligoribonucleotides were cleaved from the solid support and soaked in a solution of 28% ammonia water and ethanol (3:1) at 50 °C for 16 h. Then the mixture was centrifuged, and the supernatant was transferred to another centrifuge tube. After the supernatant was concentrated to dryness by evaporation, the residue was purified by C18 reversed-phase chromatography using 0.1 M TEAA and acetonitrile as the mobile phase, and DMTr was removed using a 3% trifluoroacetic acid solution. The target oligonucleotides were collected, then lyophilized, identified as the target products by LC-MS, and quantified by UV (260 nm).

The resulting single-stranded oligonucleotides were complementarily paired in an equimolar ratio and annealed. The final double-stranded siRNA was dissolved in 1× PBS, and the solution was adjusted to the concentration required for the experiment and set aside for later use.

**Table 1. The sequences of an siRNA targeting the MUC5B gene**

| SS strand 5'-3' | AS strand 5'-3' |
|---|---|
| GUG CUU UAA CUA CAA UAU ACA | UGU AUA UUG UAG UUA AAG CAC AU |

**Table 2. The sequences of modified siRNAs targeting the MUC5B gene**

| Double strand No. | SS strand | AS strand |
|---|---|---|
| TJR100072 | GmsUmsGmCmUmUmUfAmAfCfUfAm CmAmAmUmAmUmAmsCmsAm | VPUmsGfsUmAmUmAfUmUfGfUmAmG mUmUfAmAfAmGmCmAmCmsAmsUm |
| TJR100073 | GmsUmsGmCmUm(Uhd')UfAmAfCfUf AmCmAmAmUmAmUmAmsCmsAm | VPUmsGfsUmAmUmAfUmUfGfUmAmG mUmUfAmAfAmGmCmAmCmsAmsUm |
| TJR100344 | GmsUmsGmCmUm(NA0124')UfAmAfC fUfAmCmAmAmUmAmUmAmsCmsAm | VPUmsGfsUmAmUmAfUmUfGfUmAmG mUmUfAmAfAmGmCmAmCmsAmsUm |
| TJR100345 | GmsUmsGmCmUm(NA0131') UfAmAfCfUfAmCmAmAmUmAmUm AmsCmsAm | VPUmsGfsUm AmUmAf UmUfGf UmAmGm UmUfAm AfAmGm CmAmCms AmsUm |

**Table 3. The sequences of siRNAs targeting the APP gene**

| SS strand 5'-3' | AS strand 5'-3' |
|---|---|
| CCG GUC CCA GGU UAU GAC ACU | AGU GUC AUA ACC UGG GAC CGG AU |
| UCG GUC CCA GGU UAU GAC ACU | AGU GUC AUA ACC UGG GAC CGA AU |

**Table 4. The sequences of modified siRNAs targeting the APP gene**

| Double strand No. | SS strand | AS strand |
|---|---|---|
| TJR102160 | CmsCmsGmGmUm(Chd')CfCmAfGf GfUmUmAmUmGmAmCmAmsCms Um | AmsGfsUmGmUmCfAmUfAfAmCmCm UmGfGmGfAmCmCmGmGmsAmsUm |
| TJR102161 | CmsCmsGmGmUmCmCfCmAfGfGf UmUmAmUmGmAmCmAmsCms(N A0124') | AmsGfsUmGmUmCfAmUfAfAmCmCm UmGfGmGfAmCmCmGmGmsAmsUm |
| TJR102162 | CmsCmsGmGmUmCmCfCmAfGfGf UmUmAmUmGmAmCmAmsCms(N A0133') | AmsGfsUmGmUmCfAmUfAfAmCmCm UmGfGmGfAmCmCmGmGmsAmsUm |
| TJR102178 | (Uhd')sCmsGmGmUmCmCfCmAfG fGfUmUmAmUmGmAmCmAmsCm sUm | AmsGfsUmGmUmCfAmUfAfAmCmCm UmGfGmGfAmCmCmGmAmsAmsUm |
| TJR102179 | (NA0124')sCmsGm GmUmCm CfCmAfGfGfUmUmAmUmGmAmC mAmsCmsUm | AmsGfsUmGmUmCfAmUfAfAmCmCm UmGfGmGfAmCmCmGmAmsAmsUm |
| TJR102180 | (NA0133’)sCmsGmGmUmCm CfCmAfGfGfUmUmAmUmGmAmC mAmsCmsUm | AmsGfsUmGmUmCfAmUfAfAmCmCm UmGfGmGfAmCmCmGmAmsAmsUm |

**Table 5. The sequences of an siRNA targeting the PMP22 gene**

| SS strand 5'-3' | AS strand 5'-3' |
|---|---|
| CCU CCU GUU GCU GAG UAU CAA | UUG AUA CUC AGC AAC AGG AGG AG |

**Table 6. The sequences of modified siRNAs targeting the PMP22 gene**

| Double strand No. | SS strand | AS strand |
|---|---|---|
| TJR102149 | CmsCmsUmCmCm(Uhd')GmUmUfGfCf UmGmAmGmUmAmUmCmsAmsAm | VPUmsUfsGmAmUmAfCmUmCmAmGmC mAmAfCmAfGmGmAmGmGmsAmsGm |
| TJR102150 | CmsCmsUmCmCm(NA0124') GmUmUfGfCfUmGmAmGmUmAmUm CmsAmsAm | VPUmsUfsGmAmUmAfCmUmCmAmGmC mAmAfCmAfGmGmAmGmGmsAmsGm |
| TJR102151 | CmsCmsUmCmCm(NA0133') GmUmUfGfCfUmGmAmGmUmAmUm CmsAmsAm | VPUmsUfsGmAmUmAfCmUmCmAmGmC mAmAfCmAfGmGmAmGmGmsAmsGm |

**Table 7. The sequences of siRNAs targeting the MAPT gene**

| SS strand 5'-3' | AS strand 5'-3' |
|---|---|
| UUC ACU UUA UCA AUA GUU CCA | UGG AAC UAU UGA UAA AGU GAA UU |
| UUC ACU UUA UCA AUA GUU CCU | AGG AAC UAU UGA UAA AGU GAA UU |

**Table 8. The sequences of modified siRNAs targeting the MAPT gene**

| Double strand No. | SS strand | AS strand |
|---|---|---|
| TJR102217 | (Uhd’)sUmsCmAmCmUmUmUmAfU fCfAmAmUmAmGmUmUmCmsCms Am | VPUmsGfsGmAmAmCfUmAmUmUmGm AfUmAfAmAfGmUmGmAmAmsUmsUm |
| TJR102218 | (NA0124’)sUmsCmAmCmUm UmUmAfUfCfAmAmUmAmGmUmU m CmsCmsAm | VPUmsGfsGmAmAmCfUmAmUmUmGm AfUmAfAmAfGmUmGmAmAmsUmsUm |
| TJR102219 | (NA0133')sUmsCmAmCmUm UmUmAfUfCfAmAmUmAmGmUmU mCmsCmsAm | VPUmsGfsGmAmAmCfUmAmUmUmGm AfUmAfAmAfGmUmGmAmAmsUmsUm |
| TJR101646 | UmsUmsCmAmCm(Uhd')UmUmAfU fCfAmAmUmAmGmUmUmCmsCms Am | VPUmsGfsGmAmAmCfUmAmUmUmGm AfUmAfAmAfGmUmGm AmAmsUmsUm |
| TJR101647 | UmsUmsCmAmCm(NA0124')UmUm AfUfCfAmAmUmAmGmUmUmCms CmsAm | VPUmsGfsGmAmAmCfUmAmUmUmGm AfUmAfAmAfGmUmGmAmAmsUmsUm |
| TJR101740 | UmsUmsCmAmCm(NA0133')UmUm AfUfCfAmAmUmAmGmUmUmCms CmsAm | VPUmsGfsGmAmAmCfUmAmUmUmGm AfUmAfAmAfGmUmGmAmAmsUmsUm |

In the sequences of the modified siRNAs of the present disclosure, the structure of Uhd' is: 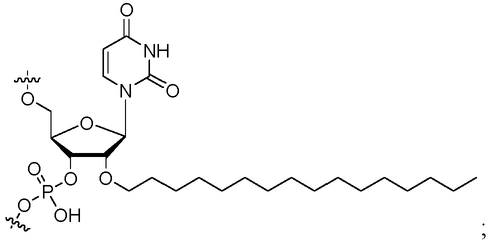 the structure of Chd' is: 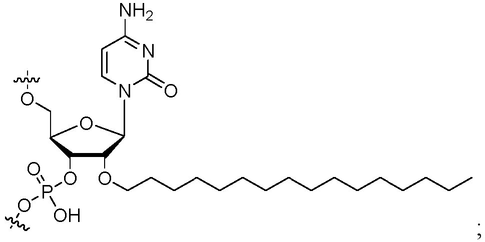 the structure of NA0124' is: 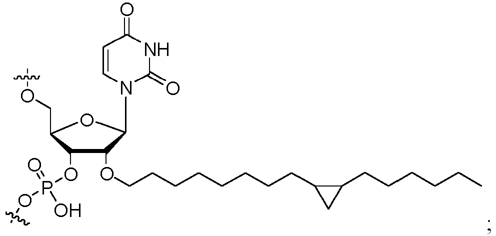 the structure of NA0 13 1' is: 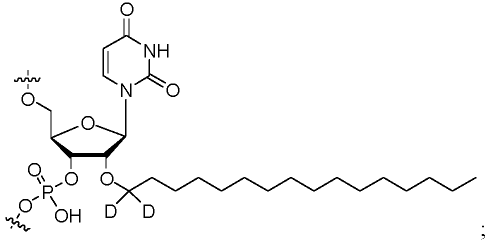 the structure of NA0133' is: 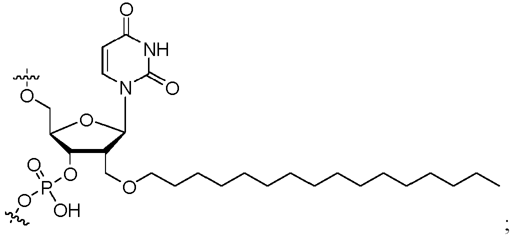 the structure of VPUms is:

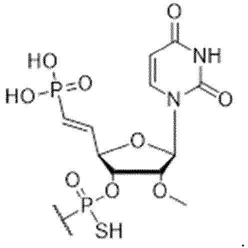

### Example 3: Evaluation of In Vitro Anti-MUC5B Activity of Oligonucleotides of the Present Disclosure Using MLE-12 Cells

The *in vitro* anti-MUC5B activity of the 4 siRNA sequences in the table was assessed using 3 concentration gradients in MLE-12 cells.

The specific steps of the siRNA *in vitro* anti-MUC5B activity assay were as follows:
In the MLE-12 cell line, the 4 siRNA sequences were assayed for inhibitory activity against MUC5B mRNA levels by performing a transfection experiment using a transfection reagent.

The experimental materials and instruments are detailed in Table 9 and Table 10.

**Table 9. Experimental consumables and reagents**

| Name | Company | Cat. No./model | Batch No. | Shelf life |
|---|---|---|---|---|
| MLE-12 cells | Haixing Biosciences | TCM-C745 | / | / |
| Special culture medium for MLE-12 cells | Haixing Biosciences | TCM-G745 | / | / |
| OPTI-MEM | GIBCO | 31985-070 | / | 2022/1/30 |
| Trypsin | GIBCO | 25200-056 | 2323203 | 2023-9-30 |
| Lipofectamine RNAi Max | Invitrogen | 13778-150 | 2335092 | 2023-6-18 |
| PBS(1X) | Corning | 21-040-CVR | 29619004 | 2021-10 |
| High-throughput cellular RNA extraction kit | FireGen | FG0417 | DE09A | 2023-5-8 |
| PrimeScript^{™} II 1st Strand cDNA Synthesis Kit | Takara | 6210B (A × 4) | ALE1835A | 2023-06 |
| TB Green Premix | Takara | RR820B(AX2) | ALE1616A | 2023-12 |

**Table 10. Experimental instruments**

| Name | Company | Cat. No./model |
|---|---|---|
| Nanodrop | Thermo | Nanodrop One |
| qPCR system | ABI | 7500 Fast |
| PCR gradient gene amplification system | DLAB | TC1000-G |
| Fully automated nucleic acid extraction system | ALL SHENG | Auto-Pure96 |

### Experimental steps:

### (I) Cell plating

### 1. Experimental preparation

1.1. MLE-12 cell preparation: The cells were purchased from Haixing Biosciences. Cells adhering to the wall needed to be counted after complete digestion. If the cell viability was greater than or equal to 95%, the cells could be used.

1.2. Special culture medium for MLE-12 cells: The culture medium was purchased from Haixing Biosciences, stored at 4 °C, and, before the experiment, taken out and equilibrated to room temperature.

1.3. 96-well cell culture plates.

1.4. PBS and trypsin were stored at 4 °C and, before the experiment, taken out and equilibrated to room temperature.

### 2. Cell plating

The day before transfection, MLE-12 cells were seeded at a density of 1.5 × 10⁴ cells/well into a 96-well plate with 100 µL of culture medium per well.

2.1. The culture medium was incubated in a 37 °C water bath for 20 min for later use.

2.2. The cells were washed once with PBS. After trypsin was added, the cells were placed in an incubator and digested for 2 min. The culture medium was added to stop the digestion. The cells were centrifuged and counted. 20 µL of uniform cell suspension was pipetted and well mixed with 1 µL of cell counting dye. The mixture was left to stand for 1 min to stain the cells. 10 µL of the suspension was pipetted onto a cell counting plate, and the number of viable cells (green) was calculated.

2.3. According to the cell counting result, a proper volume of culture medium was added. 100 µL was taken and added to the 96-well plate, ensuring that the cell amount was 1.5 × 10⁴ cells/well. 200 µL of PBS was added to the wells on the periphery to seal them, preventing the culture medium from volatilizing. The cell plate was placed in a 5% CO₂ incubator at 37 °C and incubated overnight. The next day, transfection was performed.

### (II) Cell transfection experiment

### 1. Experimental preparation

1.1. siRNA sample preparation: siRNA samples were quantified to 1 µM and stored at -20 °C until use. They needed to be briefly centrifuged before use.

1.2. Opti-MEM culture medium and the transfection reagent Lipofectamine RNAi Max were stored at 4 °C.

1.3. 8-tube strips.

### 2. Cell transfection experiment

2.1. Before transfection, the special culture medium for MLE-12 cells was preheated, and the culture medium in the cell plate was changed to the special culture medium for MLE-12 cells, 90 µL/well.

2.2. siRNA dilution: The siRNAs were taken out from the -20 °C place, thawed, mixed well, and diluted to different concentrations according to Table 11 as working solutions for later use. The solutions were prepared fresh before use. An 8-tube strip was taken and marked as Tube A: 40 µL of Opti-MEM was added to each well, and 10 µL, of siRNA (stock solution) was added to the well with the highest concentration. After thorough mixing, 10 µL. was taken out and serially diluted to obtain different concentrations of siRNA. The dilutions were incubated at room temperature for 15 min and added to the 96-well plate (with final concentrations of 10 nM, 1 nM, 0.1 nM, and 0 nM, respectively).

**Table 11. A multi-concentration dilution protocol for siRNA samples**

| Final concentration of siRNA (nM) | Add Opti-MEM and siRNA |
|---|---|
| 10 | 10 µL of siRNA (stock solution) + 40 µL of Opti-MEM |
| 1 | 10 µL siRNA + 40 µL Opti-MEM |
| 0.1 | 10 µL siRNA + 40 µL Opti-MEM |

2.3. Preparation of Lipofectamine RNAi Max: Lipofectamine RNAi Max was diluted with Opti-MEM and left to stand for 5 min. The specific preparation ratio was adding 0.9 µL of Lipofectamine RNAi Max to 15 µL of Opti-MEM.

2.4. The prepared Lipofectamine RNAi Max was aliquoted into an 8-tube strip, with 30 µL per tube. After the aliquots were well pipetted (bubble formation was avoided), 30 µL of siRNA at different concentrations was taken from Tube A and added to the 8-tube strip containing Lipofectamine RNAi Max, and the mixtures were incubated at room temperature for 20 min.

2.5. The above mixtures were added to the culture plate at 10 µL/well, with 3 replicate wells set for each concentration.

2.6. The place was incubated in a CO₂ incubator at 37 °C for 24 h.

### (III) Sample collection and detection

1. After 24 h, cells were collected, and total cellular RNA extraction was performed using a high-throughput cellular RNA extraction kit.
2. Reverse transcription and Q-PCR detection.

In the quantitative real-time PCR detection, an SYBR Green detection experiment was used. Information about the primers is shown in Table 12.

**Table 12. The sequences of the primers**

| | |
|---|---|
| GAPDH-MF | AGGTCGGTGTGAACGGATTTG |
| GAPDH-MR | GGGGTCGTTGATGGCAACA |
| MUC5B-MF | TCCCTAGCATGAGCGCCTTA |
| MUC5B-MR | CCACGACGCAGTTGGATGTT |

### 3. Results analysis

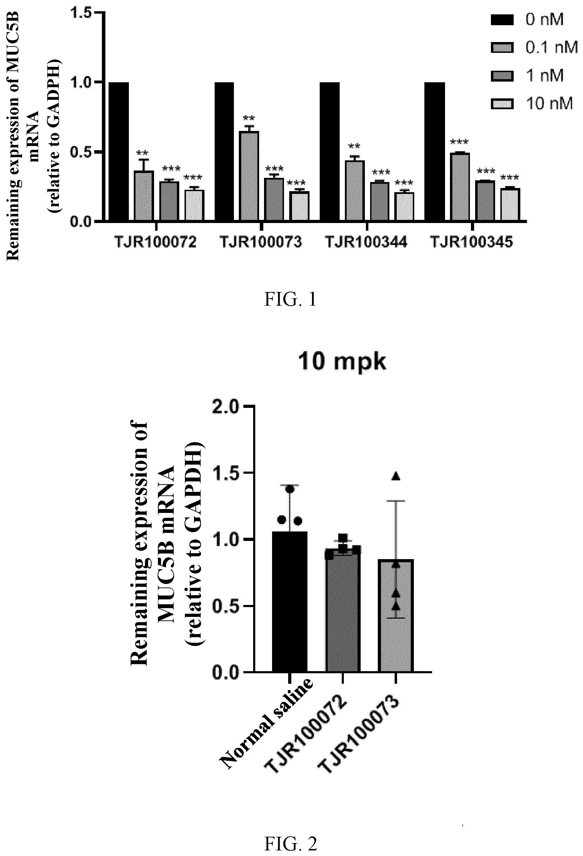

After the Q-PCR detection experiment was completed, the corresponding Ct values were obtained based on the threshold automatically set by the system. The expression of a certain gene can be relatively quantified by comparing the Ct values. Comparing Ct refers to calculating the differences in gene expression through the differences from the Ct value of the internal reference gene, which is also referred to as 2^{-△△Ct}, where ΔΔCt = [(Ct target gene in experimental group - Ct internal reference in experimental group) - (Ct target gene in control group - Ct internal reference in control group)]. Inhibition rate (%) = (1 - remaining level of target gene expression) × 100%. The experimental results are shown in Table 13 and FIG. 1.

It can be seen from the results that TJR100072, TJR100073, TJR100344, and TJR100345 exhibited the same level of inhibition of MUC5B mRNA in the cell transfection experiment.

**Table 13. mRNA expression levels in cells in the treatment groups at different concentrations**

| | 0 nM | | 0.1 nM | | 1nM | | 10 nM | |
|---|---|---|---|---|---|---|---|---|
| TJR100072 | 1.00 | 1.00 | 0.42 | 0.31 | 0.30 | 0.28 | 0.24 | 0.22 |
| TJR100073 | 1.00 | 1.00 | 0.62 | 0.67 | 0.33 | 0.30 | 0.23 | 0.20 |
| TJR100344 | 1.00 | 1.00 | 0.46 | 0.42 | 0.29 | 0.28 | 0.20 | 0.22 |
| TJR100345 | 1.00 | 1.00 | 0.50 | 0.49 | 0.29 | 0.29 | 0.23 | 0.24 |

### Example 4: Inhibitory Activity of Oligonucleotides of the Present Disclosure Against Mouse MUC5B Gene

The specific steps were as follows: C57BL/6N female mice aged 6 weeks (purchased from Zhejiang Vital River Laboratory Animal Technology Co., Ltd.) were acclimatized for one week before the experiment. On day 0, before administration, the compounds were prepared in normal saline, and each mouse was dosed at 10 mg/kg. Twelve mice were weighed and divided into 3 groups of 4: a normal saline group, TJR100072-10mpk, and TJR100073-10mpk. After anesthesia with isoflurane, the mice were intratracheally given the test compounds using a mouse pulmonary administration apparatus (purchased from Yuyan Instruments). On day 10, the mice were euthanized, and the whole lungs were collected after cardiac blood collection, cut into small pieces, and then placed in RNA Later. The MUC5B mRNA levels in the whole lungs were determined by RT-PCR. The experimental results are shown in Table 14 and FIG. 2.

It can be seen from the results that, compared to the normal saline group, TJR100072, without lipid delivery, did not exhibit significant inhibitory activity, while the positive molecule TJR100073 exhibited relatively weak inhibitory activity.

**Table 14. Expression levels of mouse MUC5B mRNA**

| | Normal saline | TJR100072 | TJR100073 |
|---|---|---|---|
| 4 animals | 1.15 | 1.01 | 0.60 |
| | 0.56 | 0.92 | 0.82 |
| | 1.14 | 0.93 | 1.48 |
| | 1.38 | 0.88 | 0.50 |
| Mean | 1.06 | 0.94 | 0.85 |

### Example 5: Inhibitory Activity of Oligonucleotides of the Present Disclosure Against Mouse MUC5B Gene

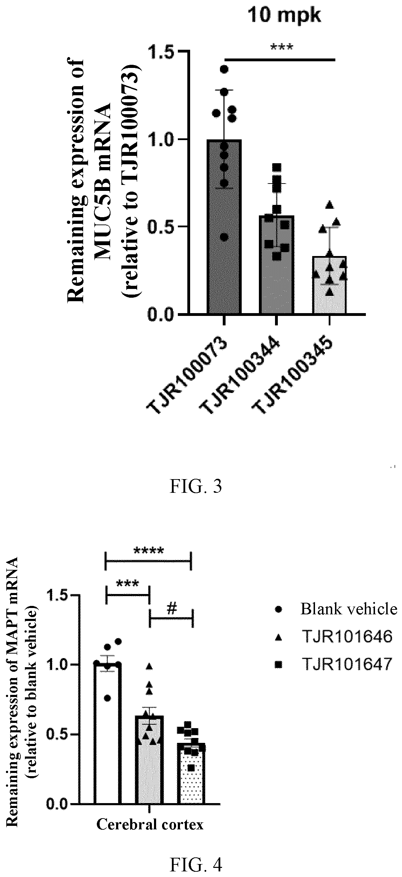

The specific steps were as follows: C57BL/6N female mice aged 6 weeks (purchased from Zhejiang Vital River Laboratory Animal Technology Co., Ltd.) were acclimatized for one week before the experiment. On day 0, before administration, the compounds were prepared in normal saline, and each mouse was dosed at 10 mg/kg. Thirty mice were weighed and divided into 3 groups of 10: TJR100073-10mpk, TJR100344-10mpk, and TJR100345-10mpk. After anesthesia with isoflurane, the mice were intratracheally given the test compounds using a mouse pulmonary administration apparatus (Yuyan Instruments). On day 10, the mice were euthanized, and the whole lungs were collected after cardiac blood collection, cut into small pieces, and then placed in RNA Later. The MUC5B mRNA levels in the whole lungs were determined by RT-PCR. The experimental results are shown in Table 15 and FIG. 3.

It can be seen from the results that both TJR100344 and TJR100345 exhibited better inhibitory activity than the positive molecule TJR100073.

**Table 15. Expression levels of mouse MUC5B mRNA**

| | TJR100073 | TJR100344 | TJR100345 |
|---|---|---|---|
| 10 animals | 1.27 | 0.38 | 0.27 |
| | 0.96 | 0.51 | 0.53 |
| | 0.84 | 0.40 | 0.22 |
| | 1.40 | 0.72 | 0.13 |
| | 1.15 | 0.60 | 0.49 |
| | 1.17 | 0.84 | 0.29 |
| | 1.12 | 0.55 | 0.35 |
| | 0.91 | 0.33 | 0.63 |
| | 0.44 | 0.77 | 0.23 |
| | 0.75 | - | 0.20 |
| Mean | 1.00 | 0.57 | 0.33 |

### Example 6: Evaluation of In Vitro Anti-APP Activity of Oligonucleotides of the Present Disclosure Using A172 Cells

The *in vitro* anti-APP activity of the oligonucleotides of the present disclosure was assessed using 7 concentration gradients in A172 cells (purchased from Immocell Biotechnology).

A172 cells were cultured at 37 °C with 5% CO₂ in a Dulbecco's modified eagle medium containing 10% fetal bovine serum. 24 h before transfection, A172 cells were seeded at a density of 1 × 10⁴ cells/well into a 96-well plate with 100 µL of culture medium per well. The cells were transfected with the oligonucleotides of the present disclosure using Lipofectamine RNAi MAX (ThermoFisher, 13778150) by following the instructions of the product, with two replicate wells set for each concentration. After 48 h of treatment, total cellular RNA extraction was performed using an FG0417-L/FG0418-XI, high-throughput cellular RNA extraction kit (FireGen, magnetic bead method), followed by an RNA reverse transcription experiment (Takara, RR037A) and quantitative real-time PCR (Thermo, 4444557) detection. The mRNA level of human APP was determined and corrected based on the GAPDH internal reference gene level.

The instruments involved in this experiment are shown in Table 16.

In the real-time quantitative PCR detection, a probe Q-PCR detection experiment was used. Information about the primers is shown in Table 17.

**Table 16. Experimental instruments**

| Name | Company | Cat. No./model |
|---|---|---|
| Nanodrop | Thermo | Nanodrop One |
| qPCR system | ABI | 7500 Fast |
| PCR system | Life | Pro-Flex PCR system |

In the real-time quantitative PCR detection, a probe Q-PCR detection experiment was used. Information about the primers is shown in Table 17.

**Table 17. Taqman primer information**

| Primer name | Primer sequence (5'-3') |
|---|---|
| hAPP-Forward | GACAGACAGCACACCCTAAA |
| hAPP-Reverse | CACACGGAGGTGTGTCATAA |
| hAPP-Probe | 5'6-FAM-ATCCCAAGAAAGCCGCTCAGATCC-3'BHQ1 |
| hGAPDH-Forward | GACCCCTTCATTGACCTCAACTAC |
| hGAPDH-Reverse | TTGACGGTGCCATGGAATTT |
| hGAPDH-Probe | 5'VIC-TTACATGTTCCAATATGATTCC-3'BHQ1 |

### Results analysis method:

After the Q-PCR detection experiment was completed, the corresponding Ct values were obtained based on the threshold automatically set by the system. The expression of a certain gene can be relatively quantified by comparing the Ct values. Comparing Ct refers to calculating the differences in gene expression through the differences from the Ct value of the internal reference gene, which is also referred to as 2^{-△△Ct}, where ΔΔCt = [(Ct target gene in experimental group - Ct internal reference in experimental group) - (Ct target gene in control group - Ct internal reference in control group)]. Inhibition rate (%) = (1 - remaining level of target gene expression) × 100%. The results are expressed in terms of the remaining percentage of human APP mRNA expression relative to the cells in the MOCK group (untreated group). The inhibition rate IC₅₀ results are shown in Table 18.

It can be seen from the results that the oligonucleotides of the present disclosure exhibited strong inhibitory effects on APP mRNA.

**Table 18. Remaining percentages of APP mRNA expression in cells in the treatment groups at different concentrations and IC₅₀**

| Oligonucleoti de | Remaining percentage of APP mRNA expression (mean, %) | | | | | | | IC₅₀, nM |
|---|---|---|---|---|---|---|---|---|
| | 10 nM | 2 nM | 0.4 nM | 0.08 nM | 0.016 nM | 0.0032 nM | 0.00064 nM | |
| TJR102161 | 10.95 | 46.43 | 79.66 | 90.45 | 90.52 | 85.54 | 91.33 | 1.758 |
| TJR102162 | 7.45 | 43.46 | 94.06 | 108.12 | 103.77 | 104.96 | 96.78 | 1.706 |
| TJR102178 | 3.69 | 12.56 | 57.47 | 101.39 | 105.78 | 100.35 | 105.17 | 0.468 |
| TJR102179 | 3.82 | 7.60 | 46.73 | 93.41 | 103.42 | 98.42 | 105.54 | 0.366 |
| TJR102180 | 3.38 | 7.72 | 42.81 | 79.95 | 91.03 | 85.59 | 88.05 | 0.319 |

### Example 7: Evaluation of In Vitro Anti-PMP22 Activity of Oligonucleotides of the Present Disclosure Using A172 Cells

The *in vitro* anti-PMP22 activity of the oligonucleotides of the present disclosure was assessed using 7 concentration gradients in A172 cells.

A172 cells were cultured at 37 °C with 5% CO₂ in a Dulbecco's modified eagle medium containing 10% fetal bovine serum. 24 h before transfection, A172 cells were seeded at a density of 1 × 10⁴ cells/well into a 96-well plate with 100 µL of culture medium per well.

The cells were transfected with the oligonucleotides of the present disclosure using Lipofectamine RNAi MAX (ThermoFisher, 13778150) by following the instructions of the product, with two replicate wells set for each concentration. After 48 h of treatment, total cellular RNA extraction was performed using an FG0417-L/FG0418-XI, high-throughput cellular RNA extraction kit (FireGen, magnetic bead method), followed by an RNA reverse transcription experiment (Takara, RR037A) and quantitative real-time PCR (Thermo, 4444557) detection. The mRNA level of human PMP22 was determined and corrected based on the GAPDH internal reference gene level.

The instruments involved in this experiment are shown in Table 16 above.

In the real-time quantitative PCR detection, a probe Q-PCR detection experiment was used. Information about the primers is shown in Table 19.

**Table 19. Taqman primer information**

| Primer name | Primer sequence (5'-3') |
|---|---|
| hPMP22-Forward | GTGGCATCTCAACTCGGATTAC |
| hPMP22-Reverse | CGTTTCCGCAAGATCACATAGA |
| hPMP22-Probe | 5'6-FAM-CTACGGTTTCGCCTACATCCTGGC-3'BHQ1 |
| hGAPDH-Forward | GACCCCTTCATTGACCTCAACTAC |
| hGAPDH-Reverse | TTGACGGTGCCATGGAATTT |
| hGAPDH-Probe | 5'VIC-TTACATGTTCCAATATGATTCC-3'BHQ1 |

### Results analysis method:

After the Q-PCR detection experiment was completed, the corresponding Ct values were obtained based on the threshold automatically set by the system. The expression of a certain gene can be relatively quantified by comparing the Ct values. Comparing Ct refers to calculating the differences in gene expression through the differences from the Ct value of the internal reference gene, which is also referred to as 2^{-△△Ct}, where ΔΔCt = [(Ct target gene in experimental group - Ct internal reference in experimental group) - (Ct target gene in control group - Ct internal reference in control group)]. Inhibition rate (%) = (1 - remaining level of target gene expression) × 100%. The results are expressed in terms of the remaining percentage of human PMP22 mRNA expression relative to the cells in the MOCK group (untreated group). The inhibition rate IC₅₀ results are shown in Table 20.

It can be seen from the results that the oligonucleotides of the present disclosure exhibited strong inhibitory effects on PMP22 mRNA.

**Table 20. Remaining percentages of PMP22 mRNA expression in cells in the treatment groups at different concentrations and IC₅₀**

| Oligonucleotide | Remaining percentage of PMP22 mRNA expression (mean, %) | | | | | | | IC₅₀, nM |
|---|---|---|---|---|---|---|---|---|
| | 10 nM | 2 nM | 0.4 nM | 0.08 nM | 0.016 nM | 0.0032 nM | 0.00064 nM | |
| TJR102149 | 5.80 | 6.21 | 9.75 | 22.54 | 65.72 | 98.68 | 112.75 | 0.0260 |
| TJR102150 | 6.56 | 5.91 | 10.37 | 25.29 | 64.78 | 96.74 | 110.01 | 0.0269 |
| TJR102151 | 5.71 | 6.88 | 10.60 | 25.09 | 66.64 | 95.42 | 100.17 | 0.0290 |

### Example 8: Evaluation of In Vitro Anti-MAPT Activity of Oligonucleotides of the Present Disclosure Using Neuro2α Cell Free Uptake

An *in vitro* free uptake experiment was performed on the oligonucleotides of the present disclosure using 5 concentrations (1000 nM, 300 nM, 30 nM, 3 nM, and 0 nM) in Neuro2α cells (purchased from Shanghai Genetimes ExCell Biotechnology Co. Ltd.) to assess their *in vitro* anti-MAPT activity.

Neuro2α cells were cultured at 37 °C with 5% CO2 in an Eagle's Minimum Essential Medium (EMEM) containing 10% fetal bovine serum. 24 h before free uptake, Neuro2α cells were seeded at a density of 1.5 × 10⁴ cells/well into a 96-well plate with 100 µL of culture medium per well. On day 2, the oligonucleotides of the present disclosure were diluted in a 2% fetal bovine serum culture medium, with final concentrations of 1000 nM, 300 nM, 30 nM, 3 nM, and 0 nM. After the cells were incubated with the oligonucleotides of the present disclosure for 72 h, total cellular RNA extraction was performed using an FG0417-L/FG0418-XI, high-throughput cellular RNA extraction kit (FireGen, magnetic bead method), followed by an RNA reverse transcription experiment (Takara, RR037A) and quantitative real-time PCR (Thermo, 4444557) detection. The mRNA level of mouse MAPT was determined and corrected based on the mouse GAPDH internal reference gene level.

The instruments involved in this experiment are shown in Table 16 above.

In the real-time quantitative PCR detection, a probe Q-PCR detection experiment was used. Information about the primers is shown in Table 21.

**Table 21. Taqman primer information**

| Primer name | Primer sequence (5'-3') |
|---|---|
| mMAPT-Forward | CTCTCAGGTTTGTGGAGGTAATC |
| mMAPT-Reverse | CCAGGGTTCTTAGGAGGTATTTG |
| mMAPT-Probe | 5' 6-FAM-CTTCTCTTCCTGCT-3'BHQ1 |
| mGAPDH-Forward | AACAGCAACTCCCACTCTTC |
| mGAPDH-Reverse | CCTGTTGCTGTAGCCGTATT |
| mGAPDH-Probe | 5'VIC-AAAGTTGTCATTGAGAGCAATGCCAGC-3'BHQ1 |

### Results analysis method:

After the Q-PCR detection experiment was completed, the corresponding Ct values were obtained based on the threshold automatically set by the system. The expression of a certain gene can be relatively quantified by comparing the Ct values. Comparing Ct refers to calculating the differences in gene expression through the differences from the Ct value of the internal reference gene, which is also referred to as 2^{-△△Ct}, where ΔΔCt = [(Ct target gene in experimental group - Ct internal reference in experimental group) - (Ct target gene in control group - Ct internal reference in control group)]. Inhibition rate (%) = (1 - remaining level of target gene expression) × 100%. The results are expressed in terms of the remaining percentage of mouse MAPT mRNA expression relative to the cells in the MOCK group (untreated group). The experimental results are shown in Table 22.

**Table 22. The anti-MAPT activity of the oligonucleotides of the present disclosure in Neuro2α cells**

| Oligonucleotide | Remaining percentage of MAPT mRNA expression (%) | | | | | | | | | |
|---|---|---|---|---|---|---|---|---|---|---|
| | 1000 nM | | 300 nM | | 30 nM | | 3 nM | | 0 nM | |
| TJR102217 | 55.5 | 59.7 | 53 | 55.4 | 67.7 | 65.2 | 85 | 74.6 | 96.3 | 96.7 |
| TJR102218 | 51.4 | 53.4 | 57.6 | 52.6 | 72.8 | 63.2 | 78.8 | 79.5 | 96.4 | 101.2 |
| TJR102219 | 53.1 | 60.7 | 58.4 | 51.2 | 63.1 | 61.9 | 75.8 | 72.9 | 98.4 | 94.8 |

It can be seen from the results that the oligonucleotides of the present disclosure exhibited strong inhibitory effects on MAPT mRNA in the cell free uptake experiment.

### Example 9: Inhibitory Activity of Oligonucleotides of the Present Disclosure Against Mouse MAPT Gene

The specific steps were as follows: C57BL/6J male mice aged 8 weeks (purchased from Zhejiang Vital River Laboratory Animal Technology Co., Ltd.) were acclimatized for one week before the experiment. On day 0, before administration, the compounds were prepared in normal saline, and each mouse was dosed at 5 mg/kg. Mice were weighed and divided into 3 groups of 6-10: a blank vehicle group, TJR101646-5mpk, and TJR101647-5mpk. After anesthesia with Zoletil, unilateral intracerebroventricular (ICV) administration was performed using a stereotaxic apparatus (RWD). A standard sterile surgical procedure was used to perform a single 2-µL unilateral injection into the right lateral ventricle at the following position: AP = -0.2 mm (posterior to bregma), ML = -1.0 mm, and DV = +2.4 mm. After a midline incision was made in the scalp, a burr hole was created at the selected coordinates using the stereotaxic coordinates and a cranial drill. Subsequently, the dura mater was punctured at the center of the burr hole, and the needle was lowered to the specified depth to reach the lateral ventricle. The glass electrode filling volume was slightly greater than 2 µL. The administration volume was 2 µL, and the injection rate was 1 µL/min. After infusion, the needle was left in place and stabilized for 2 min, and the needle was slowly withdrawn. When the needle tip was within the cortical region, the withdrawal was paused for 30 s. On day 14, the mice were euthanized, and their cerebral cortex tissues were collected, snap-frozen in liquid nitrogen, and then stored at -80 °C. The MAPT mRNA levels in the cerebral cortexes of the mice were determined by RT-PCR. The experimental results are shown in Table 23 and FIG. 4.

It can be seen from the results that TJR101647 exhibited better inhibitory activity than the positive molecule TJR101646.

**Table 23. The inhibitory activity of TJR101647 against mouse MAPT mRNA**

| Endpoint | | Group | Cerebral cortex |
|---|---|---|---|
| Day 14 | MAPT expression level (mean) | Blank vehicle | 1.01 |
| | | TJR101646 | 0.634 |
| | | TJR101647 | 0.44 |
| | Inhibition rate% (relative to blank vehicle) | TJR101646 | 37.23 |
| | | TJR101647 | 56.44 |

### Example 10: Inhibitory Activity of Oligonucleotides of the Present Disclosure Against Mouse MAPT Gene

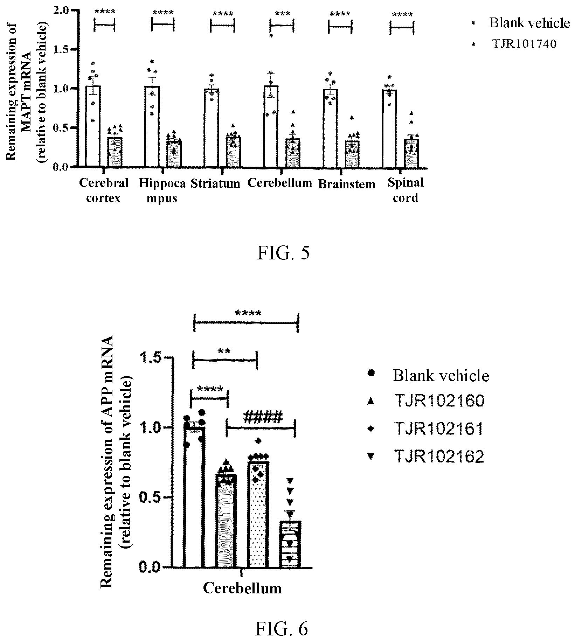

The specific steps were as follows: C57BL/6J male mice aged 8 weeks (purchased from Zhejiang Vital River Laboratory Animal Technology Co., Ltd.) were acclimatized for one week before the experiment. On day 0, before administration, the compounds were prepared in normal saline, and each mouse was dosed at 5 mg/kg. Mice were weighed and divided into 2 groups of 6-10: a blank vehicle group and TJR101740-5mpk. After anesthesia with Zoletil, unilateral intracerebroventricular (ICV) administration was performed using a stereotaxic apparatus (RWD). A standard sterile surgical procedure was used to perform a single 2-µL unilateral injection into the right lateral ventricle at the following position: AP = -0.2 mm (posterior to bregma), ML = -1.0 mm, and DV = +2.4 mm. After a midline incision was made in the scalp, a burr hole was created at the selected coordinates using the stereotaxic coordinates and a cranial drill. Subsequently, the dura mater was punctured at the center of the burr hole, and the needle was lowered to the specified depth to reach the lateral ventricle. The glass electrode filling volume was slightly greater than 2 µL. The administration volume was 2 µL, and the injection rate was 1 µL/min. After infusion, the needle was left in place and stabilized for 2 min, and the needle was slowly withdrawn. When the needle tip was within the cortical region, the withdrawal was paused for 30 s. On day 14, the mice were euthanized, and their brain tissues were collected, snap-frozen in liquid nitrogen, and then stored at -80 °C. The MAPT mRNA level in each brain tissue of the mice was determined by RT-PCR. The experimental results are shown in Table 24 and FIG. 5.

It can be seen from the results that TJR101740 exhibited strong inhibitory activity against the MRNA level of MAPT in each brain tissue of the mice after unilateral intracerebroventricular administration.

**Table 24. The inhibitory activity of TJR101740 against mouse MAPT mRNA**

| Endpoint | | Group | Cerebral cortex | Hippocampus | Striatum | Cerebellum | Brainstem | Spinal cord |
|---|---|---|---|---|---|---|---|---|
| Day 14 | MAPT expression level (mean) | Blank vehicle | 1.038 | 1.033 | 1.007 | 1.052 | 1.01 | 1.005 |
| | | TJR101740 | 0.382 | 0.338 | 0.393 | 0.375 | 0.353 | 0.377 |
| | Inhibition rate% (relative to blank vehicle) | TJR101740 | 63.2 | 67.3 | 61 | 64.4 | 65 | 62.5 |

### Example 11: Inhibitory Activity of Oligonucleotides of the Present Disclosure Against Mouse APP Gene

The specific steps were as follows: C57BL/6J male mice aged 8 weeks (purchased from Zhejiang Vital River Laboratory Animal Technology Co., Ltd.) were acclimatized for one week before the experiment. On day 0, before administration, the compounds were prepared in normal saline, and each mouse was dosed at 5 mg/kg. Mice were weighed and divided into 4 groups of 6-8: a blank vehicle group, TJR102160, TJR102161, and TJR102162. After anesthesia with Zoletil, unilateral intracerebroventricular (ICV) administration was performed using a stereotaxic apparatus (RWD). A standard sterile surgical procedure was used to perform a single 2-µL unilateral injection into the right lateral ventricle at the following position: AP = -0.2 mm (posterior to bregma), ML = -1.0 mm, and DV = +2.4 mm. After a midline incision was made in the scalp, a burr hole was created at the selected coordinates using the stereotaxic coordinates and a cranial drill. Subsequently, the dura mater was punctured at the center of the burr hole, and the needle was lowered to the specified depth to reach the lateral ventricle. The glass electrode filling volume was slightly greater than 2 µL. The administration volume was 2 µL, and the injection rate was 1 µL/min. After infusion, the needle was left in place and stabilized for 2 min, and the needle was slowly withdrawn. When the needle tip was within the cortical region, the withdrawal was paused for 30 s. On day 7, the mice were euthanized, and their cerebellum tissues were collected, snap-frozen in liquid nitrogen, and then stored at -80 °C. The APP mRNA levels in the cerebella of the mice were determined by RT-PCR. The experimental results are shown in Table 25 and FIG. 6.

**Table 25. The inhibitory activity of the oligonucleotides of the present disclosure against mouse APP mRNA**

| Endpoint | | Group | Cerebellum |
|---|---|---|---|
| Day 7 | MAPT expression level (mean) | Blank vehicle | 1.007 |
| | | TJR102160 | 0.67 |
| | | TJR102161 | 0.764 |
| | | TJR102162 | 0.339 |
| | Inhibition rate% (relative to blank vehicle) | TJR102160 | 33.47 |
| | | TJR102161 | 24.15 |
| | | TJR102162 | 66.36 |

It can be seen from the results that the oligonucleotides of the present disclosure exhibited strong inhibitory effects on APP mRNA in the *in vivo* mouse experiment.

## Claims

1. An oligonucleotide, comprising at least one compound represented by formula (I) or a pharmaceutically acceptable salt thereof, wherein a structure of the compound represented by formula (I) is 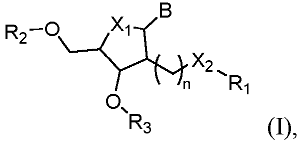 wherein:
X₁ is selected from the group consisting of O, S, N, and C atoms;
X₂ is selected from the group consisting of O and S atoms;
R₁ is selected from the group consisting of C₁₀-C₃₀ linear alkyl, and C₁₀-C₃₀ linear alkyl interrupted by one or more O or S atoms; optionally, the C₁₀-C₃₀ linear alkyl is substituted with one or more Rₐ, or optionally, C₃-₆ cycloalkyl forms between two adjacent carbon atoms of the C₁₀-C₃₀ linear alkyl;
R₂ and R₃ are identical or different and are each independently selected from the group consisting of a bond, hydrogen, an activated phosphoester group, an activated phosphite ester group, a phosphoramidite group, a solid support, an internucleotide linking group linked to the oligonucleotide, -P(=O)(OH)-O-, -P(=O)(SH)-O-, -P(=O)(OH)-O-nucleoside, -P(=O)(SH)-O-nucleoside, -P(=O)(OH)-O-oligonucleotide fragment, -P(=O)(SH)-O-oligonucleotide fragment, and a hydroxy protecting group;
each Rₐ is independently selected from the group consisting of hydrogen, deuterium, halogen, hydroxy, cyano, alkyl, haloalkyl, alkoxy, cycloalkyl, and heterocycloalkyl;
n is selected from the group consisting of 0 and 1;
B is a base;
the compound represented by formula (I) is not selected from the group consisting of: 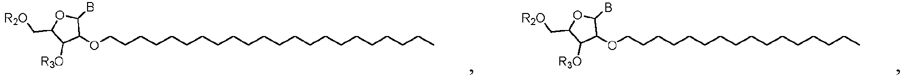 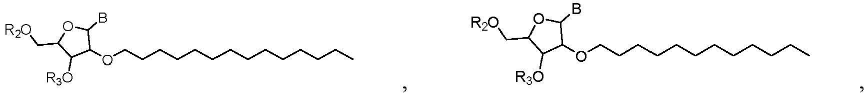 and

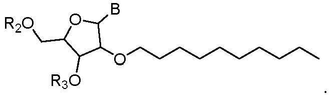

2. The oligonucleotide according to claim 1, wherein the structure of the compound represented by formula (I) is selected from the group consisting of: 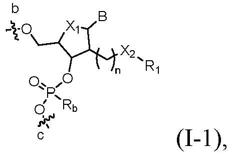 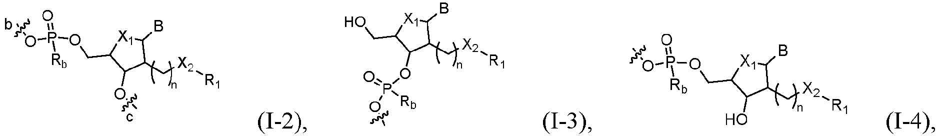 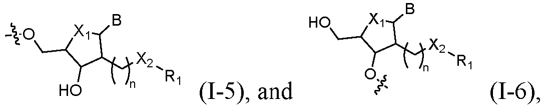 wherein R_{b} is selected from the group consisting of OH and SH, and ends b and c are linked to adjacent nucleotides on two sides of the structure of the compound represented by formula (I), respectively.

3. The oligonucleotide according to any one of claims 1 to 2, wherein X₁ is selected from the group consisting of O and S atoms, preferably from the group consisting of an O atom.

4. The oligonucleotide according to any one of claims 1 to 3, wherein X₂ is selected from the group consisting of O and S atoms, preferably from the group consisting of an O atom.

5. The oligonucleotide according to any one of claims 1 to 4, wherein R₁ is selected from the group consisting of C₁₄-C₂₄ linear alkyl, and C₁₄-C₂₄ linear alkyl interrupted by one or more O or S atoms.

6. The oligonucleotide according to any one of claims 1 to 5, wherein each Rₐ is independently selected from the group consisting of hydrogen, deuterium, halogen, C₁₋₆ alkyl, and C₁₋₆ alkoxy; preferably, each Rₐ is independently selected from the group consisting of hydrogen, deuterium, F, methyl, and methoxy.

7. The oligonucleotide according to any one of claims 1 to 6, wherein R₁ is selected from the group consisting of: 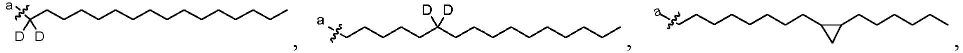 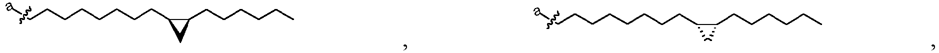 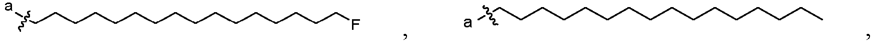 and 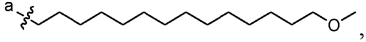 wherein end a is linked to X₂.

8. The oligonucleotide according to any one of claims 1 to 7, wherein B is selected from the group consisting of adenine, guanine, cytosine, uracil, and thymine.

9. The oligonucleotide according to any one of claims 1 to 8, wherein the structure of the compound represented by formula (1) is selected from the group consisting of: 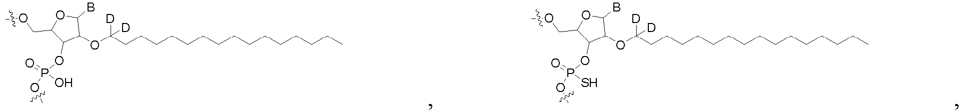 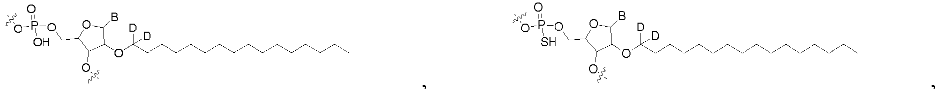 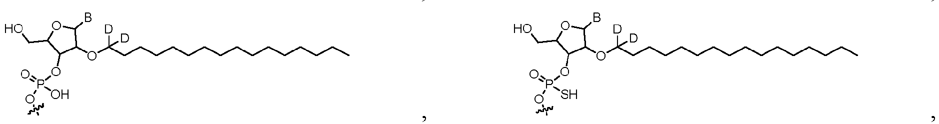 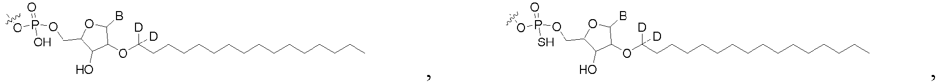 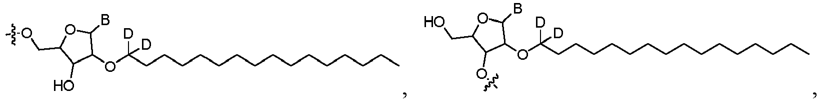 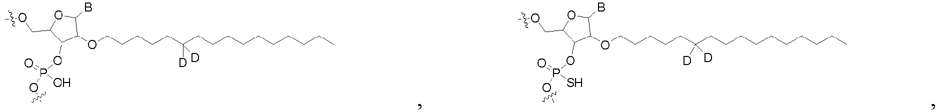 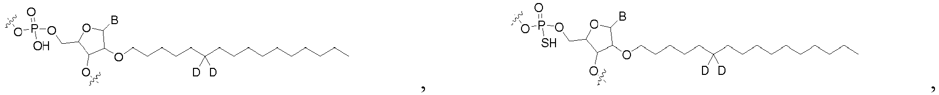 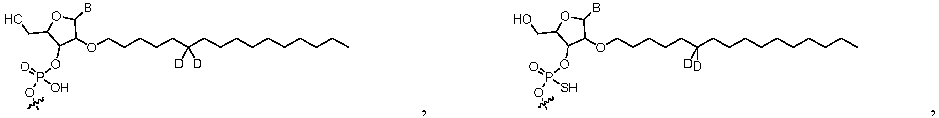 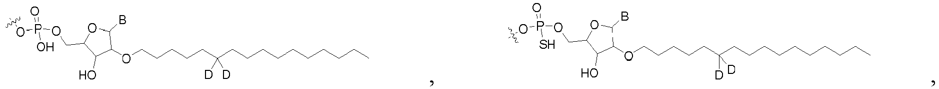 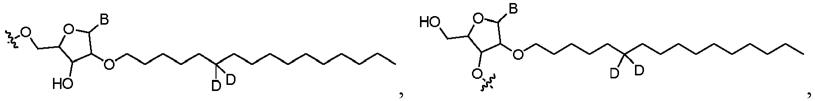 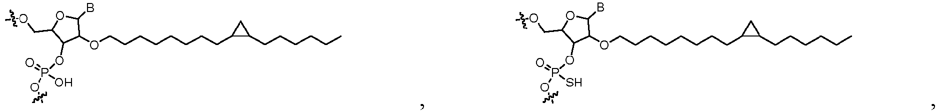 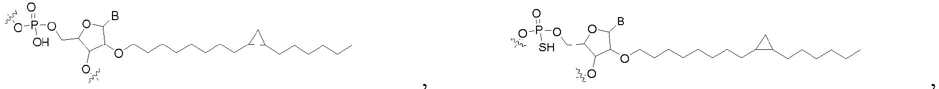 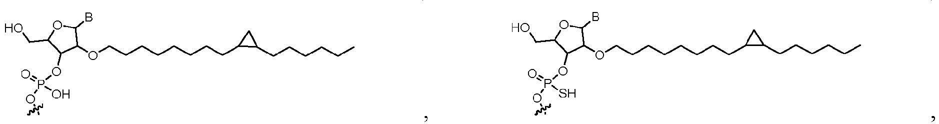 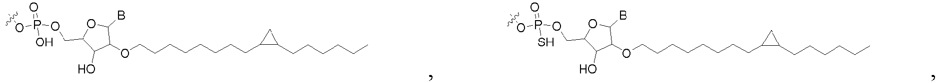 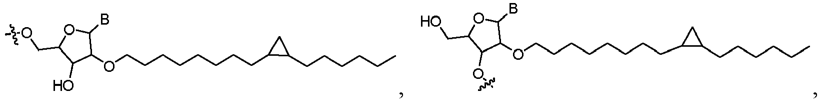 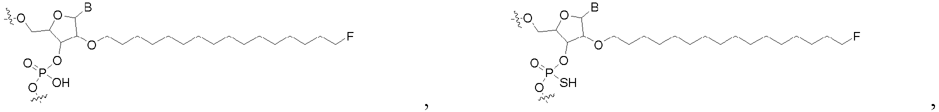 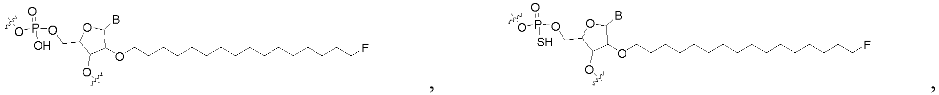 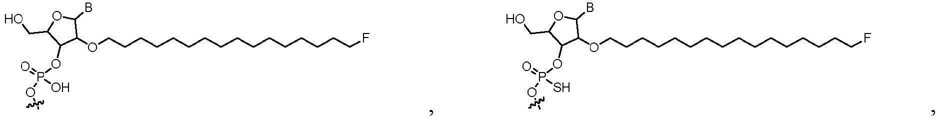 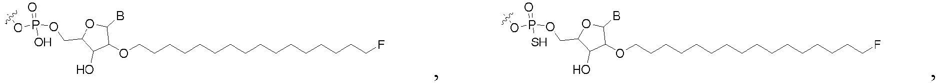 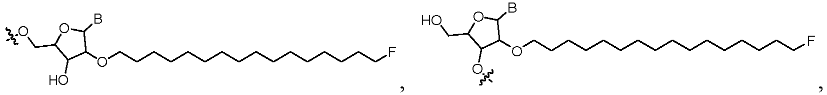 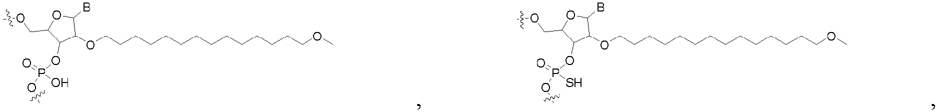 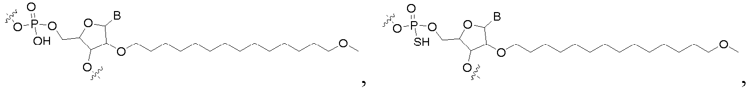 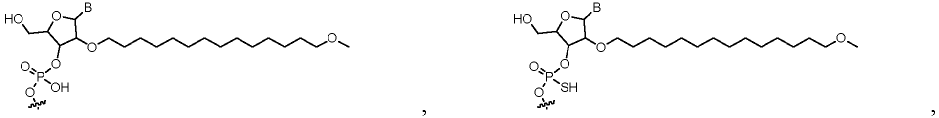 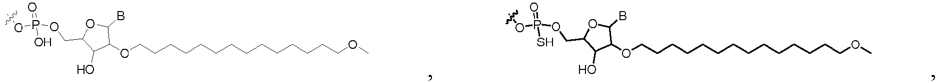 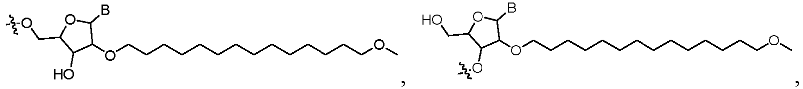 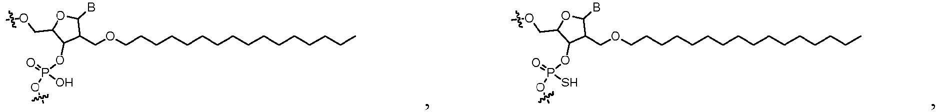 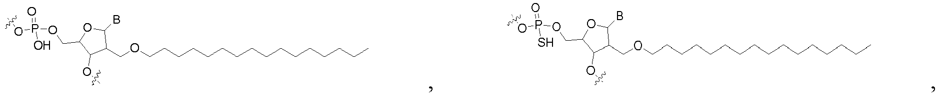 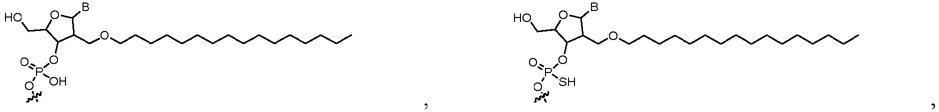 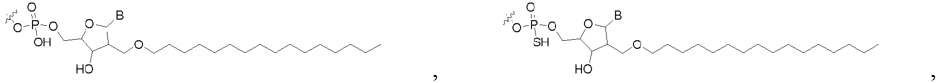 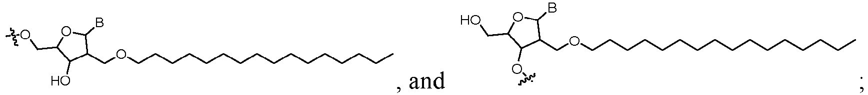 preferably, the structure of the compound represented by formula (1) is selected from the group consisting of:

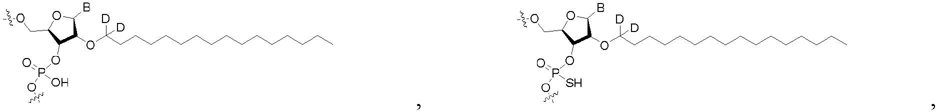

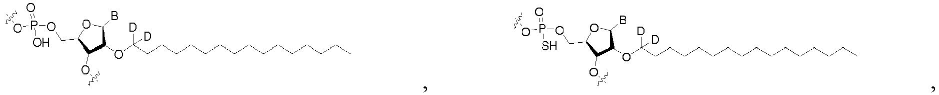

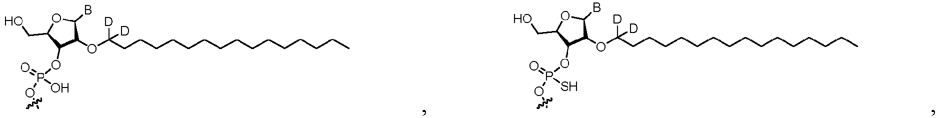

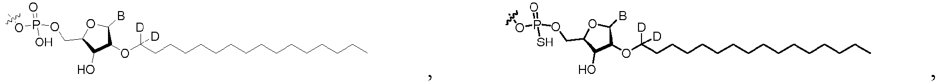

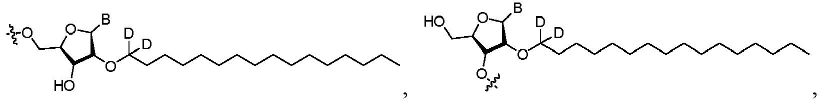

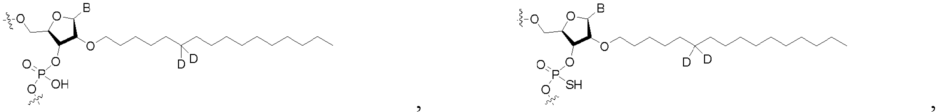

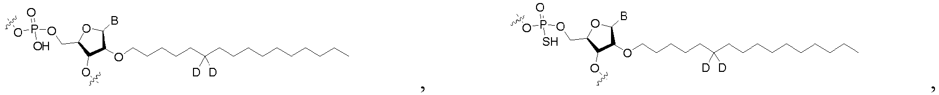

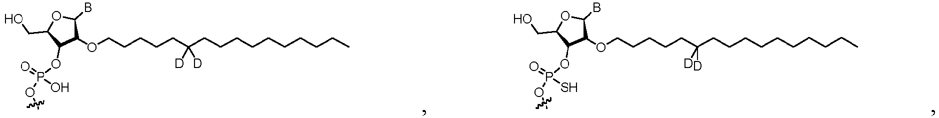

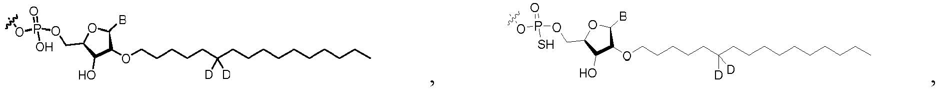

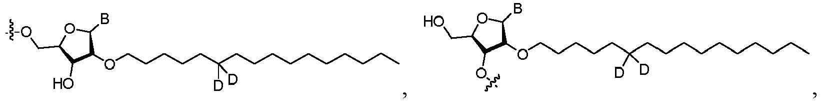

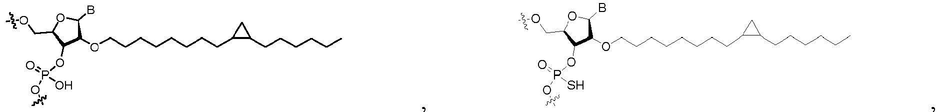

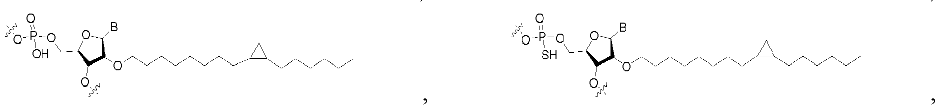

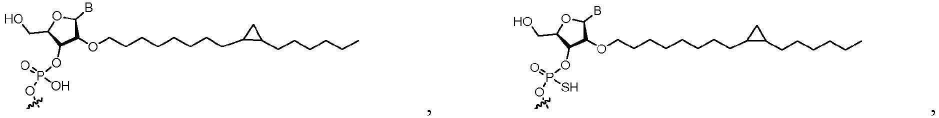

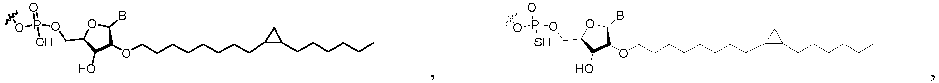

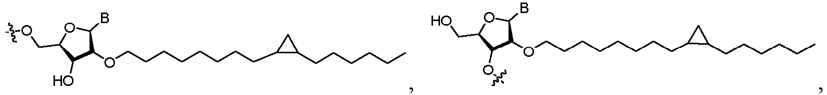

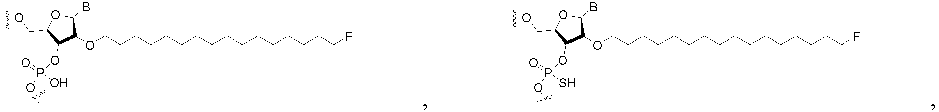

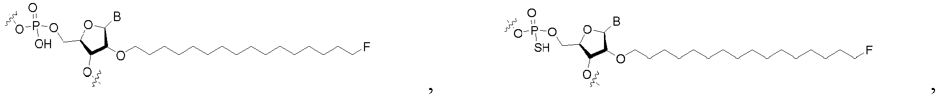

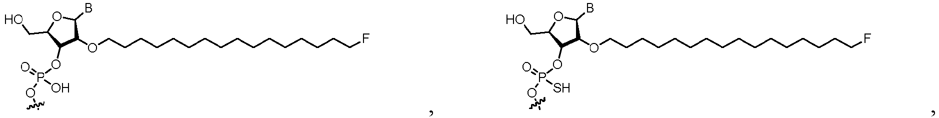

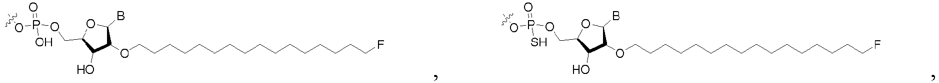

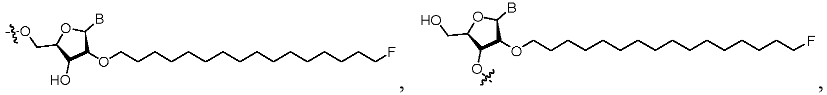

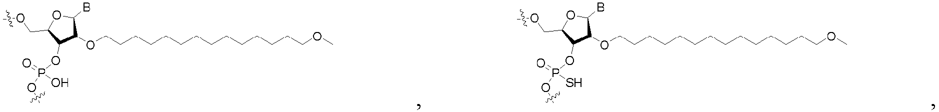

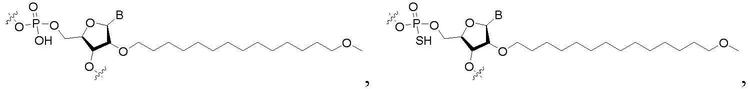

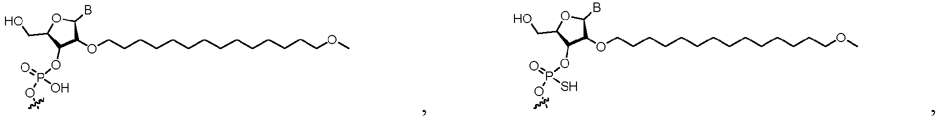

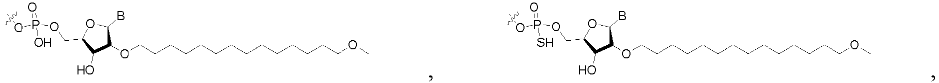

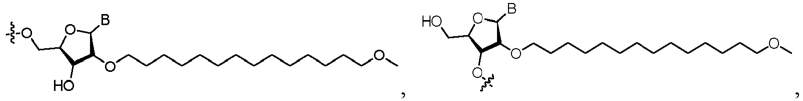

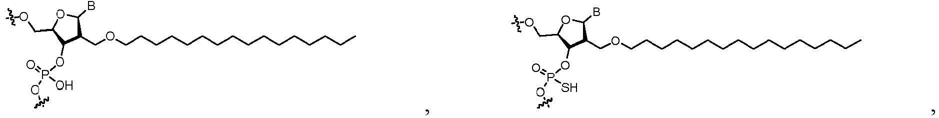

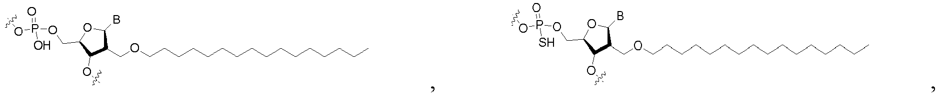

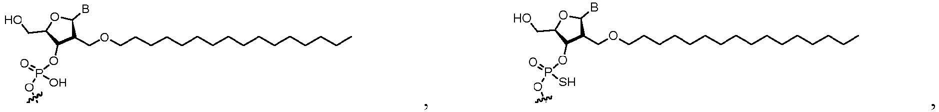

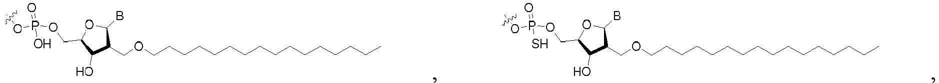

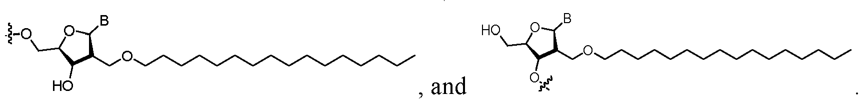

10. The oligonucleotide according to any one of claims 1 to 9, wherein the oligonucleotide comprises 1, 2, 3, or 4 compounds represented by formula (I) or pharmaceutically acceptable salts thereof, preferably, the oligonucleotide comprises 1 compound represented by formula (I) or pharmaceutically acceptable salt thereof.

11. The oligonucleotide according to any one of claims 1 to 10, wherein the oligonucleotide comprises a sense strand and an antisense strand that form a double-stranded region, and the compound represented by formula (I) or the pharmaceutically acceptable salt thereof is located at at least one of the following nucleotide positions:
position 1 of the 5' end of the sense strand;
position 1 of the 3' end of the sense strand;
position 1 of the 5' end of the antisense strand;
position 1 of the 3' end of the antisense strand;
positions 2-8 in the middle of the sense strand; and
positions 2-8 in the middle of the antisense strand;
preferably, the compound represented by formula (I) or the pharmaceutically acceptable salt thereof is located at at least one of the following nucleotide positions:
position 1 of the 5' end of the sense strand;
position 1 of the 3' end of the sense strand; and
position 6 in the middle of the sense strand.

12. The oligonucleotide according to any one of claims 2 to 11, wherein the compound represented by formula (I) or the pharmaceutically acceptable salt thereof is located at position 6 in the direction from the 5' end to the 3' end of the sense strand, end b is linked to the fifth nucleotide of the 5' end of the sense strand, and end c is linked to the seventh nucleotide of the 5' end of the sense strand, wherein the B in formula (I) represents the base of the nucleotide at position 6 in the direction from the 5' end to the 3' end of the sense strand;
or the compound represented by formula (I) or the pharmaceutically acceptable salt thereof is located at the nucleotide at position 1 of the 5' end of the sense strand, wherein B represents the base of the nucleotide at position 1 of the 5' end of the sense strand;
or the compound represented by formula (I) or the pharmaceutically acceptable salt thereof is located at the nucleotide at position 1 of the 3' end of the sense strand, wherein B represents the base of the nucleotide at position 1 of the 3' end of the sense strand.

13. The oligonucleotide according to claim 12, wherein the structure of the compound represented by formula (I) is selected from the group consisting of:

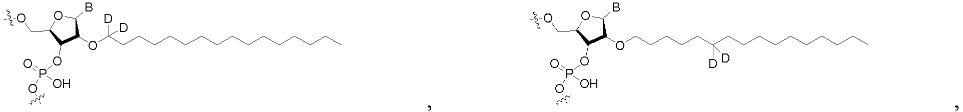

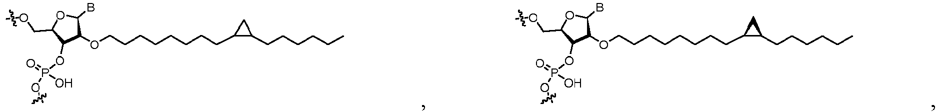

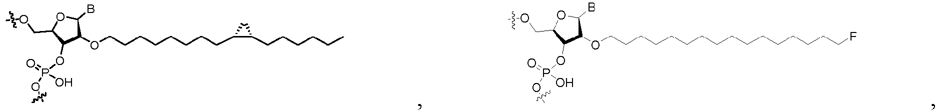

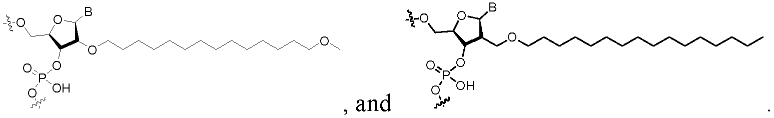

14. The oligonucleotide according to any one of claims 1 to 13, wherein the oligonucleotide is a double-stranded RNAi inhibitor molecule comprising a sense strand and an antisense strand that form a double-stranded region; preferably, the sense strand is 15-35 nucleotides in length, and the antisense strand is 15-30 nucleotides in length; more preferably, the length ratio of the sense strand to the antisense strand is 19/21, 21/23, or 23/25.

15. The oligonucleotide according to any one of claims 1 to 14, wherein in the oligonucleotide, at least one nucleotide other than the structure of the compound represented by formula (I) is a modified nucleotide.

16. The oligonucleotide according to any one of claims 11 to 15, wherein at least one phosphoester group in the sense strand and/or the antisense strand is a phosphoester group with a modifying group, preferably a phosphorothioate diester group or a 5'-vinyl phosphodiester group.

17. A compound represented by formula (II) or a pharmaceutically acceptable salt thereof: 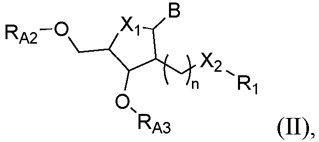 wherein:
B, X₁, X₂, R₁, and n are defined as described in any one of claims 1 to 9;
R_{A2} is selected from the group consisting of a leaving group, and R_{A3} is selected from the group consisting of a phosphorus-containing reactive group; or R_{A3} is selected from the group consisting of a leaving group, and R_{A2} is selected from the group consisting of a phosphorus-containing reactive group;
the compound represented by formula (II) is not selected from the group consisting of: 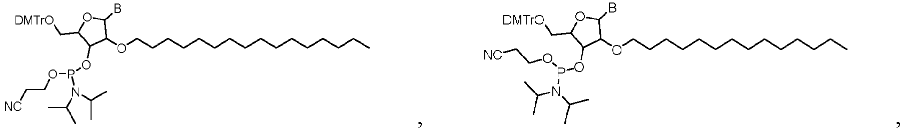 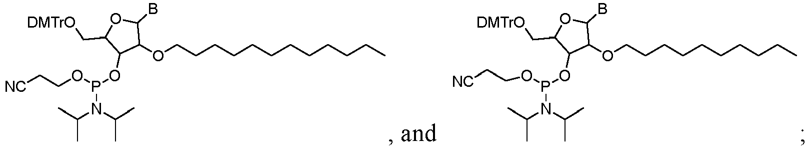
preferably, the compound represented by formula (II) is selected from the group consisting of 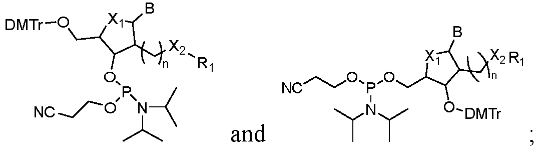
more preferably, the compound represented by formula (II) is selected from the group consisting of:

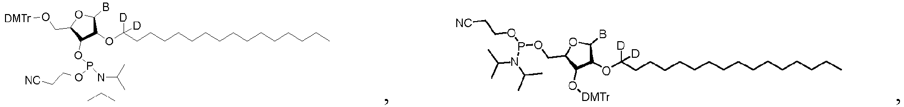

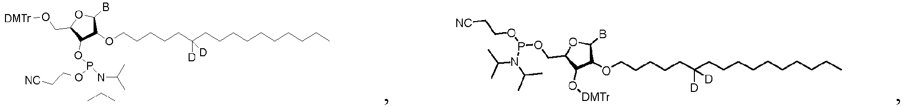

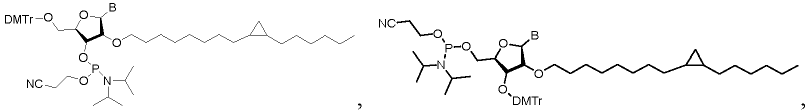

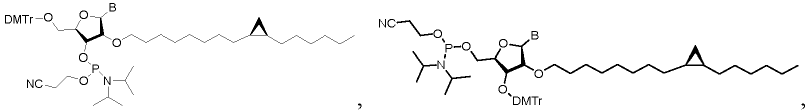

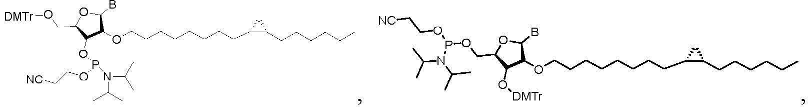

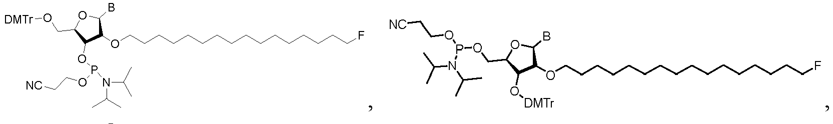

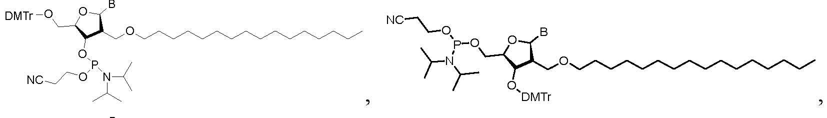

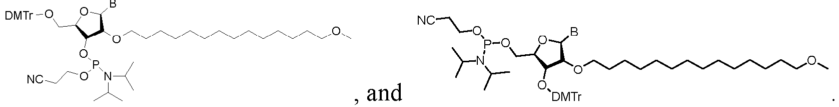

18. A method for preparing the oligonucleotide according to any one of claims 1 to 17, comprising the following step:
1) synthesizing the compound represented by formula (II) or the pharmaceutically acceptable salt thereof according to claim 17,
wherein optionally, the method further comprises 2) using the compound represented by formula (II) or the pharmaceutically acceptable salt thereof synthesized in step 1) to synthesize the oligonucleotide according to any one of claims 1 to 16.

19. A pharmaceutical composition, comprising the oligonucleotide according to any one of claims 1 to 16, wherein preferably, the pharmaceutical composition further comprises one or more pharmaceutically acceptable excipients.

20. A method for inhibiting expression of a target gene, comprising administering to a subject an effective amount or effective dose of the oligonucleotide according to any one of claims 1 to 16 or the pharmaceutical composition according to claim 19.

21. Use of the oligonucleotide according to any one of claims 1 to 16 or the pharmaceutical composition according to claim 19 in the manufacture of a medicament for preventing and/or treating diseases related to lung, eye, and central nervous system disorders, as well as tumor-related diseases.

22. A method for delivering an oligonucleotide extrahepatically, comprising administering to a subject an effective amount or effective dose of the oligonucleotide according to any one of claims 1 to 16 or the pharmaceutical composition according to claim 19.

23. The method according to claim 22, wherein the oligonucleotide is delivered into the lungs, eyes, or central nervous system; preferably, the oligonucleotide is delivered to the central nervous system.

24. The method according to claim 22, wherein the oligonucleotide is delivered to a tumor; preferably, the oligonucleotide is delivered to pancreatic cancer, prostate cancer, breast cancer, and lung cancer; more preferably, the oligonucleotide is delivered to pancreatic cancer and prostate cancer.
